# EUROPEAN PATENT APPLICATION

(11) **EP 4 785 874 A1**
(43) Date of publication of application: **05.08.2026**
(21) Application number: 25155847.4
(22) Date of filing: 04.02.2025
(51) Int. Cl.: A61B 8/08, A61B 8/00

(54) **AUTOMATIC TWIN CHARACTERIZATION WITH ABNORMALITY DETECTION AND GROWTH SURVEILLANCE FROM BLIND SWEEP PROTOCOL**

(71) Applicant: Koninklijke Philips N.V., 5656 AE Eindhoven (NL)
(72) Inventor: SARDAR, Mousumi, Eindhoven (NL); V, Manikanda Krishnan, Eindhoven (NL); VAJINEPALLI, Pallavi, 5656AG Eindhoven (NL)
(74) Representative: Philips Intellectual Property & Standards

(57) **Abstract**

A system includes a processor configured for communication with an ultrasound probe. The processor is configured to control the ultrasound probe to obtain ultrasound image frames during a blind sweep protocol on a patent with a pregnancy, and provide the ultrasound image frames as an input to a deep learning network trained to detect fetal anatomies. The processor is also configured to generate, as an output of the deep learning network, detections of the fetal anatomies within the ultrasound image frames, generate an anatomical map based on the fetal anatomies, determine that the pregnancy is a multiple gestation based on the anatomical map, and display an output representative of the determination that the pregnancy is a multiple gestation.

## Description

### FIELD OF THE INVENTION

The subject matter described herein relates to devices, systems, and methods for using ultrasound data from a blind abdominal imaging sweeps to characterize fetal position (e.g., fetal lie and/or fetal spine position), detect abnormalities, and monitor growth in multiple gestation pregnancies.

### BACKGROUND OF THE INVENTION

Ultrasound imaging remains out of reach for most pregnant women in developing countries because it requires a trained sonographer to acquire and interpret the images. As twins continue to increase in frequency, routine ultrasound surveillance for the complications of twin pregnancies may also be increasingly necessary.

Ultrasound imaging is often used for diagnostic purposes in an office or hospital setting, but may also be used in resource-constrained care settings (e.g., homes, accident sites, ambulances, mobile health facilities, etc.) by emergency personnel, home health nurses, midwives, etc., who may lack ultrasound expertise. To facilitate ultrasound image acquisition by untrained or minimally trained users, a "blind sweep" protocol may be employed, in which the user follows pre-determined probe paths (e.g., sweeping out a pattern on the patient's abdomen) during imaging.

Ultrasound imaging is a vital component of high-quality obstetric care. For example, multiple gestation (MG) pregnancy requires constant monitoring by health care providers to avoid conditions that may threaten the lives of the fetuses and the mother at birth. For labor management, the diagnosis of MG, as well as fetal orientation (including fetal presentation and fetal lie), may be essential to guarantee delivery viability. It may also be highly desirable to detect abnormalities such as conjoined fetuses. Unlike urban areas, the population in rural zones experience difficulties in accessing healthcare monitoring. Although telemedicine has helped bring medical technology closer to these regions, the diagnosis still requires medical specialists.

With multiple pregnancies, both the pregnant person and fetuses face a higher risk of complications. Prenatal checkups occur more frequently with twin pregnancies, making it possible to detect issues in earlier stages. Thus, twin detection in early pregnancy will help mother and fetuses to get any necessary specialized care.

The information included in this Background section of the specification, including any references cited herein and any description or discussion thereof, is included for technical reference purposes only and is not to be regarded as subject matter by which the scope of the disclosure is to be bound.

### SUMMARY OF THE INVENTION

The invention is defined by the claims. Advantageous embodiments are provided in the dependent claims. Disclosed is a multiple gestation analysis system that, following a blind sweep protocol (sweeps at particular locations along body without trying to find specific anatomy), detects anatomical features in the captured images and uses them for an automatic workflow of fetus characterization (including multiple gestation, fetal lie and presentation) and monitoring of the labor/evolution of the fetuses, through an anatomical spatio-temporal map, constructed from a deep-learning anatomical detector/segmentation model using imaging data from a blind sweep. We address the problem of ultrasound access in under-resourced areas by presenting a method that can automatically identify multiple gestation, chorionicity and characterize each fetus from the data obtained with use of the blind sweep protocol. The present disclosure provides for the use of deep learning techniques for detecting fetal and maternal anatomies and visual markers to identify twin pregnancy, including chronicity and twin labelling. Additionally, the present disclosure targets the abnormalities (if any) and twin growth over different gestational ages.

The workflow also includes sweep calibration steps which help user to ensure complete capture of fetal anatomies for correct assessment.

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions. One general aspect includes a system a processor configured for communication with an ultrasound probe, where the processor is configured to: control the ultrasound probe to obtain a plurality of ultrasound image frames during a blind sweep protocol on a patent with a pregnancy; provide the plurality of ultrasound image frames as an input to a deep learning network trained to detect a plurality of fetal anatomies; generate, as an output of the deep learning network, a plurality of detections of the plurality of fetal anatomies within the plurality of ultrasound image frames; generate an anatomical map based on the plurality of fetal anatomies; determine that the pregnancy is a multiple gestation based on the anatomical map; and provide, to a display in communication with the processor, an output representative of the determination that the pregnancy is the multiple gestation. Other embodiments of this aspect include corresponding computer systems, apparatus, and computer programs recorded on one or more computer storage devices, each configured to perform the actions of the methods. In particular, another general aspect includes method for multiple gestation pregnancy analysis, comprising: controlling an ultrasound probe to obtain a plurality of ultrasound image frames during a blind sweep protocol on a patent with a pregnancy;
providing the plurality of ultrasound image frames as an input to a deep learning network trained to detect a plurality of fetal anatomies;
generating, as an output of the deep learning network, a plurality of detections of the plurality of fetal anatomies within the plurality of ultrasound image frames; generating an anatomical map based on the plurality of fetal anatomies;
determining that the pregnancy is a multiple gestation based on the anatomical map; and
providing, to a display, an output representative of the determination that the pregnancy is the multiple gestation. Yet another general aspect includes a non-transitory computer-readable medium storing instructions that, when executed by a processor, cause the processor to perform the above method.

Implementations may include one or more of the following features. In some aspects, the processor is configured to: generate a plurality of binary masks based on the plurality of detections, where an individual binary mask is associated with an individual ultrasound image frame in a sweep of the blind sweep protocol; and generate the map based on the plurality of binary masks, where the map is representative of the sweep as a whole. This advantageously extends the ability to assess fetal anatomies by ensuring each frame contributes to a reliable and detailed overview of the pregnancy. Generating binary masks for the ultrasound image frames enhances precision in mapping fetal anatomies. This leads to a more comprehensive anatomical map, reflecting the entire sweep, which may aid in accurately detecting multiple gestations.

In some aspects, the anatomical map may include a plurality of detection regions representative of the plurality of fetal anatomies, where, to determine that the pregnancy is the multiple gestation, the processor is configured to: perform connected component analysis (CCA) on the first anatomical map; determine, based on the CCA, that: the pregnancy is the multiple gestation when the anatomical map may include at least two detection regions for a first fetal anatomy and at least two detections regions for a second fetal anatomy; the pregnancy is not the multiple gestation when the anatomical map does not may include at least two detection regions for the first fetal anatomy and at least two detection regions for the second fetal anatomy. Performing CCA on the anatomical map advantageously enables the processor to systematically identify and count distinct anatomical regions, which increases the accuracy in determining whether the pregnancy involves multiple gestations. By providing precise detection and differentiation of anatomical regions, the performance of deep learning for identifying multiple fetuses may be advantageously enhanced.

In some aspects, the output representative of the determination that the pregnancy is the multiple gestation may include the anatomical map. Including the anatomical map as part of the output provides a visual confirmation of the multiple gestation determination, which facilitates better assessment and understanding for medical professionals, further enhancing diagnostic confidence. This greatly supports the transforming of raw data into a clear, actionable visual representation. In some aspects, the deep learning network is trained to detect a plurality of maternal anatomies, wherein the plurality of detections may include the plurality of maternal anatomies, wherein the processor is configured to: determine a placentation of the multiple gestation based on at least one of the plurality of detections or the anatomical map; and provide, to the display, an output representative of the placentation. Training the deep learning network to detect maternal anatomies, in addition to fetal anatomies, advantageously allows for the determination of placentation. This provides a deeper layer of information, and may improve diagnostic capabilities related to the type of multiple gestation. In this way, pregnancy health can be monitored comprehensively.

In some aspects, the placentation of the multiple gestation may include one of: monochorionic and monoamniotic; monochorionic and diamniotic; or dichorionic and diamniotic. By specifying the type of placentation, a more detailed analysis can be ensured, which further aids in appropriate clinical decision-making. This advantageously enhances the capability to precisely classify the nature of multiple gestations, leading to better tailored clinical responses. In some aspects, when the placentation of the multiple gestation may include monochorionic and monoamniotic, the processor is configured to determine whether a multiple gestation abnormality is suspected; and provide, to the display, an output representative of whether the multiple gestation abnormality is suspected, wherein the multiple gestation abnormality may include two fetuses sharing one fetal anatomy. The ability to detect anatomical sharing in monochorionic/monoamniotic pregnancies may advantageously allow early identification of potential abnormalities. This enables timely medical interventions, improving outcomes, which makes it possible to provide accurate early-warning signs of anomalies within multiple gestations. In some aspects, the processor is configured to identify a first fetus and the second fetus distinctly from one another, based on the anatomical map. Clear distinction of each fetus on the anatomical map is critical for evaluation in multiple gestations. This supports better monitoring and management for each fetus, and highlights a distinct way deep learning networks can be implemented by ensuring accurate, distinguishable mapping of each fetus.
In some aspects, the anatomical map is generated based on the plurality of detections in a sweep of the blind sweep protocol resulting in a first spine detection region and a second spine detection region in the anatomical map that are spaced from one another by at least a threshold distance. In some aspects, to identify the first fetus and the second fetus, the processor is configured to perform a hierarchical clustering on the anatomical map; and separate the anatomical mask into a plurality of detection regions representative of a first fetus and a plurality of detection regions representative of the second fetus. Hierarchical clustering on the anatomical map may greatly facilitate the organized separation of detection regions, enhancing the accuracy of identifying individual fetuses, which is essential for precise monitoring. This advantageously augments the capacity for fetal analysis by introducing a structured approach to identifying individual fetal anatomies.

In some aspects, the processor is configured to: determine a fetal lie for the first fetus and a fetal lie for the second fetus, distinctly from one another; and provide, to the display, an output representative of the fetal lie for first fetus and the fetal lie for the second fetus. In some aspects, the processor is configured to: determine a fetal spine position for the first fetus and a fetal spine position for the second fetus, distinctly from one another; and provide, to the display, an output representative of the fetal spine position for the first fetus and the fetal spine position for the second fetus. Determining fetal lie or fetal spine positions separately for each fetus provides comprehensive positioning information. Displaying this data visually helps in accurate evaluation and monitoring of each fetus. This further advances diagnostic capability by ensuring comprehensive and clear anatomical positioning.

In some aspects, the processor is configured to: perform a comparison between a fetal size parameter for the first fetus and a fetal size parameter for the second fetus; determine a relative growth between the first fetus and the second fetus; and provide, to the display, an output of the representative of the relative growth. In some aspects, the fetal size parameter for the first fetus and the fetal size parameter for the second fetus each may include at least one of a length, an area, or a volume of the anatomical map. Comparing size parameters between fetuses aids in detecting relative growth patterns, and advantageously provides with vital information for monitoring the health and development of each fetus in multiple gestations. This further extends the fetal growth metrics and comparisons available.

In some aspects, the processor is configured to: generate a report may include the anatomical map and a previous anatomical map; and output to the report to the display, wherein the first map and the previous first map each may include a detection region representative of the first fetus and a detection region representative of the second fetus, wherein sizes of the detection regions are configured to provide an indication of at least one of: the relative growth; a growth of the first fetus over time; and a growth of the second fetus over time. Creating reports with current and previous anatomical maps showing relative growth over time provides valuable insights into fetal development, which assists in tracking changes and identifying potential concerns early. These specific aspects advantageously transform single timepoint collected data into longitudinal reports. In some aspects, the processor is configured to: control the ultrasound probe to perform a calibration sweep before the blind sweep protocol; determine if a length of the calibration sweep is acceptable; prompt a user to rescan when the length of the calibration sweep is not acceptable; and prompt the user to proceed to the blind sweep protocol when the length of the calibration sweep is acceptable. Ensuring accurate calibration sweep length with user prompts improves the reliability of subsequent blind sweeps. In some aspects, the processor is configured to: if all required anatomies are not present in the plurality of ultrasound image frames, prompt the user to proceed to the blind sweep protocol. This guarantees that all necessary data for an accurate analysis has been collected. These aspects ensure the integrity and accuracy of the initial data collection process.

In some aspects, to determine whether the multiple gestation is monochorionic or dichorionic, the processor is configured to determine a quantity of placentas using at least one of: a plurality of placenta clusters based on a clustering with midpoints of the plurality of detections; or a plurality of placenta components based on connected component analysis (CCA) with the anatomical map. In some aspects, to determine whether the multiple gestation is monoamniotic or diamniotic, the processor is configured to determine if the plurality of detections include a detection of an inter-twin membrane. Using clustering or CCA to count placentas may advantageously improve in reliably identifying if the gestation is monochorionic or dichorionic. Additionally, detecting an inter-twin membrane makes it easier to distinguish monoamniotic from diamniotic gestations, offering a more comprehensive analysis of the gestation type. This improves the mapping functionality by adding refined placental classification. Implementations of the described techniques may include hardware, a method or process, or computer software on a computer-accessible medium.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the multiple gestation analysis system, as defined in the claims, is provided in the following written description of various aspects of the disclosure and illustrated in the accompanying drawings.

### BRIEF DESCRIPTION OF THE DRAWINGS

Illustrative aspects of the present disclosure will be described with reference to the accompanying drawings, of which:
Fig. 1 is a schematic, diagrammatic representation of an ultrasound imaging system, according to aspects of the present disclosure.
Fig. 2 is a schematic diagram of a processor circuit, according to aspects of the present disclosure.
Fig. 3A is a schematic, diagrammatic, cross-sectional view of a fetus surrounded by amniotic fluid within a uterus accessible through a cervix of a patient, according to aspects of the present disclosure.
Fig. 3B is a schematic, diagrammatic, cross-sectional view of a fetus surrounded by amniotic fluid within a uterus accessible through a cervix of a patient, according to aspects of the present disclosure.
Fig. 3C is a schematic, diagrammatic, cross-sectional view of a fetus surrounded by amniotic fluid within a uterus accessible through a cervix of a patient, according to aspects of the present disclosure.
Fig. 3D is a schematic, diagrammatic, cross-sectional view of a fetus surrounded by amniotic fluid within a uterus accessible through a cervix of a patient, according to aspects of the present disclosure.
Fig. 3E is a schematic, diagrammatic, cross-sectional view of a fetus within a uterus accessible by a cervix, according to aspects of the present disclosure.
Fig. 3F is a schematic, diagrammatic, cross-sectional view of a fetus within a uterus accessible by a cervix, according to aspects of the present disclosure.
Fig. 4 is a schematic, diagrammatic representation of a patient, according to aspects of the present disclosure.
Fig. 5 is a schematic, diagrammatic representation, in hybrid block diagram/flow diagram form, of an example multiple gestation analysis system, according to aspects of the present disclosure.
Fig. 6 is a schematic, diagrammatic representation, in hybrid block diagram/flow diagram form, of an example fetal orientation determination method to triage and/or monitor fetal lie and presentation, according to aspects of the present disclosure.
Fig. 7A is a schematic, diagrammatic overview, in block diagram form, of a training mode for an untrained neural network, according to aspects of the present disclosure.
Fig. 7B is a schematic, diagrammatic overview, in block diagram form, of an inference mode or clinical usage mode for the trained neural network, according to aspects of the present disclosure.
Fig. 8 is a schematic, diagrammatic illustration, in block diagram form, of the detection of anatomy (e.g., the placenta, fetal head, fetal spine, etc.), according to aspects of the present disclosure.
Fig. 9 is a schematic, diagrammatic representation, in flow diagram form, of an example fetal orientation determination method, according to aspects of the present disclosure.
Fig. 10 is a schematic, diagrammatic representation, in hybrid flow diagram/block diagram form, of an example fetal orientation determination method, according to aspects of the present disclosure.
Fig. 11 is a schematic, diagrammatic representation, in hybrid block diagram/flow diagram form, of an example 3D/2D spatio-temporal map generation process, in accordance with aspects of the present disclosure.
Fig. 12A is an ultrasound image frame with anatomy detection boxes, including an anatomy detection box for fetal anatomy type A (e.g., the fetal spine), according to aspects of the present disclosure.
Fig. 12B is a fetal spine mask generated from the ultrasound image frame of Figure 12A, according to aspects of the present disclosure.
Fig. 13A is an ultrasound image frame with anatomy detection boxes, including an anatomy detection box for fetal anatomy type B (e.g., the fetal heart), according to aspects of the present disclosure.
Fig. 13B is a fetal heart mask generated from the ultrasound image frame of Figure 13A, according to aspects of the present disclosure.
Fig. 14 is a 3D anatomical spatio-temporal map of the fetal anatomy from a vertical sweep (e.g., an M sweep), according to aspects of the present disclosure.
Fig. 15A is a 2D anatomical spatio-temporal map for a vertical sweep (e.g., an M sweep), according to aspects of the present disclosure.
Fig. 15B is a 2D anatomical spatio-temporal map for a horizontal sweep (e.g., a C2 or C3 sweep), according to aspects of the present disclosure.
Fig. 16A-16B is a schematic, diagrammatic representation, in hybrid flow diagram/block diagram form, of an example fetal lie determination method, according to aspects of the present disclosure.
Fig. 17 is a diagram indicating the meaning of different values for the fetal orientation angle Θ, according to aspects of the present disclosure.
Fig. 18 is a schematic, diagrammatic representation, of an example fetal orientation determination process, according to aspects of the present disclosure.
Fig. 19 is a 2D anatomical spatio-temporal map for a vertical sweep (e.g., an M sweep), according to aspects of the present disclosure.
Fig. 20 is a schematic, diagrammatic representation, in flow diagram form, of an example fetal lie detection process, according to aspects of the present disclosure.
Fig. 21 is a schematic, diagrammatic representation, of an example fetal orientation determination process, according to aspects of the present disclosure.
Fig. 22 is a schematic, diagrammatic representation of an example fetal orientation determination process, according to aspects of the present disclosure.
Fig. 23 is a schematic, diagrammatic representation, in hybrid flow diagram/block diagram form, of an example fetal lie determination method, according to aspects of the present disclosure.
Fig. 24 is a schematic, diagrammatic representation of an example fetal orientation determination method, according to aspects of the present disclosure.
Fig. 25 is a schematic, diagrammatic representation, in flow diagram form, of an example fetal spine presentation determination method, according to aspects of the present disclosure.
Fig. 26A is a plot of the midpoints of the anatomy detection boxes across a sweep, according to aspects of the present disclosure.
Fig. 26B is a synthesized ultrasound image generated based on the anatomy detection cluster centroids of Fig. 26A, according to aspects of the present disclosure.
Fig. 26C is a schematic, diagrammatic, side cross-sectional view of a fetus inside a uterus of a patient, according to aspects of the present disclosure.
Fig. 27A is a plot of the midpoints of the anatomy detection boxes across a sweep, according to aspects of the present disclosure.
Fig. 27B is a synthesized ultrasound image generated based on the anatomy detection cluster centroids of Fig. 27A, according to aspects of the present disclosure.
Fig. 27C is a schematic, diagrammatic, side cross-sectional view of a fetus inside a uterus of a patient, according to aspects of the present disclosure.
Fig. 28 is a schematic, diagrammatic representation of a sweep acceptance process, according to aspects of the present disclosure.
Fig. 29 is a schematic, diagrammatic representation of a sweep rejection process, according to aspects of the present disclosure.
Fig. 30 is a schematic, diagrammatic representation, in hybrid flow diagram/block diagram form, of an example sweep calibration method, according to aspects of the present disclosure.
Fig. 31 is a schematic, diagrammatic representation of accepted or acceptable calibration sweeps, according to aspects of the present disclosure.
Fig. 32 is a schematic, diagrammatic representation of rejected or unacceptable calibration sweeps, according to aspects of the present disclosure.
Fig. 33 is a schematic, diagrammatic representation of a fetal growth monitoring process, according to aspects of the present disclosure.
Fig. 34A is a set of diagrammatic views of two fetuses surrounded by a at least one amniotic sac or amnion and at least one chorionic sac or chorion within a uterus of a patient, representing different types of twins, according to aspects of the present disclosure.
Fig. 34B is a set of schematic, diagrammatic, cross-sectional views of two fetuses arranged in different positions within a uterus of a patient as seen by an ultrasound imaging plane, according to aspects of the present disclosure.
Fig. 35 is a schematic, diagrammatic representation, in hybrid block diagram/flow diagram form, of an example multiple gestation analysis system, according to aspects of the present disclosure.
Fig. 36 is a schematic, diagrammatic representation, in hybrid block diagram/flow diagram form, of an example fetal orientation determination method to triage and/or monitor multiple gestation, according to aspects of the present disclosure.
Fig. 37 is a schematic, diagrammatic representation, in hybrid block diagram / flow diagram form, of an example multiple gestation analysis method, according to aspects of the present disclosure.
Fig. 38 is a schematic, diagrammatic representation of a multiple gestation sweep selection process for vertical sweeps, according to aspects of the present disclosure.
Fig. 39 is a 3D anatomical spatio-temporal map of the fetal anatomy from a vertical sweep (e.g., an M sweep), according to aspects of the present disclosure.
Fig. 40 is a schematic, diagrammatic representation, in hybrid block diagram / flow diagram form, of an example multiple gestation detection method, according to aspects of the present disclosure.
Fig. 41 is a 2D anatomical spatio-temporal map for a vertical sweep (e.g., an M sweep), according to aspects of the present disclosure.
Fig. 42 is a schematic, diagrammatic representation, in hybrid block diagram / flow diagram form, of an example fetal placentation determination method for multiple gestation pregnancies, according to aspects of the present disclosure.
Fig. 43 is a schematic, diagrammatic representation, in flow diagram form, of an example multiple gestation abnormalities determination method, according to aspects of the present disclosure.
Fig. 44 is a schematic, diagrammatic representation, in hybrid block diagram / flow diagram form, of an example multiple gestation abnormalities determination method, according to aspects of the present disclosure.
Fig. 45 is a 3D spatio-temporal map of a vertical sweep (e.g., an M sweep), according to aspects of the present disclosure.
Fig. 46 is a schematic, diagrammatic representation, in hybrid block diagram / flow diagram form, of an example individual fetus identification method, according to aspects of the present disclosure.
Fig. 47 is a schematic, diagrammatic representation of an example individual fetus identification process, according to aspects of the present disclosure.
Fig. 48 is a diagrammatic representation of a density-based spatial clustering of applications with noise (DBSCAN) process using synthetic data points, according to aspects of the present disclosure.
Fig. 49 is a schematic, diagrammatic representation, in flow diagram form, of an example multiple gestation / twin growth surveillance method, according to aspects of the present disclosure.
Fig. 50 is an example screen display for multiple gestation grown surveillance, according to aspects of the present disclosure.
Fig. 51 is a schematic, diagrammatic representation, in flow diagram form, of an example blind sweep quality check method, according to aspects of the present disclosure.
Fig. 52 shows two sets of vertical sweeps from a 5x5 blind sweep protocol, which can serve as inputs to the quality check method of Fig. 51, according to aspects of the present disclosure.

### DETAILED DESCRIPTION OF EMBODIMENTS

Ultrasound imaging remains out of reach for most pregnant women in developing countries because it requires a trained sonographer to acquire and interpret the images. The present disclosure addresses this problem by presenting a method that can automatically identify multiple gestation, chorionicity, and characterize each fetus from the data obtained with use of the blind sweep protocol.

As twins continue to increase in frequency, routine ultrasound surveillance for the complications of twin pregnancies may also be increasingly necessary. The present disclosure provides for the use of deep learning techniques for detecting fetal and maternal anatomies and visual markers, to identify twin pregnancy including chronicity and twin labelling. Additionally, the present disclosure identifies abnormalities (if any) and twin growth over different gestational ages.

Thus, in accordance with at least one aspect of the present disclosure, a multiple gestation analysis system is provided which can identify anatomy of interest for determining multiple gestation, including fetal orientation (e.g., fetal lie and fetal presentation).

Multiple gestation pregnancy requires constant monitoring by health care providers to avoid conditions that may threaten the lives of the fetus and the mother at birth. For labor management, the diagnosis of fetal presentation may be important to guarantee delivery viability. Knowing the position of the fetuses helps a pregnancy care provider determine if it's safe for the patient to have a vaginal delivery or if they should consider a C-section (cesarean delivery).

In this work, the data is acquired from a 3x3 or 5x5 blind sweep protocol, covering the uterus of the mother. From the image sets acquired in these sweeps (also known as cine scans), the disclosed method includes generation of a 3D/2D anatomical spatio-temporal map, requiring only one transverse (M) and one longitudinal scan(C2/C3) to automatically characterize fetus including head and spine position. Ideal position of the fetus is head down, facing the birth parent's back, sometimes known as cephalic or occiput anterior presentation.

The present disclosure provides a method that can automatically identify multiple gestation, chorionicity, and characterize each fetus from the data obtained with use of the blind sweep protocol. This method is designed to work on ultrasound scans captured by inexperienced/amateur sonographers (e.g., midwives who will be trained for at most a week), using low-to-moderate quality ultrasound scans. Early in pregnancy, detection of multiple gestation from singleton pregnancy can help mother and fetus both to get better care to avoid complications and cared delivery.

Localization of the fetal spine position, along with fetal lie, provides more detailed characterization of the fetus. The positions of the head and spine during the second stage of labor can be practical indicators for predicting the occiput posterior position at delivery. In one study cohort, with occiput posterior and spine anterior position on ultrasound, none of the babies was born in the occiput posterior position. On the other hand, the fetuses presenting occiput posterior position at delivery also had a posterior spine position during the second stage of labor.

The workflow disclosed herein incorporates sweep calibration to ensure the correct capture of relative fetus anatomies during blind protocol, which can lead to more accurate assessment of the fetus' position within the uterus. Additional monitoring components in the workflow will enable a user to keep track of fetus growth and movement over different gestational periods.

The present disclosure provides a method for an end-to-end fetus characterization workflow from blind sweeps, e.g., using low cost ultrasound devices such as Philips Lumify. This method is designed to work on ultrasound scans captured by inexperienced/amateur sonographers (e.g., midwives with a week of training or less), using low-to-moderate quality ultrasound scans. Moreover, currently in a blind protocol, users (and physicians to whom the patient is referred) may not have access to the original ultrasound images. The proposed method provides a summarized view of the fetus inside the uterus through the use of a 2D/3D fetus and/or mother anatomical spatio-temporal map visualization from the captured sweep data. In some aspects, the ultrasound images themselves are not shown to the inexperienced user (who would not have the training to interpret the ultrasound images themselves). The anatomical spatio-temporal map can have different applications to assess the fetus, which gives deeper explainability to the current blind sweep protocol based solution. Additionally, spine localization and sweep calibration steps help to ensure that proper blind scans are performed, and thus improve on current patient outcomes.

### Components of the disclosed method include:

Anatomy detector: A deep learning network that takes the cine scans as input and outputs detections (e.g., bounding boxes) for the fetal and maternal anatomies. In one approach, a You Only Look Once (YOLO) deep learning network can be used as an anatomy detector which detects the individual anatomy and provides bounding box coordinates with confidence scores corresponding to each anatomy, as described below. As an alternative, a segmentation model can also be used to get the anatomical masks instead of detection model and bounding boxes.

Sweep Length Calibration: In this calibration step, the user will perform one longitudinal and one transverse scan to get a rough estimation of where to start and end the scans. In sweep Length Calibration step, user needs to perform a transverse sweep (such as the M sweep of the blind sweep protocol) and a longitudinal sweep (such as the C2 or C3 sweep). Once the sweep is performed, an anatomy detector will detect the anatomies. Once the anatomies are detected, an anatomical spatio-temporal map is generated based on the detections as described below. From the generated map, if the relevant required anatomies (head, heart, abdomen, spine, fetal urinary bladder, femur) are captured in the map, scan positions can be noted for the next blind scan. If the relative anatomies are not captured in the generated map, recalibration/ rescan may be performed. This step ensures the capture of all fetal anatomies for fetal parameter assessment.

Anatomical spatio-temporal map generation: In this step, a 3D/2D map is generated from the selected vertical and horizontal scans (e.g., M and C2 or C3). The map or visualization provides the lie of the fetus inside the uterus. In this step, the input is the bounding box co-ordinates for each relevant anatomy. A mask is generated from bounding box co-ordinates for each individual anatomy. Next, the co-ordinates of each anatomy are extracted from the corresponding mask volume, and a 3D map is generated from the coordinates. The map encompasses relevant anatomies of the fetus as well as of the mother (if required). The map can then be provided to the user as one of the outputs of the workflow.

Twin (multiple gestation) detection: With multiple pregnancies, both the pregnant person and fetuses face a higher risk of complications. Prenatal checkups occur more frequently with twin pregnancies, making it possible to detect issues in earlier stages. Thus, twin detection in early pregnancy will help mother and fetus to get necessary/ specialized care. In this step, based on the number of connected components of each anatomy in the anatomical spatio-temporal map and model, a twin pregnancy or multiple gestation pregnancy can be identified.
Placentation of twins: After determination of the diagnosis of multiple pregnancy, important additional information to determine is the precise number of fetuses and the chorionicity (number of placentae) and amnionicity (number of amniotic sacs) of the pregnancy. In this step, the system classifies the multiple gestational pregnancy with chorionicity and amniocity. While the majority (>80%) of twin pregnancies are dichorionic, monochorionic pregnancies are associated with worse perinatal outcomes, are affected by several conditions specific to twins sharing a placental circulation, and require significantly more antenatal surveillance. In monochorionic monoamniotic (MCMA) pregnancies, no membrane is seen, whereas in case of monochronic diamniotic (MCDA) an intertwin membrane is expected to be seen.

Twin localization and labeling: Once the system determines the number of fetuses, in this step, it localizes individual fetal anatomies based on a hierarchical clustering approach, and individual fetus labelling is done after localization. In this step, first the twins are localized and labeled with their respective anatomies, to keep consistency at every subsequent scan. Clinically, it may be good practice to describe each twin as fully as possible (e.g. fetus 1 is cephalic, on maternal left, and has anterior placenta, while fetus 2 is breech, on maternal right, and has posterior placenta) to minimise the chance of confusion. The visualization-based approach is helpful to represent the twins' relative positions pictorially in the maternal notes. This step may include fetal position characterization, fetal lie identification, and fetal spine localization, as described below.

### Fetal positional characterization: This block includes two components:

Fetal lie identification: In this step, fetal lie (longitudinal (cephalic/breech)/ transverse/ oblique) is estimated using the generated map.

Fetal spine localization: Spine localization classifies the spine as anterior (spine lies opposite to mother's spine) or posterior (spine lies towards mother's spine). Once the lie of the fetus is identified, the system can select an appropriate scan based on the lie. If the fetus has a longitudinal lie (i.e. in alignment with mother's spine), the system may select a longitudinal scan (e.g., C2 or C3). On the other hand, if the fetus is in a transverse lie, the system may select a transverse scan (e.g., M). The midpoints of the bounding boxes of the heart, amniotic fluid, placenta, and spine are calculated for the selected scan. A 2D plot can then be created from these midpoints to visualize the relative position of the anatomies.

Placenta location estimation can then be done with respect to the amniotic fluid for the selected scan, as described for example in U.S. Provisional Application No. 63/611,810, filed December 19, 2023, and titled "Low-Lying Placenta and/or Placenta Location in Ultrasound Imaging with Blind Sweep Protocol", incorporated by reference as though fully set forth herein. Once the placenta location is determined, the system can assess the relative position of the centroids of the spine and heart clusters with respect to the placenta cluster centroid. If the heart cluster centroid comes in between the placenta and spine cluster centroids, this indicates that the spine location is opposite to the placenta location. Similarly, if the heart centroid cluster doesn't come in between placenta and spine cluster, this indicates that the spine is in alignment with the placenta location.

Twin abnormality screening: Placentation of twins as monochorionic enables the system to further analyze whether the twins are conjoined (abnormal) or not. If present, possible abnormalities may be identified from the anatomical spatio-temporal map. Based on different abnormality types, the map may contain fused components of a particular anatomy (like head fused/ spine fused etc.).

Growth surveillance: Assessment of fetal growth in twins is an important parameter clinically. Although twins will rarely be identical in size, significant growth discrepancies are associated with poor perinatal outcomes in a continuous fashion, and may be more important in relation to perinatal outcome than the absolute size of the individual fetuses. A growth discrepancy of 25% predicts poor perinatal outcomes in twins, and most national bodies recommend a discrepancy of 20-25% as a trigger for referral to fetal medicine experts or additional monitoring. In this step, approximate crown-rump length (CRL) can be calculated for individual fetus. Additionally, size fetus head, abdomen for each fetus can share some weightage to calculate fetus 1 parameter, and fetus 2 parameter. Based on the ratio of the fetuses, the system can provide an approximate measurement of the growth of the twins.

Fetus growth and/or fetal movement over different gestational ages (GAs) can be tracked using the fetal-mother anatomical spatio-temporal map. Growth charts may be used as standard practice with measurements performed using the ultrasound system. One problem is that measurements can typically only be done on a real ultrasound scan, whereas in blind protocol, there is no ultrasound scan showing the anatomies for measurement. Alternately, with the present disclosure, the fetal-maternal anatomy spatio-temporal map can be used to track the growth and monitor fetal movement over the different gestation ages (GAs).

An evaluation of fetal movement is also considered a valuable indicator of fetal health. Over the different GAs, if fetus positional characterization doesn't change, this might be indicative of some abnormality, or may be a precursor to fetal death.

The present disclosure aids substantially in the capture of high-quality fetal characterizations (e.g., multiple gestation, chorionicity, amnioticity, fetal presentation and fetal lie) by minimally trained users, by detecting fetal anatomy, assessing the images to determine fetal characteristics, and requiring that the user repeat any sweeps that do not capture the required anatomy. Implemented on a processor in communication with an ultrasound probe, the multiple gestation analysis system disclosed herein provides practical improvements in the quality of diagnosis, prophylaxis, and treatment available to pregnant patients in underserved areas. This improved imaging methodology transforms a process that is heavily reliant on professional experience into one that is accurate and repeatable even for minimally trained personnel, without the normally routine need to train clinicians such as emergency department personnel to identify multiple gestation and fetal characteristics from raw ultrasound images. This unconventional approach improves the functioning of the ultrasound imaging system, by providing reliable, repeatable imaging in hospital, office, vehicle, field, and home settings, as well as referral recommendations for patients suspected to have a pregnancy complication such as monochorionic twins.

The multiple gestation analysis system may be implemented as a process at least partially viewable on a display, and operated by a control process executing on a processor that accepts user inputs from a keyboard, mouse, or touchscreen interface, and that is in communication with one or more sensor probes. In that regard, the control process performs certain specific operations in response to different inputs or selections made at different times. Certain structures, functions, and operations of the processor, display, sensors, and user input systems are known in the art, while others are recited herein to enable novel features or aspects of the present disclosure with particularity.

These descriptions are provided for exemplary purposes only, and should not be considered to limit the scope of the multiple gestation analysis system. Certain features may be added, removed, or modified without departing from the spirit of the claimed subject matter.

For the purposes of promoting an understanding of the principles of the present disclosure, reference will now be made to the aspects illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one aspect may be combined with the features, components, and/or steps described with respect to other aspects of the present disclosure. For the sake of brevity, however, the numerous iterations of these combinations will not be described separately.

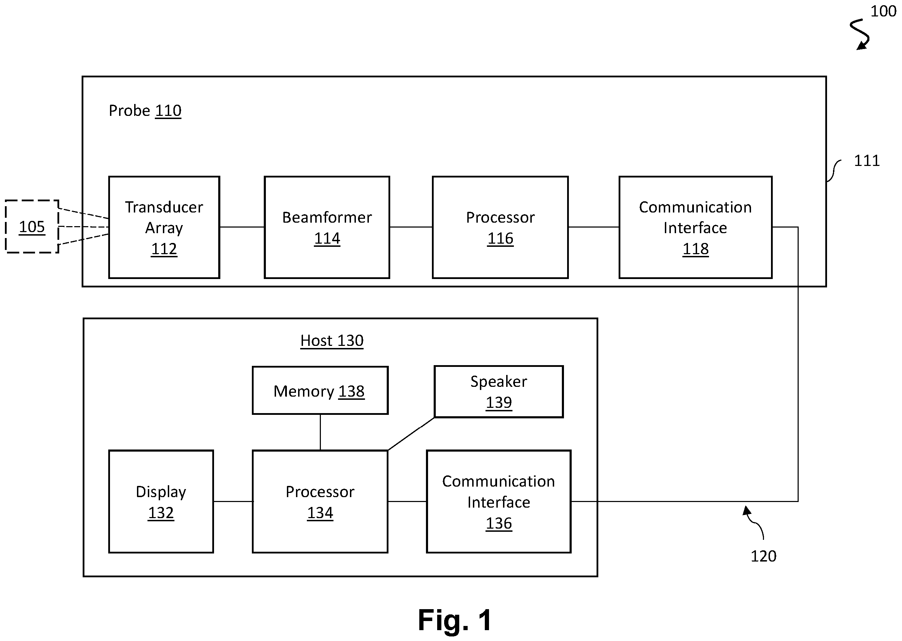

Fig. 1 is a schematic, diagrammatic representation of an ultrasound imaging system 100, according to aspects of the present disclosure. The ultrasound imaging system 100 may for example be used to acquire ultrasound video sweeps, which can then be analyzed by a human clinician or an artificial intelligence to diagnose medical conditions.

The ultrasound imaging system 100 is used for scanning an area or volume of a subject's body. A subject may include a patient of an ultrasound imaging procedure, or any other person, or any suitable living or non-living organism or structure. The ultrasound imaging system 100 includes an ultrasound imaging probe 110 in communication with a host 130 over a communication interface or link 120. The probe 110 may include a transducer array 112, a beamformer 114, a processor circuit 116, and a communication interface 118. The host 130 may include a display 132, a processor circuit 134, a communication interface 136, and a memory 138 storing subject information.

In some aspects, the probe 110 is an external ultrasound imaging device including a housing 111 configured for handheld operation by a user. The transducer array 112 can be configured to obtain ultrasound data while the user grasps the housing 111 of the probe 110 such that the transducer array 112 is positioned adjacent to or in contact with a subject's skin. The probe 110 is configured to obtain ultrasound data of anatomy within the subject's body while the probe 110 is positioned outside of the subject's body for general imaging, such as for abdomen imaging, liver imaging, etc. In some aspects, the probe 110 can be an external ultrasound probe, a transthoracic probe, and/or a curved array probe.

In other aspects, the probe 110 can be an internal ultrasound imaging device and may comprise a housing 111 configured to be positioned within a lumen of a subject's body for general imaging, such as for abdomen imaging, liver imaging, etc. In some aspects, the probe 110 may be a curved array probe. Probe 110 may be of any suitable form for any suitable ultrasound imaging application including both external and internal ultrasound imaging.

In some aspects, aspects of the present disclosure can be implemented with medical images of subjects obtained using any suitable medical imaging device and/or modality. Examples of medical images and medical imaging devices include x-ray images (angiographic images, fluoroscopic images, images with or without contrast) obtained by an x-ray imaging device, computed tomography (CT) images obtained by a CT imaging device, positron emission tomography-computed tomography (PET-CT) images obtained by a PET-CT imaging device, magnetic resonance images (MRI) obtained by an MRI device, single-photon emission computed tomography (SPECT) images obtained by a SPECT imaging device, optical coherence tomography (OCT) images obtained by an OCT imaging device, and intravascular photoacoustic (IVPA) images obtained by an IVPA imaging device. The medical imaging device can obtain the medical images while positioned outside the subject body, spaced from the subject body, adjacent to the subject body, in contact with the subject body, and/or inside the subject body.

For an ultrasound imaging device, the transducer array 112 emits ultrasound signals towards an anatomical object 105 of a subject and receives echo signals reflected from the object 105 back to the transducer array 112. The ultrasound transducer array 112 can include any suitable number of acoustic elements, including one or more acoustic elements and/or a plurality of acoustic elements. In some instances, the transducer array 112 includes a single acoustic element. In some instances, the transducer array 112 may include an array of acoustic elements with any number of acoustic elements in any suitable configuration. For example, the transducer array 112 can include between 1 acoustic element and 10000 acoustic elements, including values such as 2 acoustic elements, 4 acoustic elements, 36 acoustic elements, 64 acoustic elements, 128 acoustic elements, 500 acoustic elements, 812 acoustic elements, 1000 acoustic elements, 3000 acoustic elements, 8000 acoustic elements, and/or other values both larger and smaller. In some instances, the transducer array 112 may include an array of acoustic elements with any number of acoustic elements in any suitable configuration, such as a linear array, a planar array, a curved array, a curvilinear array, a circumferential array, an annular array, a phased array, a matrix array, a one-dimensional (1D) array, a 1.x dimensional array (e.g., a 1.5D array), or a two-dimensional (2D) array. The array of acoustic elements (e.g., one or more rows, one or more columns, and/or one or more orientations) can be uniformly or independently controlled and activated. The transducer array 112 can be configured to obtain one-dimensional, two-dimensional, and/or three-dimensional images of a subject's anatomy. In some aspects, the transducer array 112 may include a piezoelectric micromachined ultrasound transducer (PMUT), capacitive micromachined ultrasonic transducer (CMUT), single crystal, lead zirconate titanate (PZT), PZT composite, other suitable transducer types, and/or combinations thereof.

The object 105 may include any anatomy or anatomical feature, such as a kidney, liver, and/or any other anatomy of a subject. The present disclosure can be implemented in the context of any number of anatomical locations and tissue types, including without limitation, organs including the liver, kidneys, gall bladder, pancreas, lungs; ducts; intestines; nervous system structures including the brain, dural sac, spinal cord and peripheral nerves; the urinary tract; as well as valves within the blood vessels, blood, abdominal organs, and/or other systems of the body. In some aspects, the object 105 may include malignancies such as tumors, cysts, lesions, hemorrhages, or blood pools within any part of human anatomy. The anatomy may be a blood vessel, as an artery or a vein of a subject's vascular system, including cardiac vasculature, peripheral vasculature, neural vasculature, renal vasculature, and/or any other suitable lumen inside the body. In addition to natural structures, the present disclosure can be implemented in the context of man-made structures such as, but without limitation, heart valves, stents, shunts, filters, implants and other devices.

The beamformer 114 is coupled to the transducer array 112. The beamformer 114 controls the transducer array 112, for example, for transmission of the ultrasound signals and reception of the ultrasound echo signals. In some aspects, the beamformer 114 may apply a time-delay to signals sent to individual acoustic transducers within an array in the transducer 112 such that an acoustic signal is steered in any suitable direction propagating away from the probe 110. The beamformer 114 may further provide image signals to the processor circuit 116 based on the response of the received ultrasound echo signals. The beamformer 114 may include multiple stages of beamforming. The beamforming can reduce the number of signal lines for coupling to the processor circuit 116. In some aspects, the transducer array 112 in combination with the beamformer 114 may be referred to as an ultrasound imaging component.

The processor 116 is coupled to the beamformer 114. The processor 116 may also be described as a processor circuit, which can include other components in communication with the processor 116, such as a memory, beamformer 114, communication interface 118, and/or other suitable components. The processor 116 may include a central processing unit (CPU), a graphical processing unit (GPU), a digital signal processor (DSP), an application specific integrated circuit (ASIC), a controller, a field programmable gate array (FPGA) device, another hardware device, a firmware device, or any combination thereof configured to perform the operations described herein. The processor 116 may also be implemented as a combination of computing devices, e.g., a combination of a DSP and a microprocessor, a plurality of microprocessors, one or more microprocessors in conjunction with a DSP core, or any other such configuration. The processor 116 is configured to process the beamformed image signals. For example, the processor 116 may perform filtering and/or quadrature demodulation to condition the image signals. The processor 116 and/or 134 can be configured to control the array 112 to obtain ultrasound data associated with the object 105.

The communication interface 118 is coupled to the processor 116. The communication interface 118 may include one or more transmitters, one or more receivers, one or more transceivers, and/or circuitry for transmitting and/or receiving communication signals. The communication interface 118 can include hardware components and/or software components implementing a particular communication protocol suitable for transporting signals over the communication link 120 to the host 130. The communication interface 118 can be referred to as a communication device or a communication interface module.

The communication link 120 may be any suitable communication link. For example, the communication link 120 may be a wired link, such as a universal serial bus (USB) link or an Ethernet link. Alternatively, the communication link 120 may be a wireless link, such as an ultra-wideband (UWB) link, an Institute of Electrical and Electronics Engineers (IEEE) 802.11 WiFi link, or a Bluetooth link.

At the host 130, the communication interface 136 may receive the image signals. The communication interface 136 may be substantially similar to the communication interface 118. The host 130 may be any suitable computing and display device, such as a workstation, a personal computer (PC), a laptop, a tablet, or a mobile phone.

The processor 134 is coupled to the communication interface 136. The processor 134 may also be described as a processor circuit, which can include other components in communication with the processor 134, such as the memory 138, the communication interface 136, an optional speaker 139, and/or other suitable components. The processor 134 may be implemented as a combination of software components and hardware components. The processor 134 may include a central processing unit (CPU), a graphics processing unit (GPU), a digital signal processor (DSP), an application-specific integrated circuit (ASIC), a controller, an FPGA device, another hardware device, a firmware device, or any combination thereof configured to perform the operations described herein. The processor 134 may also be implemented as a combination of computing devices, e.g., a combination of a DSP and a microprocessor, a plurality of microprocessors, one or more microprocessors in conjunction with a DSP core, or any other such configuration. The processor 134 can be configured to generate image data from the image signals received from the probe 110. The processor 134 can apply advanced signal processing and/or image processing techniques to the image signals. In some aspects, the processor 134 can form a three-dimensional (3D) volume image from the image data. In some aspects, the processor 134 can perform real-time processing on the image data to provide a streaming video of ultrasound images of the object 105. In some aspects, the host 130 includes a beamformer. For example, the processor 134 can be part of and/or otherwise in communication with such a beamformer. The beamformer in the in the host 130 can be a system beamformer or a main beamformer (providing one or more subsequent stages of beamforming), while the beamformer 114 is a probe beamformer or micro-beamformer (providing one or more initial stages of beamforming).

The memory 138 is coupled to the processor 134. The memory 138 may be any suitable storage device, such as a cache memory (e.g., a cache memory of the processor 134), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, solid state drives, other forms of volatile and non-volatile memory, or a combination of different types of memory.

The memory 138 can be configured to store subject information, measurements, data, or files relating to a subject's medical history, history of procedures performed, anatomical or biological features, characteristics, or medical conditions associated with a subject, computer readable instructions, such as code, software, or other application, as well as any other suitable information or data. The memory 138 may be located within the host 130. Subject information may include measurements, data, files, other forms of medical history, such as but not limited to ultrasound images, ultrasound videos, and/or any imaging information relating to the subject's anatomy. The subject information may include parameters related to an imaging procedure such as an anatomical scan window, a probe orientation, and/or the subject position during an imaging procedure. The memory 138 can also be configured to store information related to the training and implementation of machine learning algorithms (e.g., neural networks) and/or information related to implementing image recognition algorithms for detecting/segmenting anatomy, image quantification algorithms, and/or image acquisition guidance algorithms, including those described herein.

The display 132 is coupled to the processor circuit 134. The display 132 may be a monitor or any suitable display. The display 132 is configured to display the ultrasound images, image videos, and/or any imaging information of the object 105.

The ultrasound imaging system 100 may be used to assist a sonographer in performing an ultrasound scan. The scan may be performed in a point-of-care setting. In some instances, the host 130 is a console or movable cart. In some instances, the host 130 may be a mobile device, such as a tablet, a mobile phone, or portable computer. During an imaging procedure, the ultrasound system can acquire an ultrasound image of a particular region of interest within a subject's anatomy. The ultrasound imaging system 100 may then analyze the ultrasound image to identify various parameters associated with the acquisition of the image such as the scan window, the probe orientation, the subject position, and/or other parameters. The ultrasound imaging system 100 may then store the image and these associated parameters in the memory 138. At a subsequent imaging procedure, the ultrasound imaging system 100 may retrieve the previously acquired ultrasound image and associated parameters for display to a user which may be used to guide the user of the ultrasound imaging system 100 to use the same or similar parameters in the subsequent imaging procedure, as will be described in more detail hereafter.

In some aspects, the processor 134 may utilize deep learning-based prediction networks to identify parameters of an ultrasound image, including an anatomical scan window, probe orientation, subject position, identify and location of anatomical features, and/or other parameters. In some aspects, the processor 134 may receive metrics or perform various calculations relating to the region of interest imaged or the subject's physiological state during an imaging procedure. These metrics and/or calculations may also be displayed to the sonographer or other user via the display 132.

In some aspects, the host 130 may also include a speaker 180. The speaker 180 may for example be used to provide advisory tones, beeps, or other auditory feedback to the user.

Before continuing, it should be noted that the examples described above are provided for purposes of illustration, and are not intended to be limiting. Other devices and/or device configurations may be utilized to carry out the operations described herein.

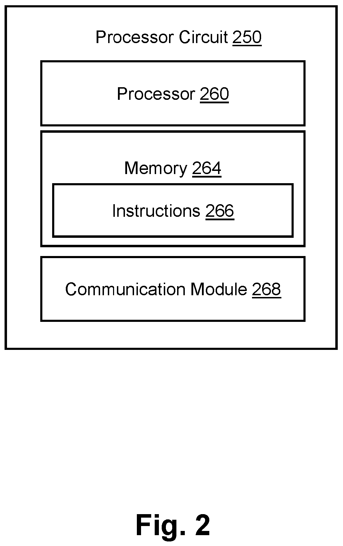

Fig. 2 is a schematic diagram of a processor circuit 250, according to aspects of the present disclosure. The processor circuit 250 may be implemented in the ultrasound imaging system 100, or other devices or workstations (e.g., third-party workstations, network routers, etc.), or on a cloud processor or other remote processing unit, as necessary to implement the method. As shown, the processor circuit 250 may include a processor 260, a memory 264, and a communication module 268. These elements may be in direct or indirect communication with each other, for example via one or more buses.

The processor 260 may include a central processing unit (CPU), a digital signal processor (DSP), a controller, or any combination of general-purpose computing devices, reduced instruction set computing (RISC) devices, application-specific integrated circuits (ASICs), field programmable gate arrays (FPGAs), or other related logic devices, including mechanical and quantum computers. The processor 260 may also comprise another hardware device, a firmware device, or any combination thereof configured to perform the operations described herein. The processor 260 may also be implemented as a combination of computing devices, e.g., a combination of a DSP and a microprocessor, a plurality of microprocessors, one or more microprocessors in conjunction with a DSP core, or any other such configuration.

The memory 264 may include a cache memory (e.g., a cache memory of the processor 260), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an aspect, the memory 264 includes a non-transitory computer-readable medium. The memory 264 may store instructions 266. The instructions 266 may include instructions that, when executed by the processor 260, cause the processor 260 to perform the operations described herein. Instructions 266 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 268 can include any electronic circuitry and/or logic circuitry to facilitate direct or indirect communication of data between the processor circuit 250, and other processors or devices. In that regard, the communication module 268 can be an input/output (I/O) device. In some instances, the communication module 268 facilitates direct or indirect communication between various elements of the processor circuit 250 and/or the ultrasound imaging system 100. The communication module 268 may communicate within the processor circuit 250 through numerous methods or protocols. Serial communication protocols may include but are not limited to United States Serial Protocol Interface (US SPI), Inter-Integrated Circuit (I²C), Recommended Standard 232 (RS-232), RS-485, Controller Area Network (CAN), Ethernet, Aeronautical Radio, Incorporated 429 (ARINC 429), MODBUS, Military Standard 1553 (MIL-STD-1553), or any other suitable method or protocol. Parallel protocols include but are not limited to Industry Standard Architecture (ISA), Advanced Technology Attachment (ATA), Small Computer System Interface (SCSI), Peripheral Component Interconnect (PCI), Institute of Electrical and Electronics Engineers 488 (IEEE-488), IEEE-1284, and other suitable protocols. Where appropriate, serial and parallel communications may be bridged by a Universal Asynchronous Receiver Transmitter (UART), Universal Synchronous Receiver Transmitter (USART), or other appropriate subsystem.

External communication (including but not limited to software updates, firmware updates, model sharing between the processor and central server, or readings from the ultrasound imaging system 100) may be accomplished using any suitable wireless or wired communication technology, such as a cable interface such as a universal serial bus (USB), micro USB, Lightning, or FireWire interface, Bluetooth, Wi-Fi, ZigBee, Li-Fi, or cellular data connections such as 2G/GSM (global system for mobiles) , 3G/UMTS (universal mobile telecommunications system), 4G, long term evolution (LTE), WiMax, or 5G. For example, a Bluetooth Low Energy (BLE) radio can be used to establish connectivity with a cloud service, for transmission of data, and for receipt of software patches. The controller may be configured to communicate with a remote server, or a local device such as a laptop, tablet, or handheld device, or may include a display capable of showing status variables and other information. Information may also be transferred on physical media such as a USB flash drive or memory stick.

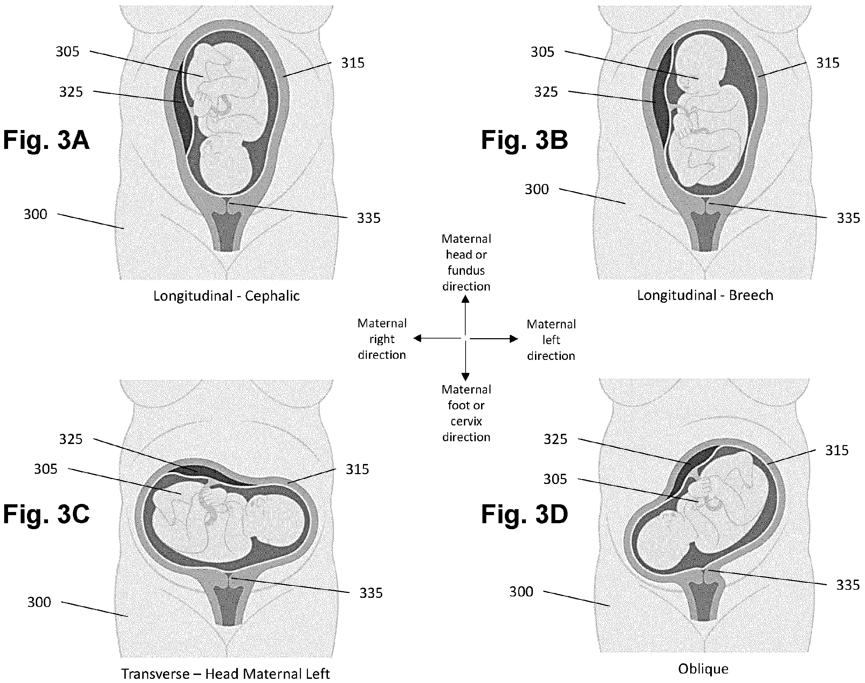

Figs. 3A-3D show a front view of the mother's body.

Fig. 3A is a schematic, diagrammatic, cross-sectional view of a fetus 305 surrounded by amniotic fluid 325 within a uterus 315 accessible through a cervix 335 of a patient 300, according to aspects of the present disclosure.

In describing fetal lie, different sources use different terms to describe how the fetus is positioned inside uterus. Sometimes different sources use terms like fetal lie, fetal presentation, fetal positioning, etc. in different ways and/or interchangeably. For purposes of this document, fetal lie is the axis/dimension along with a majority of the length of the fetus (e.g., crown to rump length) extends; this could also be described axis/dimension that the fetus is orientated relative to the mother's spine (where the mother's spine is taken as kind of a longitudinal reference line). With a longitudinal lie, the length of the fetal body extends along the axis including the maternal head/fundus and maternal foot/cervix directions. Two types of longitudinal lie include cephalic (head down) and breech (head up). With a transverse lie, the length of the fetal body extends along the axis of the maternal right and left directions (e.g., perpendicular, approximately 90 degrees relative to the axis of the maternal head/fundus and maternal foot/cervix directions). Two types of longitudinal lie include head on maternal left (shown in Fig. 3C), head on maternal right (head would be on opposite side of what is shown in Fig. 3C). With an oblique lie, the length of the fetal body extends at oblique angle (non-zero, non-90 degrees) relative to both the maternal fundal-caudal axis and the maternal left-right axis.

In the example shown in Fig. 3A, the fetus is in a longitudinal-cephalic lie. Determining the lie of the fetus is one object of the present disclosure.

Fig. 3B is a schematic, diagrammatic, cross-sectional view of a fetus 305 surrounded by amniotic fluid 325 within a uterus 315 accessible through a cervix 335 of a patient 300, according to aspects of the present disclosure. In the example shown in Fig. 3B, the fetus is in a longitudinal-breech lie. Determining the lie of the fetus is one object of the present disclosure.

Fig. 3C is a schematic, diagrammatic, cross-sectional view of a fetus 305 surrounded by amniotic fluid 325 within a uterus 315 accessible through a cervix 335 of a patient 300, according to aspects of the present disclosure. In the example shown in Fig. 3A, the fetus is in a transverse - head maternal left lie. Determining the lie of the fetus is one object of the present disclosure.

Fig. 3D is a schematic, diagrammatic, cross-sectional view of a fetus 305 surrounded by amniotic fluid 325 within a uterus 315 accessible through a cervix 335 of a patient 300, according to aspects of the present disclosure. In the example shown in Fig. 3A, the fetus is in an oblique lie. Determining the lie of the fetus is one object of the present disclosure.

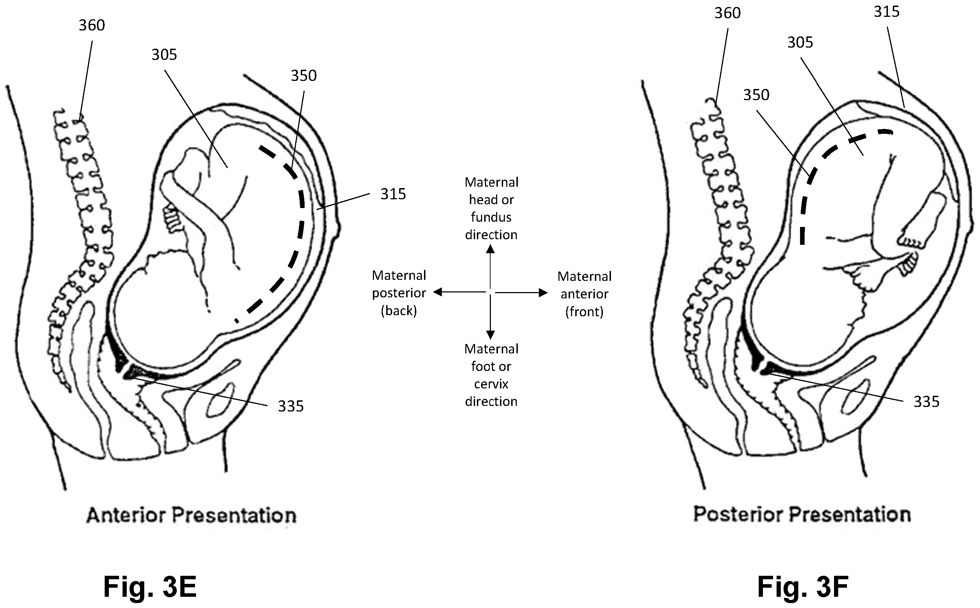

Figs. 3E and 3F are side views of the mother's body (rotated 90 degrees relative to front views shown in Figs. 3A-3D).

Fig. 3E is a schematic, diagrammatic, cross-sectional view of a fetus 305 within a uterus 315 accessible by a cervix 335, according to aspects of the present disclosure.

As described above, different sources use different terms to describe how the fetus is positioned inside uterus. Sometimes different sources use terms like fetal lie, fetal presentation, fetal positioning, etc. in different ways and/or interchangeably. For purposes of this document, fetal presentation refers to the direction the fetus' spine is facing relative to the anterior-posterior axis of the mother. Thus, the maternal posterior (inferior) direction means fetal spine facing toward the maternal back or maternal spine, while the maternal anterior (superior) direction means fetal spine facing toward the maternal front or maternal abdomen/belly (e.g., where the ultrasound probe is positioned to perform ultrasound imaging during the blind sweep protocol).

Thus, the present disclosure describes where the fetus's spine is, in relation to the mother's anatomy (e.g., mother's spine or the mother's abdomen/belly). Other sources describe this as the direction that the fetus' front is facing when inside the uterus (e.g., anterior is facing the mother's spine, while posterior is facing the mother's belly). Thus, fetal presentation includes two types: anterior (fetal spine is positioned proximate to maternal anterior/front; fetal spine is positioned opposite to mother's spine) and posterior (fetal spine is positioned proximate to maternal posterior/back; fetal spine is positioned towards mother's spine).

In the example shown in Fig. 3E, the fetus is in an anterior presentation, with the fetal spine 350 facing away from the maternal spine 360.

Fig. 3F is a schematic, diagrammatic, cross-sectional view of a fetus 305 within a uterus 315 accessible by a cervix 335, according to aspects of the present disclosure. In the example shown in Fig. 3F, the fetus is in a posterior presentation, with the fetal spine 350 facing toward the maternal spine 360.

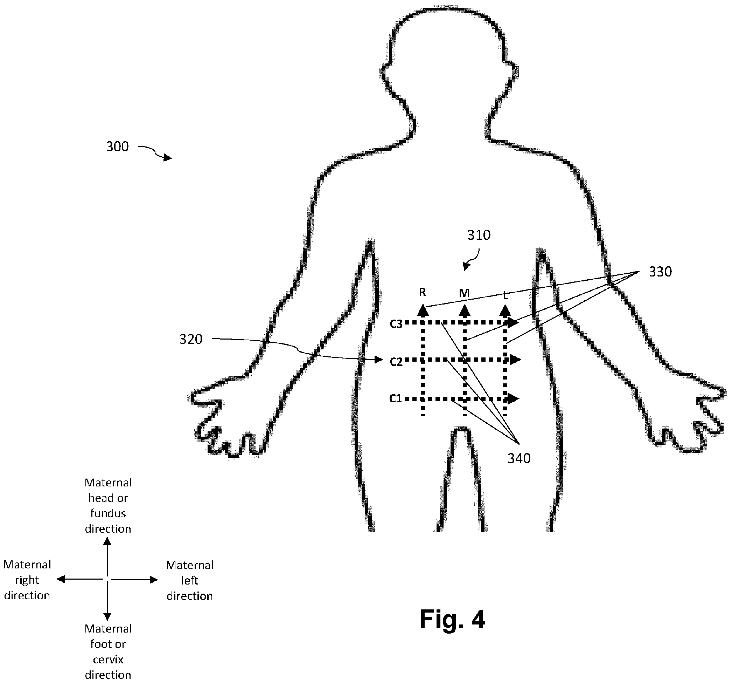

Fig. 4 is a schematic, diagrammatic representation of a patient 300, according to aspects of the present disclosure. Visible on the abdomen 310 of the patient 300 is a desired sweep pattern 320 intended to capture images of desired features of the patient's anatomy. The sweep pattern 320 includes multiple vertical sweep lines 330 and multiple horizontal sweep lines 340. Each sweep line 330, 340 represents a desired path for one imaging sweep of the abdomen 310. In the example shown in Fig. 4, the sweep pattern includes three vertical sweep lines 330 labeled L (patient's left), M (patient's middle), and R (patient's right), all in an upward direction with respect to the patient, and three horizontal sweep lines 340 labeled C1 (bottom), C2 (middle), and C3 (top), all in a right-to-left direction with respect to the patient. However, it is understood that a sweep pattern 320 may include more or fewer sweep lines 330, including vertical sweep lines 330, horizontal sweep lines 330, or combinations thereof, in any combination of upward, downward, left, or right directions, based on the patient's fundal height. For example, if the patient's belly is bigger in size, more sweeps may be needed. Furthermore, a sweep pattern 320 may cover other portions of the patient's body, including but not limited to the head, neck, spine, limbs, etc. Examples of blind sweep protocol include but are not limited to obstetric sweep imaging (OSI), volume sweep imaging (VSI), 6-Stage, Fetal Age Machine Learning Initiative (FAMLI), and Philips.

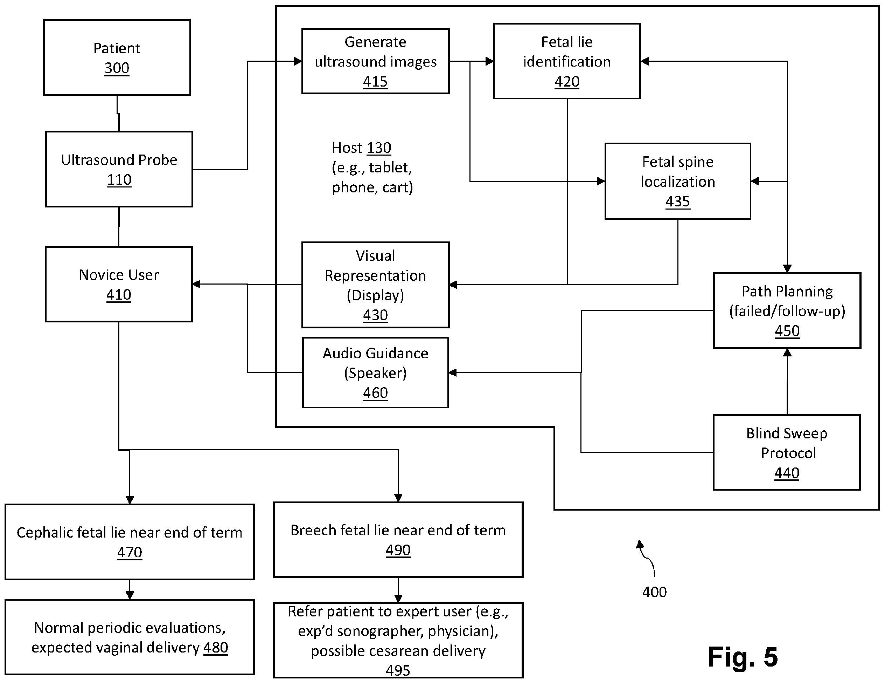

These sweep patterns represent desired probe motion information, including desired positions, a desired velocity or velocities, and/or a desired orientation of the ultrasound probe while the ultrasound probe is obtaining a plurality of ultrasound image frames during the sweep. It is noted that the desired sweep patterns or blind sweep protocols stored in a memory of the processor may include only vertical sweeps, only horizontal sweeps, may include a grid (e.g., 3x3, 5x5, etc.) of vertical and horizontal sweeps, and may also include associated parameters such as desired probe motion (e.g., positions, velocities, and/or orientations) stored in the memory (e.g., blind sweep protocol 440 in Fig. 5). Depending on the implementation, sweeps may include curved, diagonal, and other types of sweeps. The protocols and their associated parameters can for example be based on standards established by authorities in the field (physician organizations, sonographer organizations, etc.), published in scholarly journals/textbooks, etc.

Fig. 5 is a schematic, diagrammatic representation, in hybrid block diagram/flow diagram form, of an example multiple gestation analysis system 400, according to aspects of the present disclosure. An ultrasound probe 110 operated by a novice user 410 performs a blind sweep protocol 440 on the body of a patient 300 and sends ultrasound imaging data to a host 130, such as a tablet, smartphone, ultrasound cart, etc. In step 415, the host 130 generates ultrasound images using the ultrasound image data obtained by the ultrasound probe 110. The host 130 can control the ultrasound probe 110 to obtain the ultrasound image data (e.g., the host 130 establishes communication with the ultrasound probe 110, the host 130 sends control signals to start and/or stop acquisition of ultrasound image data, the host 130 sends power signals to power the ultrasound probe 110, etc.).

In step 420, the host 130 uses the ultrasound images generated in step 415 and/or the ultrasound image data used to generate the ultrasound images to determine the fetal lie, as described in more detail below. In step 435, the host 130 uses the ultrasound images generated in step 415 and/or the ultrasound image data used to generate the ultrasound images to determine the location of the fetal spine, as described in more detail below. In step 430, the host generates a visual representation and outputs the visual representation on a display (e.g., display 132 of Fig. 1), showing the fetal lie and/or fetal presentation.

In step 450, the host 130 determines whether the planned path of the blind sweep protocol 440 has been followed adequately. If no, execution proceeds to steps 430 and 460, wherein the host 130 provides feedback (e.g., audio feedback through a speaker and/or visual feedback from a display) to repeat one or multiple sweeps of the blind sweep protocol 440. In some aspects, the determination in step 450 can be based on the results of analysis in step 420 of the fetal lie and/or in step 435 of the fetal spine's location. For example, if there is insufficient ultrasound image data to perform the determinations in step 420 and/or step 435 or if the ultrasound image data that has been obtained is not suitable to perform the determinations in step 420 and/or step 435, then step 450 can determine that the one or multiple sweeps of the blind sweep protocol 440 has not been completed correctly. The one or multiple sweeps that need to be performed again can be communicated to the user through visual or audio feedback in steps 430 and 460.

In some aspects, the planned path determined in step 450 of the blind sweep protocol 440 can be used by the host 130 to determine the fetal lie in step 420 and/or the location of the fetal spine in step 435. For example, the planned path determined in step 450 can provide absolute or relative spatial information (e.g., vertical location, horizontal location) about the ultrasound image data obtained by the ultrasound probe and/or the ultrasound images generated by the host. For example, referring again to Fig. 4, the planned path determined in step 450 can indicate that the M sweep is horizontally located (left-right direction in Fig. 4) between the R sweep and the L sweep. The host 130 can use this spatial information from step 450 to process the ultrasound image data and/or ultrasound images to determine the fetal lie in step 420 and/or the location of the fetal spine in step 435.

Both fetal lie and fetal spine location can provide helpful information related delivery. For example, a cephalic (head down) lie with, fetal spine anterior presentation (baby facing mother's spine) may be ideal for vaginal delivery. Conversely, a cephalic (head down), fetal spine posterior (baby facing mother's belly) may be less ideal, but vaginal delivery may still be possible, whereas with a breech (head up) like, cesarean delivery will likely be considered.
Based on the visual representation 430, the novice user 410 makes a triage decision to either rule out or rule in a breech fetal lie. In step 470, the novice user 410 rules out breech lie (e.g., determines that the fetus is in a cephalic lie), and in step 480, the novice user 410 performs or recommends normal periodic evaluations for the patient 300. In step 490, the novice user 410 rules in breech fetal lie, and in step 495, the novice user 410 refers the patient to an expert user such as an experienced sonographer, radiologist, obstetrician, or other physician for follow-up imaging and diagnosis.

Flow diagrams and block diagrams are provided herein for exemplary purposes; a person of ordinary skill in the art will recognize myriad variations that nonetheless fall within the scope of the present disclosure. For example, any of the steps described herein may optionally include an output to a user of information relevant to the step, and may thus represent an improvement in the user interface over existing art by providing information not otherwise available. Similarly, block diagrams may show a particular arrangement of components, modules, services, steps, processes, or layers, resulting in a particular data flow. It is understood that some aspects of the systems disclosed herein may include additional components, that some components shown may be absent from some aspects, and that the arrangement of components may be different than shown, resulting in different data flows while still performing the methods described herein. The logic of flow diagrams may be shown as sequential. However, similar logic could be parallel, massively parallel, object oriented, real-time, event-driven, cellular automaton, or otherwise, while accomplishing the same or similar functions. In order to perform the methods described herein, a processor may divide each of the steps described herein into a plurality of machine instructions, and may execute these instructions at the rate of several hundred, several thousand, several million, or several billion per second, in a single processor or across a plurality of processors. Such rapid execution may be necessary in order to execute the method in real time or near-real time as described herein. For example, to provide an assessment of the fetal lie and/or fetal presentation, the system may need to generate the visual representation 430 within one second of completing the blind sweep protocol.

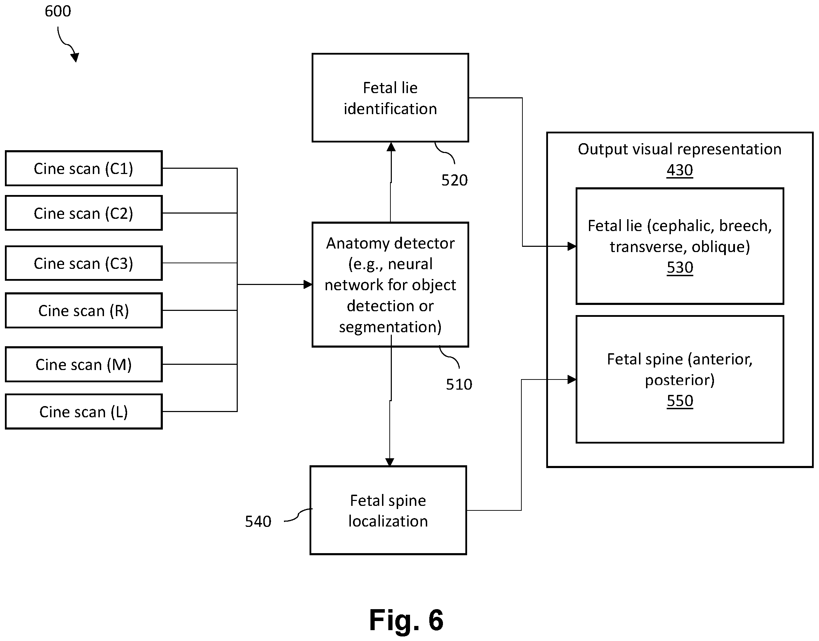

Fig. 6 is a schematic, diagrammatic representation, in hybrid block diagram/flow diagram form, of an example fetal orientation determination method 600 to triage and/or monitor fetal lie and presentation, according to aspects of the present disclosure. Images from a sweep of the blind sweep protocol can be organized into an image sequence known as a cine scan or cineloop (e.g., multiple image frames making up a video segment). The host thus receives or assembles cine scans C1, C2, C3, R, M, and L, corresponding to the blind abdominal sweeps C1, C2, C3, R, M, and L shown in Fig. 6.

In step 510, these cine scans are fed into an object detector (e.g., a trained neural network) to detect the presence of and, if present, the location of the anatomical region of interest (e.g., fetal head, fetal spine, etc.) in each ultrasound image frame of the cine scan. Depending on the implementation, execution then proceeds to either or both of steps 520 or 540.

In step 520, the method includes determining (e.g., using heuristics and/or a trained classifier) the fetal lie within the uterus, and generating an output visual representation 430 that includes a visual or text representation of the fetal lie 530.

In step 540, the method includes (e.g., using a heuristic anomaly detector and/or a trained classifier) assessing whether the fetal spine is in an anterior or posterior presentation, and generating an output visual representation 550 indicative of the fetal spine presentation.

Depending on the implementation, the visual representation 430 can include text labels or images/graphics (including both not limited to the images/graphics described herein), and/or combinations or thereof. The anatomy detector 510 can identify fetal anatomy (fetal head, fetal spine, fetal heart, fetal abdomen, fetal urinary bladder) and/or maternal anatomy (placenta, amniotic fluid, maternal spine, etc.). Inputs to the anatomy detector 510 are ultrasound image frames from horizontal sweep(s) and/or vertical sweep(s). For example, each sweep can be a cine scan (e.g., an ultrasound video made up of multiple ultrasound image frames). The output of the anatomy detector 510 can depend on the type of predictive model/network being used. For an objection detection neural network, the output may include one or more bounding boxes identifying anatomy. For a segmentation neural network, the output may include the contour or perimeter of the anatomy.

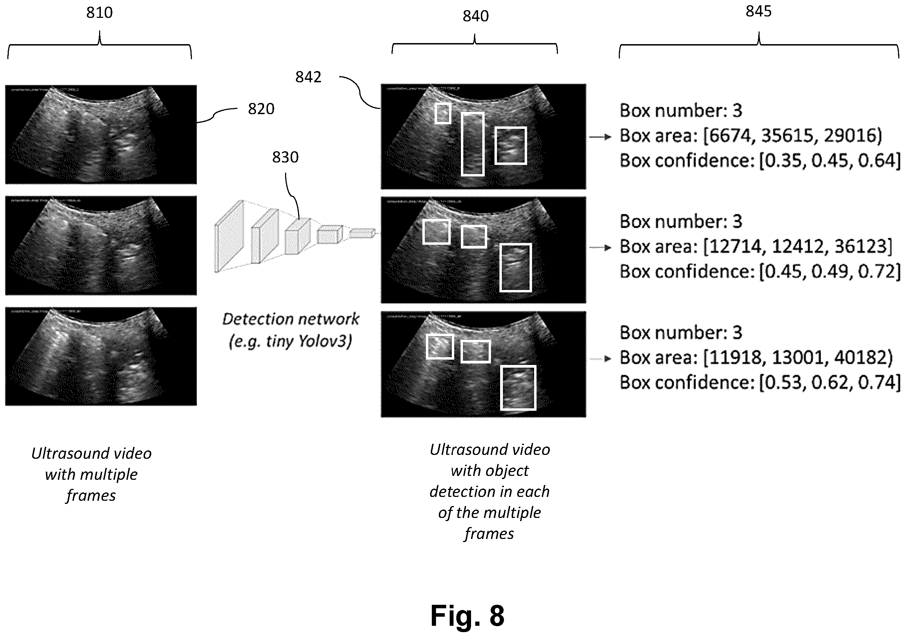

The anatomy detector may use available networks for object detection (e.g., YOLO, R-CNN, R-FCN, or otherwise as described below in Fig. 8.

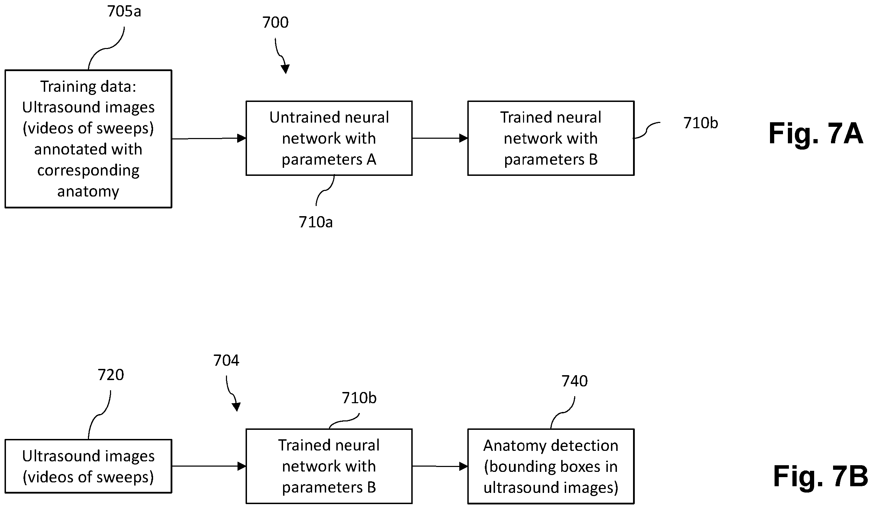

Fig. 7A is a schematic, diagrammatic overview, in block diagram form, of a training mode 700 for an untrained neural network 710a, according to aspects of the present disclosure. In the example shown in Fig. 7A, a set of training data 705a includes ultrasound cineloops of probe sweeps annotated with the corresponding anatomy (placenta, fetal head, fetal spine, etc.). The training data 705a is fed into an untrained neural network 710a in an iterative training process that will be familiar to a person of ordinary skill in the art.

The parameters of a network model (e.g., the weights at each artificial neuron) are initialized with initial values A that may be random values or with results from training on prior datasets. In an iterative process, the network is used to make detection inferences on the training images, the results are compared with the ground truth annotations, and an optimizer is used to adjust the network parameters B until a metric of accuracy is maximized.

Thus, an output of this training process 700 is a trained neural network 710b, wherein the parameters B (e.g., weights) are optimized for generating accurate bounding boxes for the anatomy imaged in the training data 705a.

Fig. 7B is a schematic, diagrammatic overview, in block diagram form, of an inference mode or clinical usage mode 704 for the trained neural network 710b, according to aspects of the present disclosure. In clinical usage, an ultrasound video, cineloop, or cine sweep 720 of the blind sweep is fed to the trained and validated neural network 710b for analysis. The trained and validated neural network 710b then produces, as an output, anatomy detection bounding boxes 740 for each image (or the entire sweep). In some aspects, a confidence value can be determined as a normalized value in the range [0-1], where 0 indicates lowest confidence, and 1 indicates highest confidence that the detection is correct.

Fig. 8 is a schematic, diagrammatic illustration, in block diagram form, of the detection of anatomy (e.g., the placenta, fetal head, fetal spine, etc.), according to aspects of the present disclosure. A cineloop 810 comprising multiple frames 820 is fed into a trained object detector 830.

The object detector 830 may implement or include any suitable type of learning network. For example, in some aspects, the object detector 830 could include a neural network, such as a convolutional neural network (CNN). In addition, the convolutional neural network may additionally or alternatively be an encoder-decoder type network, or may utilize a backbone architecture based on other types of neural networks, such as an object detection network, classification network, etc. One example backbone network is the Darknet YOLO backbone, (e.g., Yolov3) which can be used for object detection. The CNN may for example include a set of N convolutional layers, where N may be any positive integer. Fully connected layers can be omitted when the CNN is a backbone. The CNN may also include max pooling layers and/or activation layers. Each convolutional layer may include a set of filters configured to extract features from an input (e.g., from a frame of the ultrasound video). The value N and the size of the filters may vary depending on the aspects. In some instances, the convolutional layers may utilize any non-linear activation function, such as for example a leaky rectified non-linear (ReLU) activation function and/or batch normalization. The max pooling layers gradually shrink the high-dimensional output to a dimension of the desired result (e.g., bounding boxes of a detected feature). Outputs of detection network may include numerous bounding boxes, with most having very low confidence scores and thus being filtered out or ignored. Fully connected layers may be referred to as perception or perceptive layers. In some aspects, perception/perceptive and/or fully connected layers may be found in object detector 830 (e.g., a multi-layer perceptron).

These descriptions are included for exemplary purposes; a person of ordinary skill in the art will appreciate that other types of learning models, with features similar to or dissimilar to those described above, may be used instead or in addition, without departing from the spirit of the present disclosure.

Outputs of the object detector 830 may include an annotated cineloop 840 made up of a plurality of annotated image frames 842, possibly including per-frame metrics 845 such as the confidence level of the detections.

The systems and methods disclosed herein are broadly applicable to different types of features, and can for example draw boxes around the placenta, fetal spine, fetal head, or other anatomical features depending on the implementation. The object detector can be one class or multi-class, depending how the model is built. If another detector is trained separately, then both models can be run separately (e.g., one model for each feature type). Otherwise, multiple feature classes can be identified, and enclosed in detection boxes, at the same time. The ML model for placenta detection can use exactly the same structure as a model for cervix detection. One can either train/run a single detector that detects multiple feature types (a "multi-class detector") and provides their locations as an output, along with the confidence score and feature type (class) of each detection. Alternatively, one could run several "single-class" detectors, each trained to detect a single feature type/class. These separate single-class detectors may in some cases have the same architecture (e.g., layers and connections), but may have been trained with different data (e.g., different images and/or annotations).

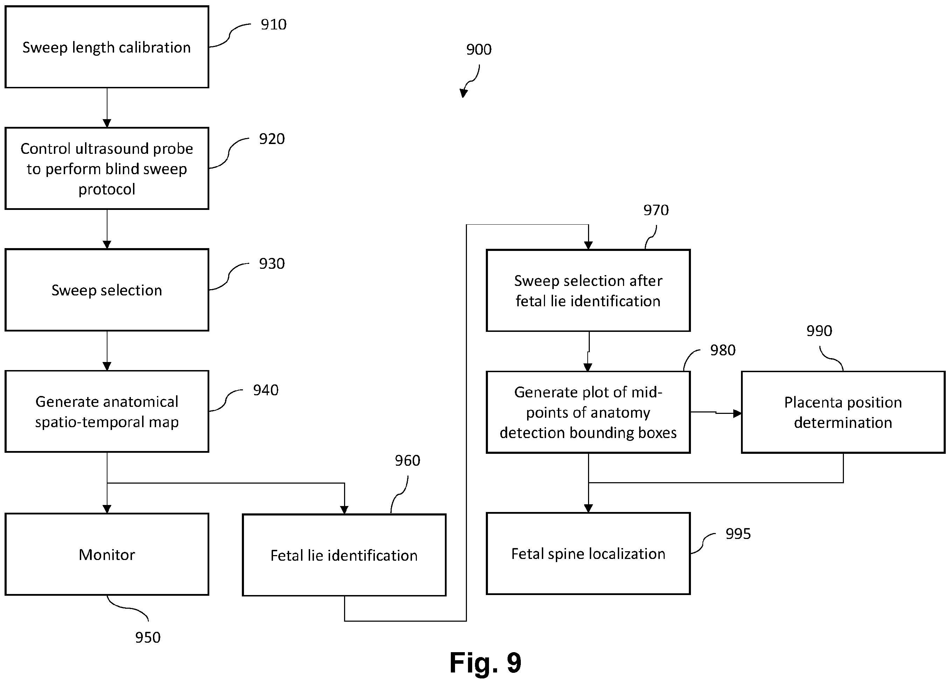

Fig. 9 is a schematic, diagrammatic representation, in flow diagram form, of an example fetal orientation determination method 900, according to aspects of the present disclosure.

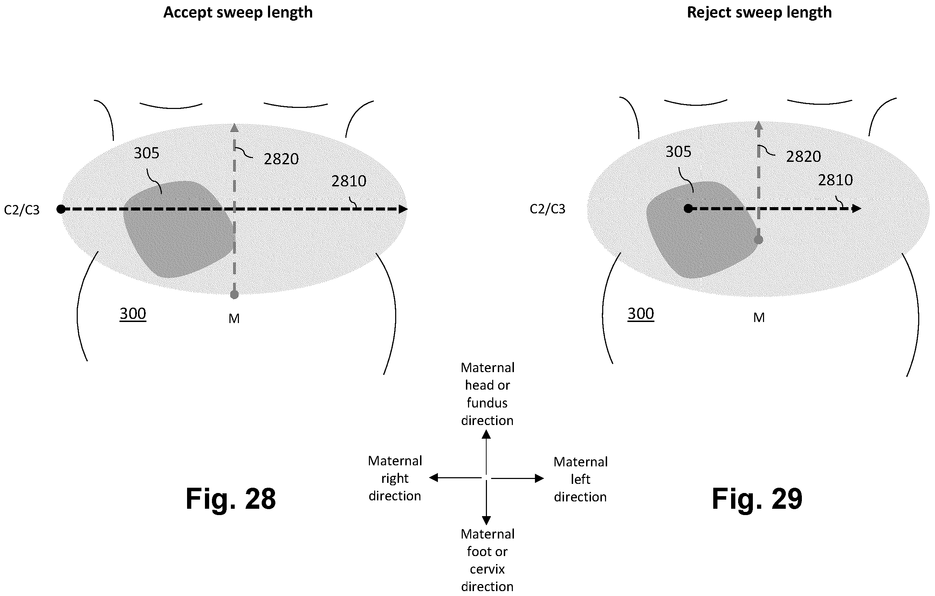

In step 910, the method 900 includes a sweep length calibration step, as described in detail in Figs. 28-31, below. Execution then proceeds to step 920.

In step 920, the method 900 includes controlling the ultrasound probe to perform the blind sweep protocol, as described in Fig. 4, above. Execution then proceeds to step 930.

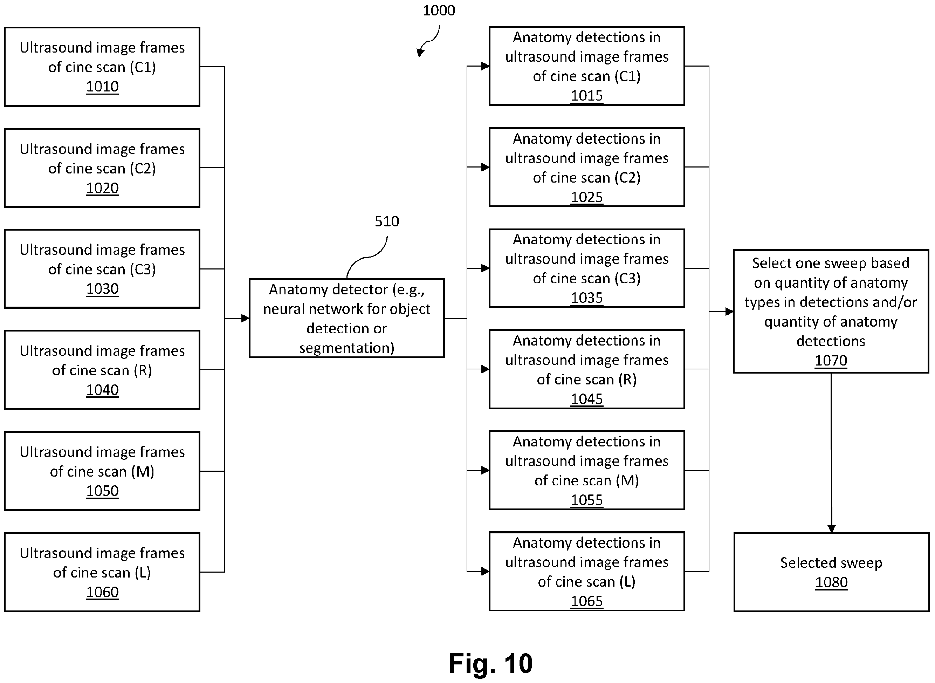

In step 930, the method 900 includes selecting a sweep from the blind sweep protocol for determining fetal lie and fetal presentation, as described in detail in Fig. 10, below. Execution then proceeds to step 940.

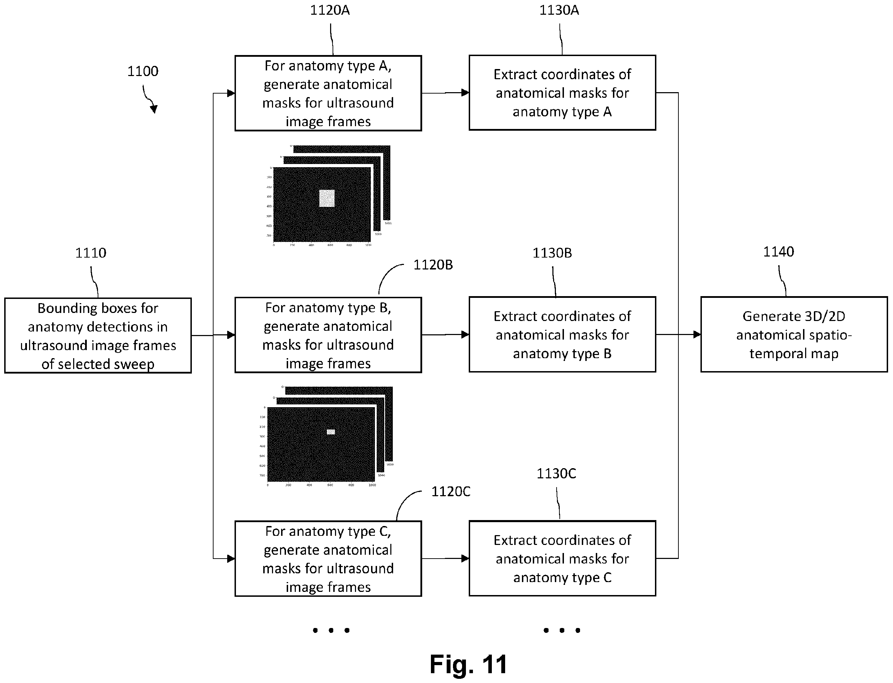

In step 940, the method 900 includes generating a spatio-temporal map, as described below in Figs. 11-15B. Execution then proceeds to steps 950 and 960.

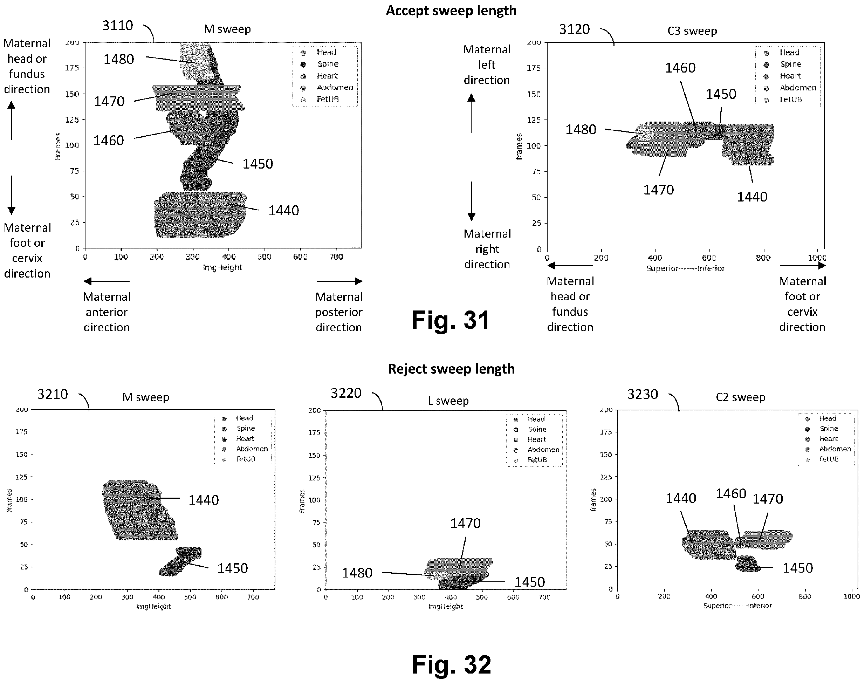

In step 950, the method includes monitoring the growth and movement of the fetus across different gestational ages, as described for example in Fig. 32, below. The method 900 is now complete.

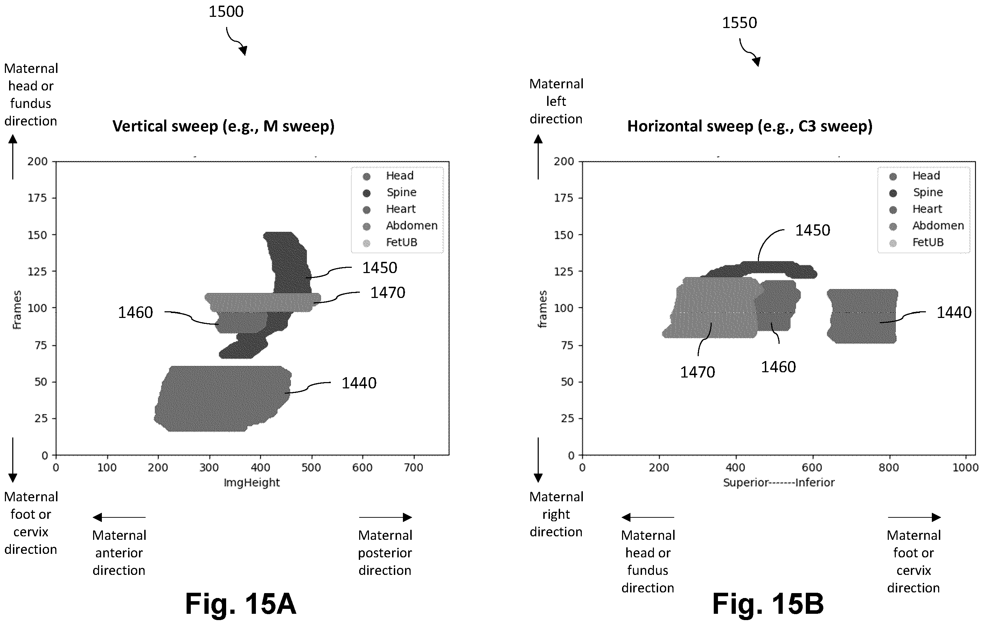

In step 960, the method 900 includes determining the fetal lie, as described in detail in Figs. 15-23, below. Execution then proceeds to step 970.

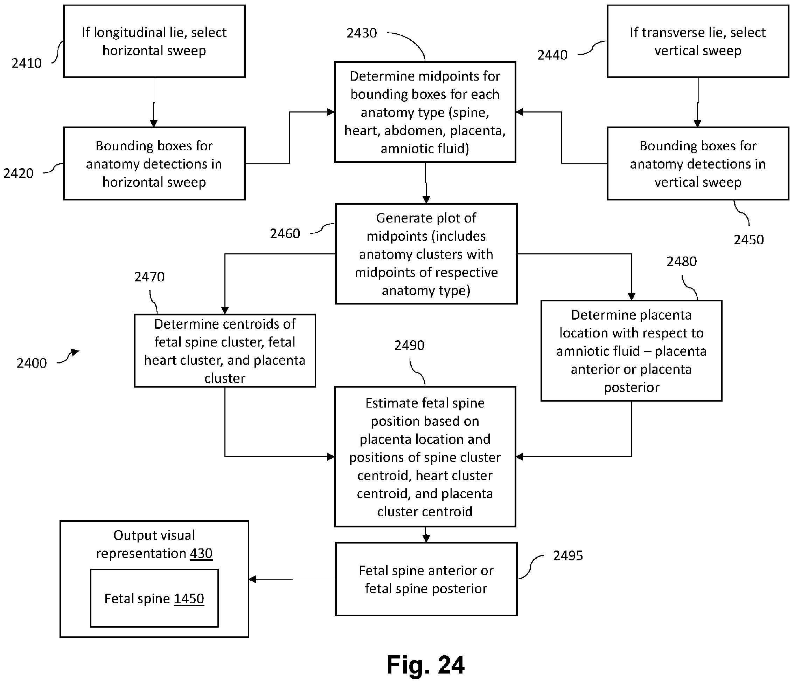

In step 970, the method 900 includes selecting a sweep after fetal lie identification, as described in detail in Fig. 24, below. Execution then proceeds to step 980.

In step 980, the method 900 includes generating a plot of the midpoints of the anatomy detection bounding boxes, as described in detail in Figs. 24-27C. Execution then proceeds to steps 995 or, optionally, to step 990.

In step 990, the method 900 includes determining the position of the placenta, as described for example in Fig. 24, below, and in U.S. Provisional Application No. 63/611,810, filed December 19, 2023, and titled "Low-Lying Placenta and/or Placenta Location in Ultrasound Imaging with Blind Sweep Protocol", incorporated by reference as though fully set forth herein. Execution then proceeds to step 995.

In step 995, the method 900 includes localizing the fetal spine, as described in detail in Figs. 24-27C, below. The method 900 is now complete.

Fig. 10 is a schematic, diagrammatic representation, in hybrid flow diagram / bloc diagram form, of an example fetal orientation determination method 1000, according to aspects of the present disclosure. Ultrasound image frames 1010, 1020, 1030, 1040, 1050, and 1060 (from scans C1, C2, C3, R, M, and L, respectively) are fed through the object detector 510 to yield anatomy detections 1015, 1025, 1035, 1045, 1055, and 1065 for scans C1, C2, C3, R, M, and L, respectively.

To obtain the image frames 1010-1060, the system must control the ultrasound probe to perform the blind sweep protocol, including multiple sweeps (e.g., multiple horizontal sweeps, multiple vertical sweeps). Selection of one sweep among multiple sweeps of the blind sweep protocol may for example be done to reduce computational complexity and/or speed up processing time. In some aspects, the system may select multiple sweeps (though less than all sweeps of blind sweep protocol). In still other aspects (e.g., where sufficient computing power and memory are available), the system may use all sweeps.

One example of selecting one sweep uses a count-based determination - based on a quantity of anatomy types in detections (e.g., which sweep has the most anatomy types) and/or the quantity of detections (e.g., total detections and/or detections of particular anatomy types). Depending on the implementation, all sweeps can be considered or can be hierarchical/preferential. For example, the system can prefer vertical sweeps, and may select a horizontal sweep only if no acceptable vertical sweep is available.

In an example, the system starts with the M sweep, and determines if the quantity of anatomy types and/or quantity of detections in that sweep satisfies certain threshold value(s). If so, the system may choose the M sweep. However, if the threshold value(s) are not satisfied, then the system may consider the L sweep and R sweep, and may choose whichever one has the greater quantity of anatomy types and/or quantity of detections.

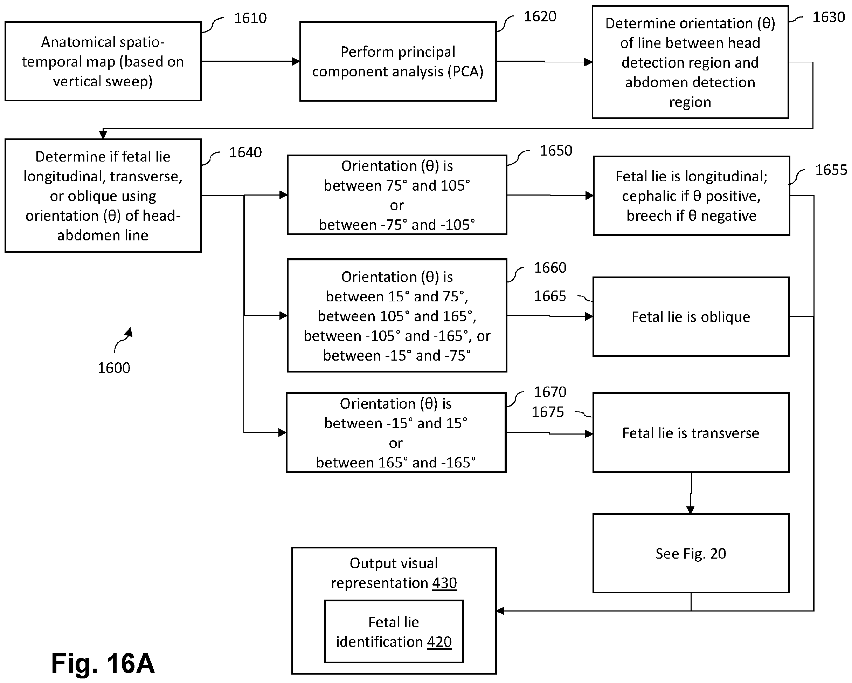

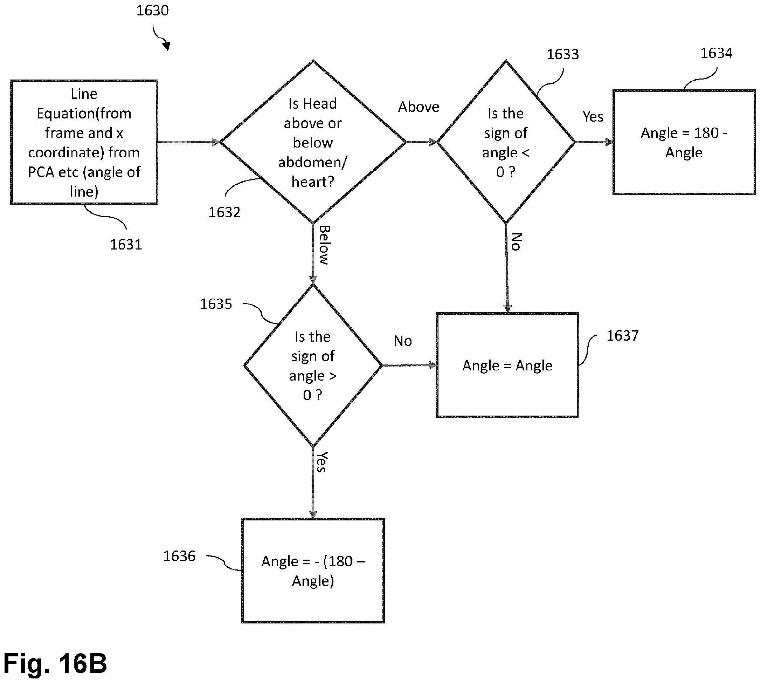

Based on experimental data, it is expected that a vertical sweep (in particular, the middle vertical sweep or M sweep) is going to be the most frequently selected, because experimental data shows that vertical sweeps (e.g., the M sweep) usually have the most anatomy types and/or detections (regardless of which of fetal lie). However, it could be any sweep that is selected and, as describe in Fig. 16, the system may in fact need to use both a vertical sweep and a horizontal sweep to construct the 3D/2D spatio-temporal model.

Fig. 11 is a schematic, diagrammatic representation, in hybrid block diagram / flow diagram form, of an example 3D/2D spatio-temporal map generation method 1100, in accordance with aspects of the present disclosure.

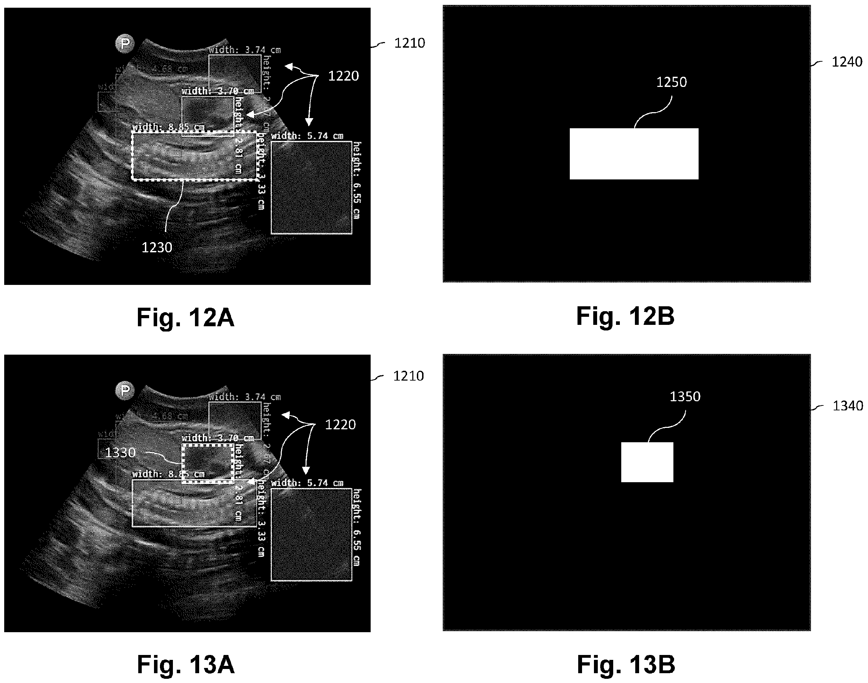

In steps 1120A 1120B, and 1120C, etc., the method 1100 includes receiving the bounding boxes 1110 for anatomy detections in the ultrasound image frames of the selected sweep, and generating masks that cover the respective bounding boxes for anatomy type A, type B, type C, etc., as shown below in Figs. 12A-13B. Execution then proceeds to steps 1130A, 1130B, 1130C, etc.

In steps 1130A, 1130B, 1130C, etc., the method 1100 includes extracting coordinates of the anatomical masks for anatomy type A, type B, type C, etc. Execution then proceeds to step 1140.

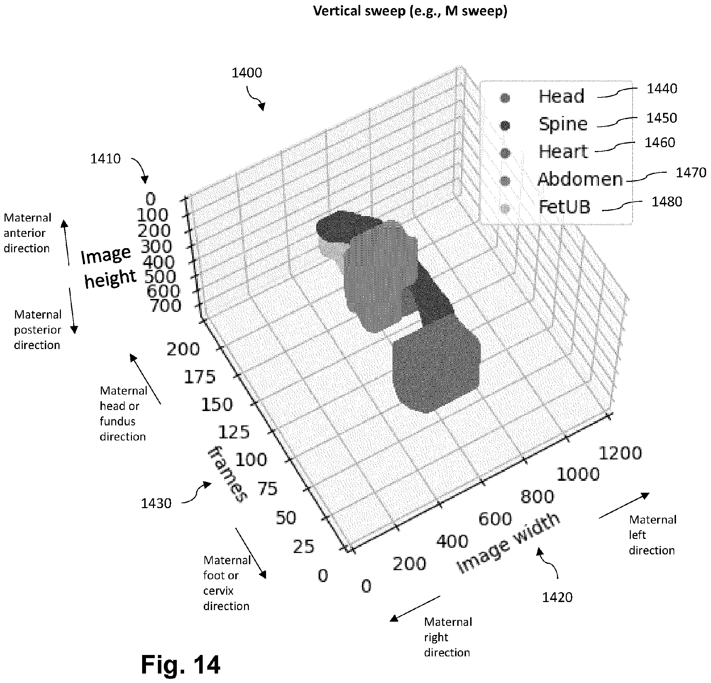

In step 1140, the method 1100 includes generating a 3D anatomical spatio-temporal map, by placing each mask in an appropriate location based on its frame number and position within the frame, as shown below in Fig. 14. The method 1100 is now complete.

Thus, the method 1100 starts with the output of the anatomy detector (e.g., a neural network for object detection). In an example, the selected sweep includes 200 frames. Some frames could have no detections, some frames could have detections of only anatomy type A, some frames could have detections of only anatomy type B, some frames could have detections of both anatomy types A and B, etc.. However, in the example, 80 of those frames have detections of anatomy type A (e.g., fetal spine), 70 of those frames have detection of anatomy type B (e.g., fetal heart) and so on. The same 200 frames from the selected sweep are then provided to the mask generation step 1130A for anatomy type A, mask generation step 1130B for anatomy type B, etc. This means that, for a single ultrasound image frame, multiple masks can be generated.

The same frames are processed differently because mask generation for anatomy type A is focusing on detections of anatomy type A, mask generation for anatomy type B is focusing on detections of anatomy type B. This is described more in Figs. 12B and 13B. In the example, the mask is binary - some pixels/area have a value of 1, and some pixels/area have a value of 0.

Fig. 12A is an ultrasound image frame 1210 with anatomy detection boxes 1220, including an anatomy detection box 1230 for fetal anatomy type A (e.g., the fetal spine), according to aspects of the present disclosure. Each bounding box 1220 is an output of the anatomy detector and represents a different anatomy, such as the head, heart, abdomen, etc. Locations of the bounding boxes can be used to generate masks, which are then used to generate the 3D spatio-temporal anatomy map.

Fig. 12B is a fetal spine mask 1240 generated from the ultrasound image frame 1210 of Fig. 12A, according to aspects of the present disclosure. The mask 1240 includes a "white" or "1" portion 1250 and a "black" or "0" portion 1260. The white portion 1250 fully and exclusively covers the fetal spine bounding box 1230 of Fig. 12A, such that each pixel located within the bounding box 1230 is colored white (e.g., a numerical value of 1), and each pixel located outside of the bounding box 1230 is colored black (e.g., a numerical value of 0). This is repeated for all ultrasound images frames of the selected sweep and for all anatomy types. The white portions 1250 of successive masks in a sweep can then be used to create the "spine" portion of a 3D spatio-temporal map, as shown below in Fig. 14.

The coordinates of the white rectangle 1250 are the same as those of the corresponding bounding box 1230 in Fig. 12A. As described in more detail in Figs. 14 and 15, the x dimension and y dimension of the white portion 1250 will depend on whether the ultrasound image frame is from a vertical sweep or a horizontal sweep. The size and location of the white portion also depends on the size and location of the fetal spine in each successive image frame of the sweep. Frames that do not include the fetal spine will not have a white portion 1250 for the mask 1240 (e.g., the mask 1240 will be entirely black).

Fig. 13A is an ultrasound image frame 1210 with anatomy detection boxes 1220, including an anatomy detection box 1330 for fetal anatomy type B (e.g., the fetal heart), according to aspects of the present disclosure. Each bounding box 1220 is an output of the anatomy detector and represents a different anatomy, such as the head, heart, abdomen, etc. Locations of the bounding boxes can be used to generate masks, which are then used to generate the 3D spatio-temporal anatomy map.

Fig. 13B is a fetal heart mask 1340 generated from the ultrasound image frame 1210 of Fig. 13A, according to aspects of the present disclosure. The mask 1340 includes a "white" or "1" portion 1350 and a "black" or "0" portion 1360. The white portion 1350 fully and exclusively covers the fetal heart bounding box 1330 of Fig. 13A, such that each pixel located within the bounding box 1330 is colored white (e.g., a numerical value of 1), and each pixel located outside of the bounding box 1330 is colored black (e.g., a numerical value of 0). The white portions 1350 of successive masks in a sweep can then be used to create the "heart" portion of a 3D spatio-temporal map, as shown below in Fig. 14.

Fig. 14 is a 3D anatomical spatio-temporal map 1400 of the fetal anatomy from a vertical sweep (e.g., an M sweep), according to aspects of the present disclosure. The spatio-temporal map includes three coordinate axes: the image height axis 1410 (spanning the maternal anterior-posterior axis), image width axis 1420 (spanning the material left-right axis), and frame number 1430 (spanning the maternal cranial-caudal axis or head-foot axis). Anatomy detections 1499 are filled in by adding each bounding box (e.g., the white portions of each mask) for each anatomy type to each frame number 1430 of the map. In the example shown in Fig. 14, frame number 100 contains a rectangle representing a detection mask or bounding box representing the fetal head 1440, whereas frame number 150 contains rectangles representing detection masks or bounding boxes for the abdomen 1470, heart 1460, and spine 1450, and frame number 175 contains rectangles representing the fetal urinary bladder 1480 and spine 1450. Thus, by summing the masks for each anatomy type and frame number, the 3D anatomical spatio-temporal map 1400 generates a 3D representation of the locations of fetal anatomy within the patient's body, which can then be used to calculate the fetal lie and/or fetal presentation. For example, in the example shown in Fig. 14, the fetus is in a longitudinal-cranial lie (see Fig. 3A), with anterior fetal spine presentation (see Fig. 3E). However, the 3D spatio-temporal map 1400 does not include details that may be visible in a raw ultrasound image, such as the sex of the fetus.

In some aspects, a 2D projection of the 3D spatio-temporal map 1400 can be displayed to the user. The system may include a user input to rotate the map 1400 to different viewing angles, and/or a user input to turn on/off anatomy detection regions (e.g., turn off placenta region and amniotic fluid region if user only wants to see fetal anatomies).

Fig. 15A is a 2D anatomical spatio-temporal map 1500 for a vertical sweep (e.g., an M sweep), according to aspects of the present disclosure. In the example shown in Fig. 15A, the 2D anatomical spatio-temporal map 1500 is a projection or cross-section of the 3D anatomical spatio-temporal map 1400 of Fig. 14, in the frame number / image height plane. Visible are the fetal head 1440, fetal spine 1450, fetal heart 1460, and fetal abdomen 1470, in a longitudinal, cephalic lie (see Fig. 3A).

Fig. 15B is a 2D anatomical spatio-temporal map 1500 for a horizontal sweep (e.g., a C2 or C3 sweep), according to aspects of the present disclosure. In the example shown in Fig. 15B, the 2D anatomical spatio-temporal map 1500 is a projection or cross-section of the 3D anatomical spatio-temporal map 1400 of Fig. 14, in the frame number / maternal head/foot plane. Visible are the fetal head 1440, fetal spine 1450, fetal heart 1460, and fetal abdomen 1470, in a longitudinal, cephalic lie (see Fig. 3A).

Figs. 15A and 15B can be representative of the same patient. The 2D maps are 2D planes/slices or projections of a 3D map; what's on the x axis and y axis will depend on whether the sweep is vertical or horizontal, and on where the 2D slice is taken from the 3D map.

Fig. 16A is a schematic, diagrammatic representation, in hybrid flow diagram/block diagram form, of an example fetal lie determination method 1600, according to aspects of the present disclosure.

In step 1620, the method 1600 includes performing principal component analysis (PCA) on an anatomical spatio-temporal map 1610 (e.g., based on a vertical sweep). In an example, inputs to the PCA include spine frame coordinates and spine midpoints along the x axis (image width i.e. maternal left, right). The PCA then outputs a straight line which fits the maximum variation along the path. PC1 will give the spread along frame axis. Similarly, PC2 and other components will give spread in the other axes. Next, the slope of the line is calculated, which yields the value of theta. Execution then proceeds to step 1630.

In step 1630, the method 1600 includes determining the orientation of a line drawn between the head detection region and the abdomen detection region (e.g., between the centers of the two regions). In some aspects, the method 1600 instead uses a line determined by the PCA, or a line drawn between the fetal abdomen and the fetal heart. Execution then proceeds to step 1640. Step 1630 is shown in more detail in Fig. 16B.

It is noted that in Fig. 16A, orientation is described as the orientation of the longest component. In other figures, below, the PCA line is not always the one that is used to determine theta. Rather, it may be the line between head detection region and the abdomen region that is used for the orientation (θ). Different approaches may be used to give more accurate results. Considering a straight line fit of two points (abdomen and head cluster midpoint), may give more accurate results and perform better for different edge cases, such as when the fetal head is not aligned with the body, but lying in a curvilinear fashion.
In step 1640, the method 1600 includes determining if the fetal lie is longitudinal, transverse, or oblique, using the orientation θ of the head-abdomen line. Whether head is below or above the abdomen /heart cluster based on that we can decide the lie. Execution then proceeds to step 1650, 1660, or 1670, depending on the value of θ.

In step 1650, the method 1600 includes determining that the orientation (Θ) is between 75° and 105° or between -75° and -105°. Execution then proceeds to step 1655.

In step 1655, the method 1600 includes determining that the fetal lie is longitudinal, and either cephalic if θ is positive, or breech if θ is negative. Execution then proceeds to step 430.

In step 1660, the method 1600 includes determining that the orientation (Θ) is between 15° and 75°, between 105° and 165°, between -105° and -165°, or between -15° and -75°. Execution then proceeds to step 1665.

In step 1665, the method 1600 includes determining that the fetal lie is oblique. Execution then proceeds to step 430.

In step 1660, the method 1600 includes determining that the orientation (Θ) is between - 15° and 15° or between 165° and -165°. Execution then proceeds to step 1675.

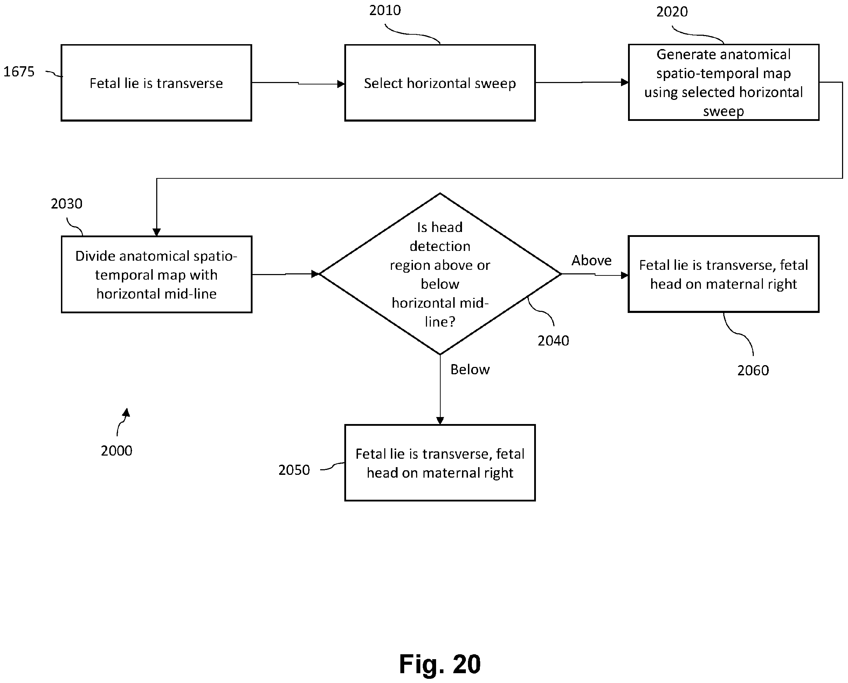

In step 1675, the method 1600 includes determining that the fetal lie is transverse. Execution then proceeds to step 2010 of Fig. 20.

In step 430, the method 1600 includes outputting a visual representation of the fetal lie identification 420. The method 1600 is now complete.

It is noted that the ranges listed for Fig. 16 are simply example values +/- 15 degrees from the ideal values. For a longitudinal lie, the ideal value is 90 degrees (breech) or -90 degrees (cephalic). For an oblique lie, the ideal values are 45 degrees, 135 degrees, -135 degrees, or -45 degrees. For a transverse lie, the ideal values are 0 degrees (head on maternal left) and 180 degrees (head on maternal right).

Fig. 16B is a schematic, diagrammatic representation, in hybrid flow diagram/block diagram form, of an example fetal lie determination method 1630, according to aspects of the present disclosure. Fig. 16B shows an expanded view of step 1630 from Fig. 16A.

In step 1631, the method 1630 begins with the angle from a line equation derived from principle component analysis as described above. Execution then proceeds to step 1632.

In step 1632, the method 1630 includes determining whether the head is above or below the abdomen and/or heart. If above, execution then proceeds to step 1633. If below, execution proceeds to step 1635.

In step 1633, the method 1630 includes determining whether the sign of the angle is less than zero. If yes, execution proceeds to step 1634. If no, execution proceeds to step 1637.

In step 1634, the method 1630 includes setting the angle equal to 180 degrees minus the angle. The method 1630 is now complete.

In step 1635, the method 1630 includes determining whether the sign of the angle is greater than zero. If yes, execution proceeds to step 1636. If no, execution proceeds to step 1637.

In step 1636, the method 1630 includes setting the angle to minus (180-angle). The method 1630 is now complete.

In step 1637, the method 1630 includes leaving the angle unchanged. The method 1630 is now complete.

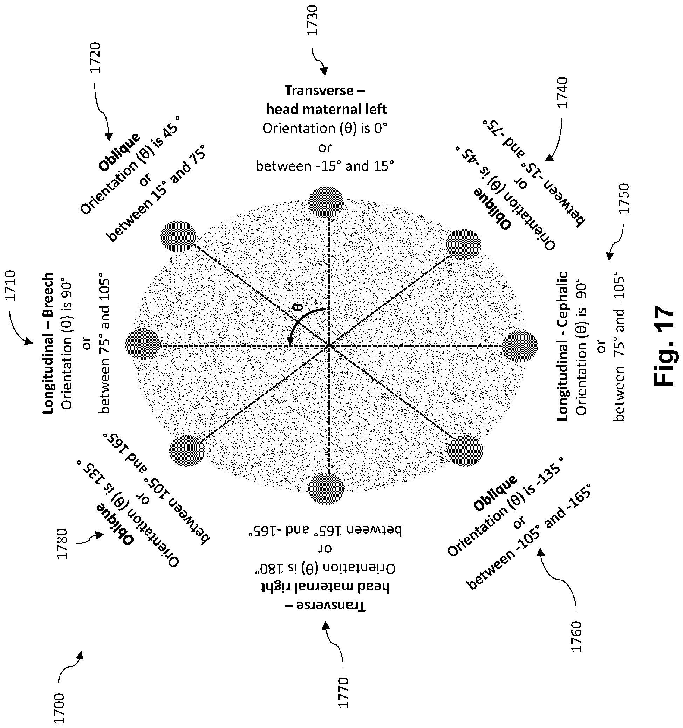

Fig. 17 is a diagram indicating the meaning of different values for the fetal orientation angle θ, according to aspects of the present disclosure. When the orientation (θ) is 90°, or between 75° and 105°, the lie is longitudinal - breech. When the orientation (Θ) is 45°, or between 15° and 75°, the lie is oblique. When the orientation (θ) is 0°, or between -15° and 15°, the lie is transverse - head maternal left. When the orientation (Θ) is -45°, or between -15° and -75°, the lie is oblique. When the orientation (θ) is -90°, or between -75° and -105°, the lie is longitudinal - cephalic. When the orientation (Θ) is - 135°, or between -105° and -165°, the lie is oblique. When the orientation (θ) is 180°, or between 165° and -165°, the lie is transverse - head maternal right. When the orientation (Θ) is 135°, or between 105° and 165°, the lie is oblique.

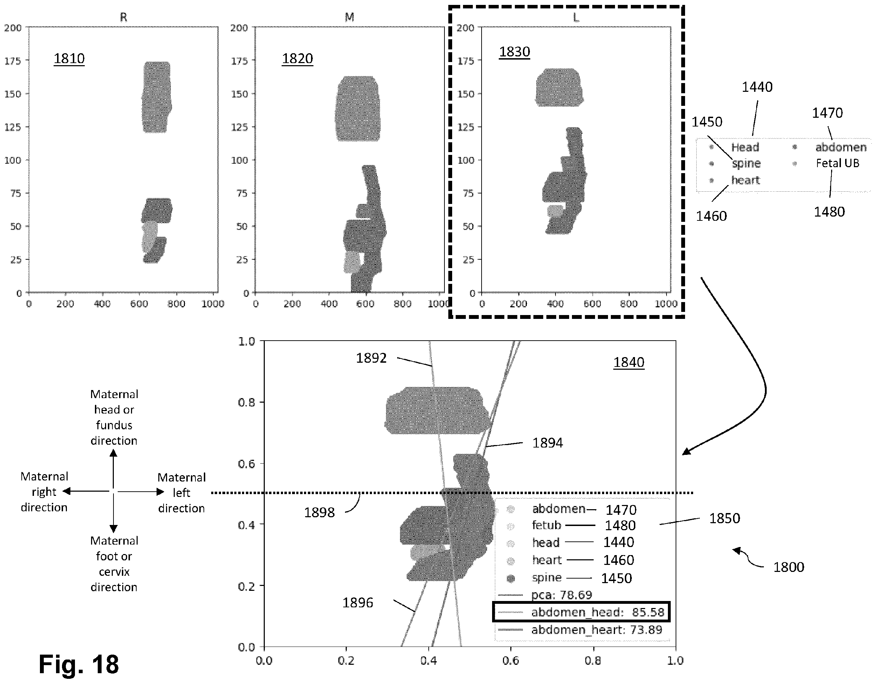

Fig. 18 is a schematic, diagrammatic representation, of an example fetal orientation determination process 1800, according to aspects of the present disclosure. Three vertical sweeps 1810, 1820, and 1830 are evaluated, and the L sweep 1830 is selected. This selection may be a count-based determination, based on the quantity of anatomy types detected and/or the total quantity of detections. As described in Fig. 11; in addition to a count-based determination, the sweep selection can also be based on where along the length of the sweep the anatomy detections begin and end. For example, in the L sweep 1830, detections are spaced from both the beginning (frame 0, which is closer to cervix) and ending (frame 200, which is closer to fundus) of the sweep; this can be favored to make sure anatomy detections are not missed/cut off. In contrast, in the M sweep, detections not spaced from the beginning of sweep; that is, detections start at frame 0, which is closer to the cervix. This may be disfavored, because there may be anatomy detections that are cut off or missed (e.g., closer towards the cervix, below the beginning of the M sweep).

The selected sweep 1840 is the L sweep 1830, and contains the same data as the L sweep 183, although it looks slightly differently because the x and y scale have been modified, and a different color-coding scheme has been used. Also, some of the anatomy regions in the L sweep at the bottom have been moved forward/backward (e.g., into or out of the page) relative to those in the L sweep at the top.

The abdomen detection region 1470 and head detection region 1440 are identified, and a line 1892 (marked "abdomen_head" in the legend 1850) is drawn between them. In this example, orientation (Θ) is the angle of that abdomen_head line, which is used to determine fetal lie. However, other lines also appear in the image. As part of the PCA process, the spine detection region 1450 is identified as the longest component, and a line 1894 (marked "pea" in the legend) is the orientation of the spine-abdomen region, such that the line 1894 passes through the spine 1450 and the abdomen 1470.

A heart detection region 1460 is also identified, and a line 1896 (labeled "abdomen_heart" in the legend) is drawn between the abdomen detection region 1470 and the heart detection region. In various aspects, the abdomen-head line, the PCA line, or the abdomen-heart line could be used at the orientation (theta) used to determine fetal lie, depending which gives more accurate results, e.g., for edge cases where the fetus is in a curved orientation.

In the example shown in Fig. 18, the orientation (Θ) is calculated to be -85.58 degrees, based on the abdomen-head line, indicating a longitudinal-breech lie for the fetus. The absolute value of the orientation θ (e.g., close to 90 degrees) indicates a longitudinal lie. The sign (positive or negative) can indicate whether the longitudinal lie is cephalic or breech. This sign convention is similar to adding a horizontal mid-line and determining whether the head detection region is above the horizontal mid-line (breech) or below the horizontal mid-line (cephalic)

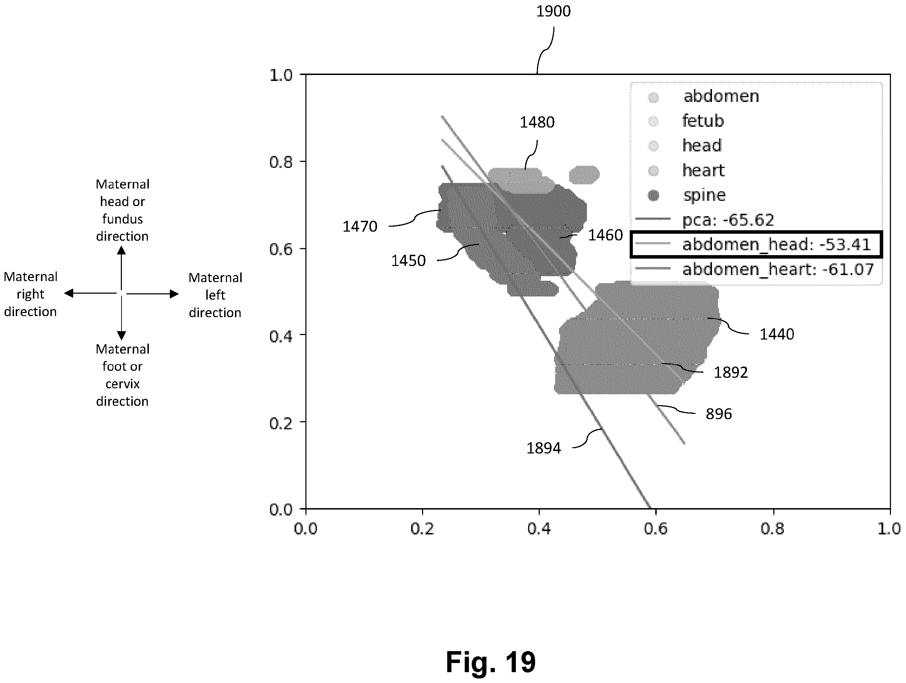

Fig. 19 is a 2D anatomical spatio-temporal map 1900 for a vertical sweep (e.g., an M sweep), according to aspects of the present disclosure. Visible are the fetal head 1440, spine 1450, heart 1460, abdomen 1470, and fetal urinary bladder 1480. Between the abdomen detection region 1470 and head detection region 1440 a line 1892 is drawn. In the example shown in Fig. 19, orientation (Θ) is the angle of that abdomen_head line 1892, which is used to determine fetal lie.

Other lines can be seen in the map 1900. As part of the PCA process, the spine detection region 1450 is identified as the longest component, and a line 1894 is drawn along the spine 1450 and passing through the abdomen 1470, thus identifying the orientation of the spine abdomen region. A heart detection region 1460 is also identified, and a line 1896 is drawn between the abdomen detection region 1470 and the heart detection region 1460. In various aspects, the PCA line 1894 or the abdomen-heart line 1896 could be used as the orientation (Θ) used to determine fetal lie, instead of the abdomen-head line 1892.

In the example shown in Fig. 19, the orientation (Θ) is -53.41 degrees, which indicates an oblique lie with the head between the transverse head maternal right and longitudinal cephalic positions (see Fig. 17).

Fig. 20 is a schematic, diagrammatic representation, in flow diagram form, of an example fetal lie detection method 2000, according to aspects of the present disclosure.

In step 1675 of Fig. 16, the method 2000 includes determining that the fetal lie is transverse. Execution then proceeds to step 2010.

In step 2010, the method 2000 includes selecting a horizontal sweep. The selection of which horizontal sweep to use could be made using a count-based technique and/or where anatomy detections begin/end (as described above in Figs. 13 and 18). Execution then proceeds to step 2020.

In step 2020, the method 2000 includes generating an anatomical spatio-temporal map using the selected horizontal sweep, using the methods described above in Fig. 14. Execution then proceeds to step 2030.

In step 2030, the method 2000 includes dividing the anatomical spatio-temporal map with a horizontal mid-line. Execution then proceeds to step 2040.

In step 2040, the method 2000 includes determining whether the head detection region (or a midpoint thereof) is above or below the horizontal mid-line. If above, execution proceeds to step 2050. If below, execution proceeds to step 2060.

In step 2050, the method 2000 includes determining that the fetal lie is transverse, with the head on the maternal right. Execution then proceeds to step 420 of Fig. 16.

In step 2060, the method 2000 includes determining that the fetal lie is transverse, with the head on the maternal left. Execution then proceeds to step 420 of Fig. 16.

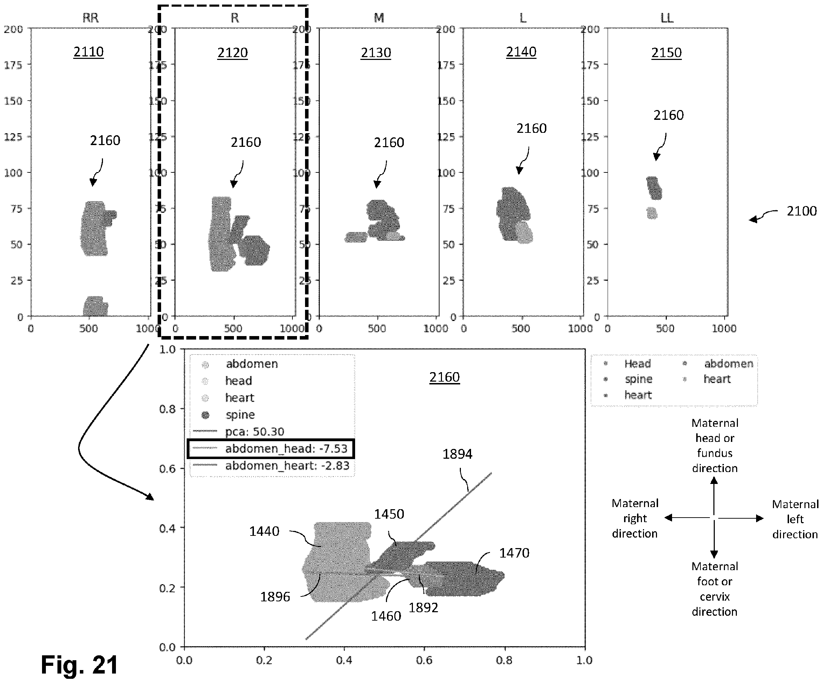

Fig. 21 is a schematic, diagrammatic representation, of an example fetal orientation determination process 2100, according to aspects of the present disclosure. Five 2D spatio-temporal maps of vertical sweeps 2110, 2120, 2130, 2140, and 2150 are evaluated, and the R sweep map 2120 is selected, based on the number of anatomy detections 2160 in the map 2120, where the anatomies begin or end, etc.

The selected map 2160 thus contains the same data as the R sweep map 2120, although it looks slightly different due to changes in the X-Y scaling, color mapping, and front-back stacking of the colors Visible in the selected sweep 2160 are the fetal head 1440, spine 1450, heart 1460, and abdomen 1470.

The abdomen detection region 1470 and head detection region 1440 are identified, and a line 1892 is drawn between them. In this example, the fetal orientation (Θ) is the angle of that abdomen-head line 1892, which is used to determine fetal lie. However, other lines appear in the image. As part of PCA process, the spine detection region 1450 is identified as the longest component, and a line 1894 (labeled "pca") is drawn along the centerline of the spine 1450. The heart detection region 1460 is also identified, and a line 1896 is drawn between the abdomen detection region 1470 and the heart detection region 1460, that is labeled "abdomen-heart". In various aspects, the PCA line 1894 or the abdomen-heart line 1896 could be used at the orientation (Θ) to determine fetal lie. In the example shown in Fig. 21, the value of the orientation (theta) is -7.53 degrees. The value of the orientation (e.g., between -15 degrees and 15 degrees) thus indicates a transverse lie.

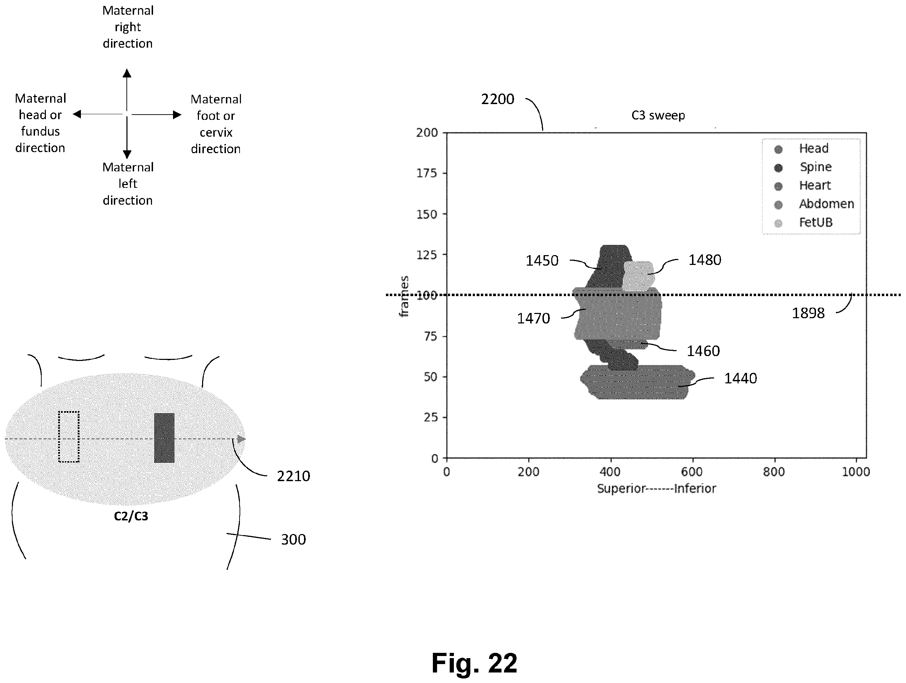

Vertical sweep data does not provide enough information to confirm whether the baby's head is to the maternal left or right with the transverse lie. Therefore, the system needs to consider a horizontal sweep, as shown below in Fig. 22. The sign (positive or negative) of θ can indicate whether the longitudinal lie is cephalic or breech. In some aspects, the line is drawn start from the abdomen detection region and going to the head detection region. When the line is going up from the abdomen detection region to get to the head detection region, the sign can be negative. This indicates that the fetal head is in the direction of the maternal head/fundus, which is a breech lie. When the line is going up from the abdomen detection region to get to the head detection region, the sign can be positive. This indicates that the fetal head is in the direction of the maternal foot/cervix, which is a cephalic lie. This sign convention is similar to adding a horizontal mid-line and determining whether the head detection region is above the horizontal mid-line (breech) or below the horizontal mid-line (cephalic).

Fig. 22 is a schematic, diagrammatic representation of an example fetal orientation determination process, according to aspects of the present disclosure. A horizontal sweep spatio-temporal map 2200 is selected based on the number and location of the detections it contains, as described in detail above. In the example shown in Fig. 22, the C2 or C3 sweep 2210 is selected. Visible are the fetal head 1440, spine 1450, heart 1460, abdomen 1470, and urinary bladder 1480.

Because the anatomical spatio-temporal map from vertical sweep indicated that fie lie is transverse (see Fig. 21, above), it is necessary to select a horizontal sweep. This can be done using a count-based determination, based on where along the length of the sweep the anatomy detections begin and end, etc. - similar to selection described in Figs. 11 and 18. All horizontal sweeps can be considered, or the consideration can be hierarchical/preferential. For example, the selection process can prefer the middle horizontal sweep (C2 when using 3x3 blind sweep protocol, or C3 when using a 5x5 blind sweep protocol). Based on experimental data, it is expected that the middle horizontal sweep (e.g., the C2 or C3 sweep) will be the selected sweep, because experimental data shows that the middle horizontal sweep usually has the most anatomy types and/or detections. However, the selection can be any horizontal sweep.

As shown in Fig. 4, a horizontal sweep in the blind sweep protocol can go from the maternal right to the maternal left. The system then generates an anatomical spatio-temporal map for the selected horizontal sweep (e.g., as shown above in Fig. 12). Next, the system estimates the position of the fetal head 1440 relative to a horizontal mid-line 1898 for the horizontal sweep. If the head detection region is below the horizontal mid-line 1898, indicates that fetal head is on maternal left (as shown in Fig. 22). Alternatively, if the head detection region 1440 is above the horizontal mid-line 1898, this indicates that the fetal head is on the maternal right.

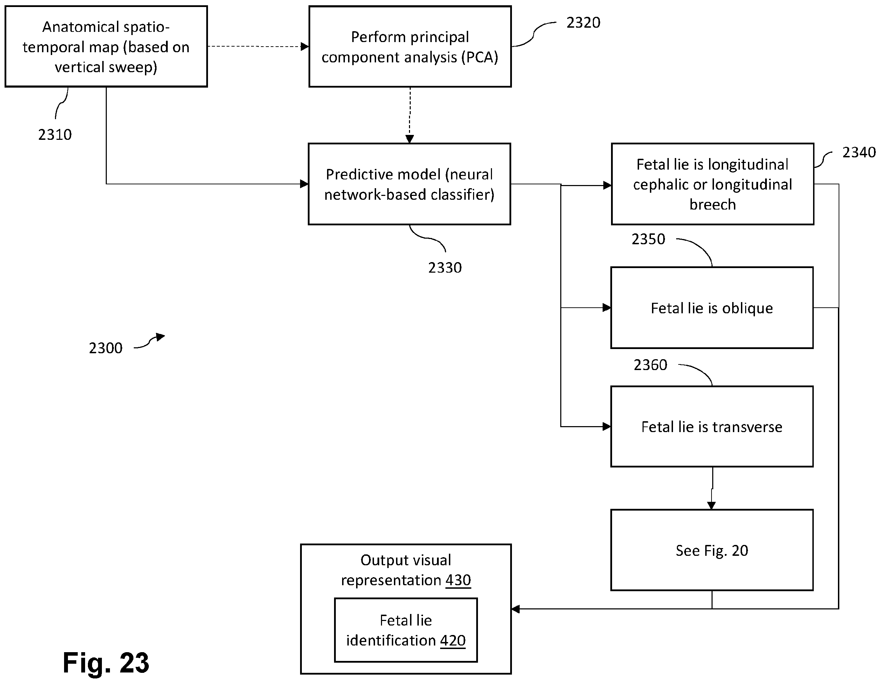

Fig. 23 is a schematic, diagrammatic representation, in hybrid flow diagram/block diagram form, of an example fetal lie determination method 2300, according to aspects of the present disclosure. A predictive model 2330 (e.g., a neural network-based classifier such as a convolutional neural network (CNN) or state vector machine (SVM)) receives, as an input, either the selected anatomical spatio-temporal map 2310 itself (e.g., selected from the available vertical sweeps), or else the components of a principal component analysis (PCA) 2320 performed on the map 2310, or a combination thereof. The classifier outputs a fetal lie prediction 2340, 2350, or 2360. It is noted that for classifying cephalic, breech, etc., a heuristic may be used instead or in addition,

In step 2340, the classifier 2330 has determined that the fetal lie is longitudinal cephalic or longitudinal breech. Execution then proceeds to step 430.

In step 2350, the classifier 2330 has determined that the fetal lie is oblique. Execution then proceeds to step 430.

In step 2360, the classifier 2330 has determined that the fetal lie is transverse. Execution then proceeds to step 2010 of Fig. 20.

In step 430, the method 2300 includes outputting a visual representation of the fetal lie identification 420. The method 2300 is now complete.

Examples of the classifier 2330 include CNN, SVM, etc. In some aspects, an ensemble approach may be used to determine the fetal lie, using a combination of predictive model (as shown here in Fig. 23) and the PCA (as shown in Fig. 16), such as the head-abdomen line (as shown in Fig. 18).

Fig. 24 is a schematic, diagrammatic representation of an example fetal orientation determination method 2400, according to aspects of the present disclosure. The method 2400 assumes that fetal lie has already been determined in Figs. 16-23.

The purpose of the method 2400 is to determine whether the fetal spine is anterior or posterior. This requires the sweep direction (vertical or horizontal) that will provide ultrasound data necessary to determine fetal spine presentation. This will depend on the fetal lie, e.g., the sweep direction used to determine fetal spine presentation should be perpendicular to the fetal lie. That is, to determine fetal spine presentation, the sweep direction crosses perpendicular across the fetus's body (not along the length of fetus's body-this is what is used to determine fetal lie, as described in Figs. 16, 23, etc.) The sweep direction crossing perpendicular across the fetus's body provides information about the fetal spine in relation to other anatomy in the uterus, which includes both other fetal anatomy (e.g., fetal heart) and maternal anatomy (e.g., placenta, amniotic fluid).

In contrast, the sweep direction extending along the length of the fetus's body is used to determine fetal lie in Figs. 16-23 (e.g., a vertical sweep parallel to/coaxial with and used to determine a longitudinal lie, a horizontal sweep is parallel to/coaxial with and used to determine a transverse lie). This provides information about the spatial relationship of the fetal anatomies to one another, which is what fetal lie (how is the fetal anatomies arranged).

In step 2410, the fetal lie is longitudinal, and a horizontal sweep is selected. Execution then proceeds to step 2420.

In step 2420, the method 2400 includes receiving the bounding boxes or masks for the anatomy detections in the selected horizontal sweep. Execution then proceeds to step 2430.

In step 2440, the fetal lie is transverse, and a vertical sweep is selected. Execution then proceeds to step 2450.

In step 2450, the method 2400 includes receiving the bounding boxes or masks for the anatomy detections in the vertical sweep. Execution then proceeds to step 2430.

In step 2430, the method 2400 includes determining the midpoints of the bounding boxes for each anatomy type (e.g., head, spine, heart, abdomen, placenta, amniotic fluid, etc.). Execution then proceeds to step 2460.

In step 2460, the method includes generating a plot of the midpoints. The plot thus includes clusters of midpoints of each respective anatomy type. Execution then proceeds to steps 2470 and 2480.

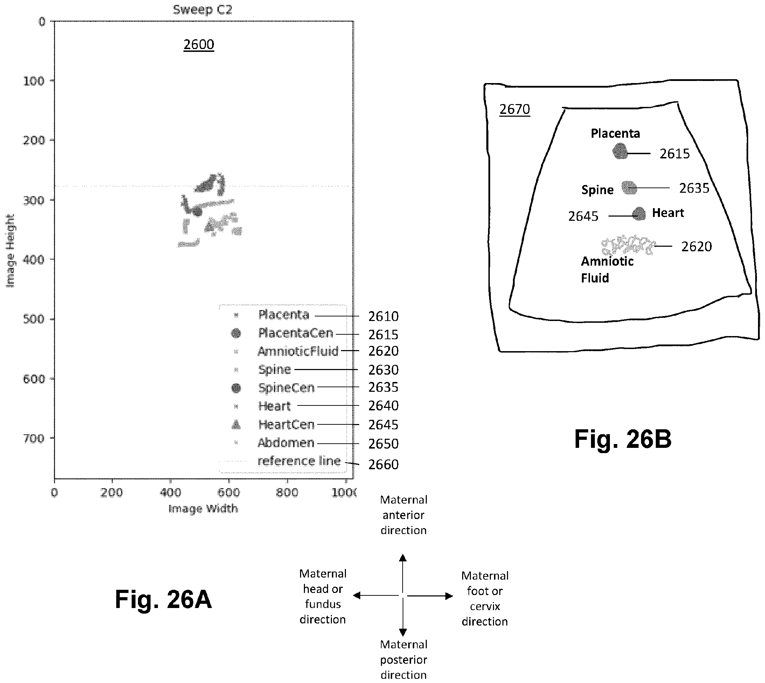

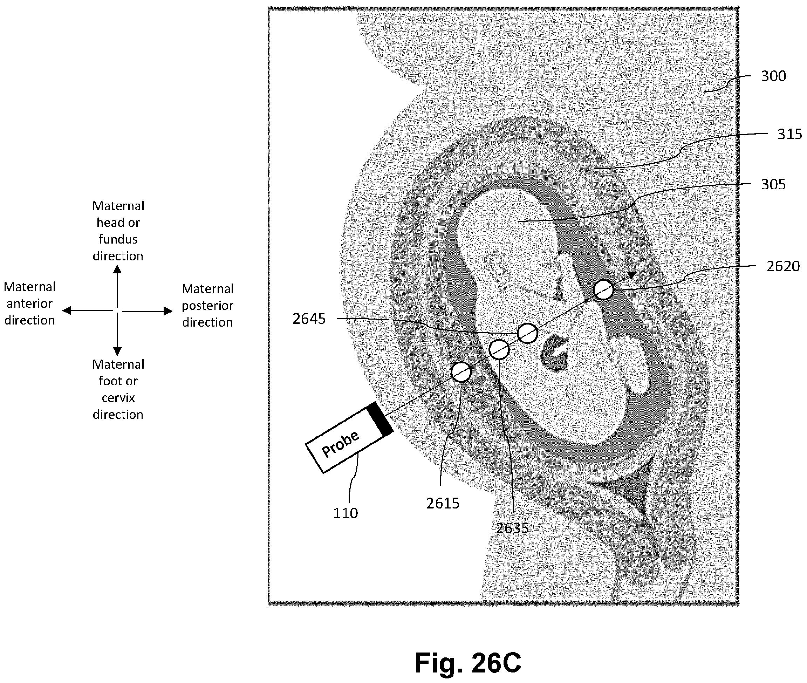

The plot of mid-points may be representative of the sweep as a whole. For example, an individual ultrasound frame can have no detections, can have detections of one anatomy type, or multiple detections of multiple anatomy types. The plot includes the mid-point of each of these bounding boxes, repeated for all ultrasound image frames in the sweep. All of the mid-points are thus plotted. In the plot, the mid-points can be grouped based on anatomy type. The mid-points of respective anatomy types can form clusters. The centroid is the center of grouping/cluster of mid-points for a respective anatomy type. See the example plots in Figs. 26 and 28 for details.

In step 2470, the method 2400 includes determining the centroids of the fetal spine cluster, the fetal heart cluster, and the placenta cluster. Execution then proceeds to step 2490.

In step 2480, the method 2400 includes determining the placenta location with respect to the amniotic fluid to determine whether the placenta is in an anterior or posterior position. Execution then proceeds to step 2490.

Determining placenta location with respect to amniotic fluid is described in U.S. Provisional Application No. 63/611,810, filed December 19, 2023, and titled "Low-Lying Placenta and/or Placenta Location in Ultrasound Imaging with Blind Sweep Protocol", incorporated by reference as though fully set forth herein.

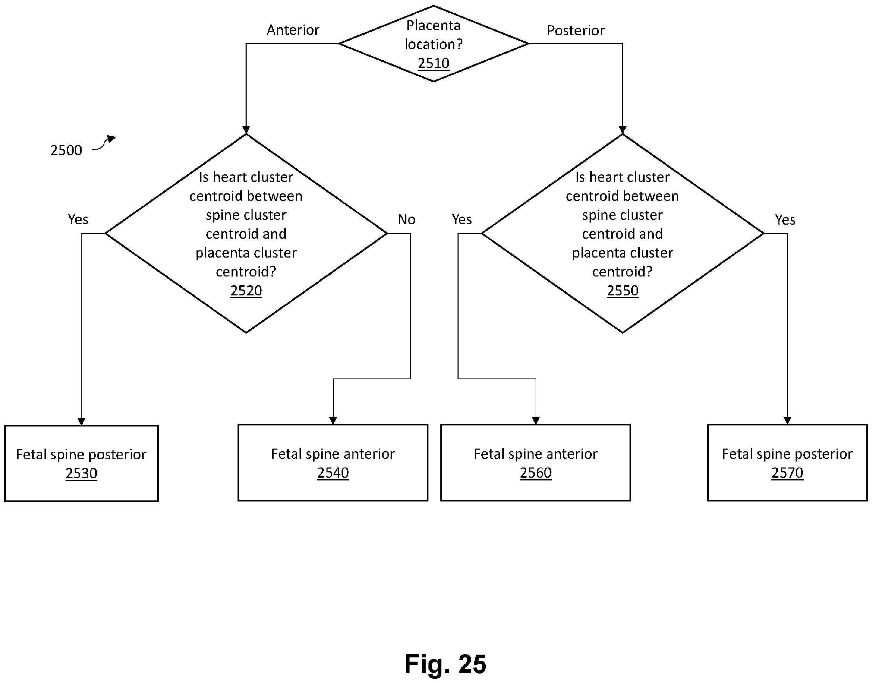

In step 2490, the method 2400 includes estimating the fetal spine position, based on the placenta location and the positions of the spine cluster centroid, heart cluster centroid, and placenta cluster centroid, as described in Fig. 25, below. Execution then proceeds to step 2495.

In step 2495, the method 2400 includes determining, based on the fetal spine position, whether the fetal presentation is fetal spine anterior or fetal spine posterior. Execution then proceeds to step 2499.

In step 2499, the method 2400 includes outputting a visual representation that includes the fetal spine 1450 and/or the fetal spine presentation 2495. Depending on the implementation, the visual representation may also include the plot of mid-points from step 2460.

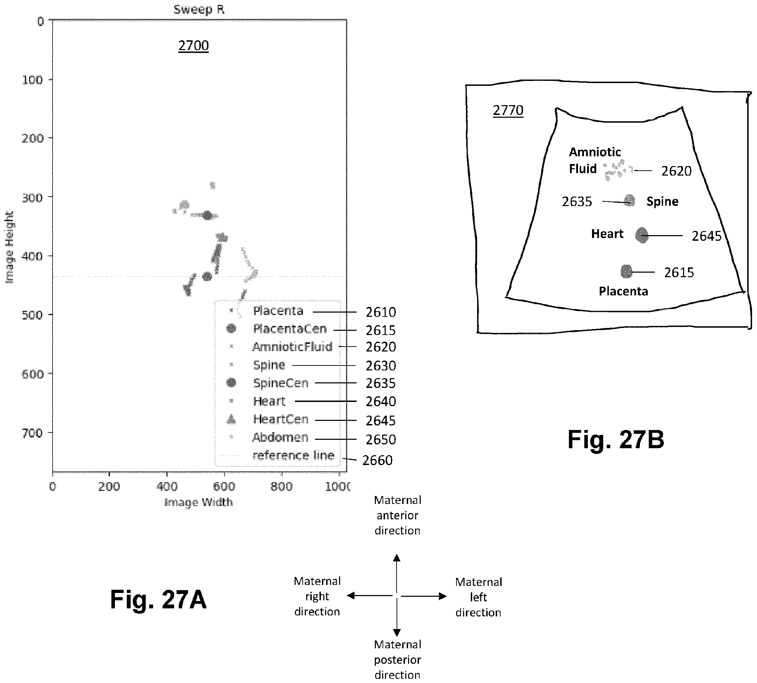

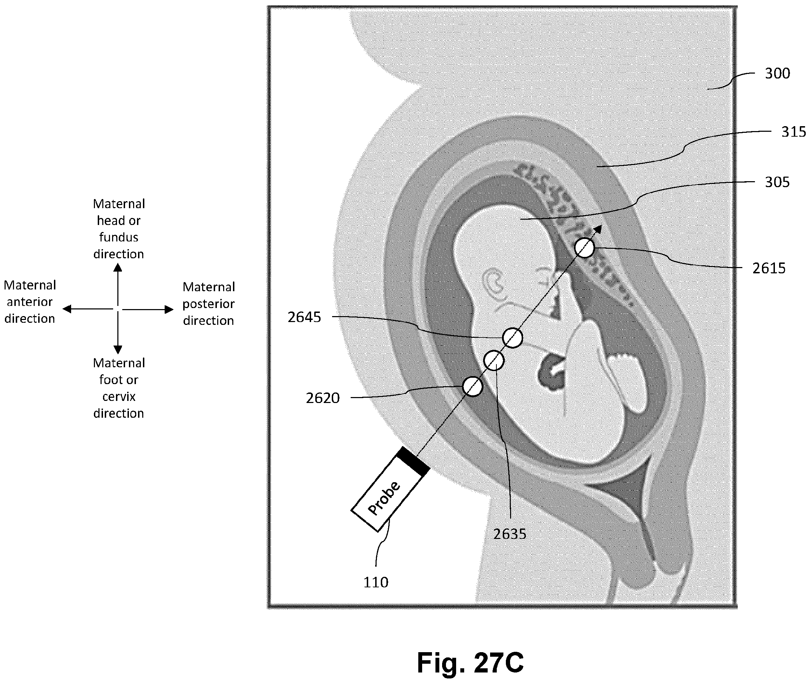

Fig. 25 is a schematic, diagrammatic representation, in flow diagram form, of an example fetal spine presentation determination method 2500, according to aspects of the present disclosure. As a whole, this flow diagram is using the expected spatial relationships (positioning) between placenta, amniotic fluid, fetal heart, and fetal spine to determine the fetal spine presentation. For example, when the placenta is anterior and the fetal spine is posterior, the expectation is that fetal heart will not be between the placenta and the fetal spine. Rather, the fetal spine will between the placenta and the fetal heart. (See Figs. 26 and 27.)

Similarly, when the placenta is posterior and the fetal spine is anterior, the expectation is that the expectation is that the fetal heart will be between the fetal spine and the placenta. (See Figs. 28 and 29.) When the placenta is posterior and the fetal spine is posterior, the expectation is that the fetal heart will be between the placenta and the fetal spine. When the placenta is anterior and the fetal spine is anterior, the expectation is that the fetal heart will not be between the placenta and the fetal spine. Rather, the fetal spine will between the placenta and the fetal heart.

In step 2510, the method 2500 includes determining whether the placenta location is anterior or posterior. If anterior, execution proceeds to step 2520. If posterior, execution proceeds to step 2550.

In step 2520, the method 2500 includers determining whether the heart cluster centroid is between the spine cluster centroid and the placenta cluster centroid. If yes, execution proceeds to step 2530. If no, execution proceeds to step 2540.

In step 2530, the method 2500 includes determining that the fetal spine presentation is posterior. The method 2500 is now complete.

In step 2540, the method 2500 includes determining that the fetal spine presentation is anterior. The method 2500 is now complete.

In step 2550, the method 2500 includes determining whether the heart cluster centroid is between the spine cluster centroid and the placenta cluster centroid. If yes, execution proceeds to step 2560. If no, execution proceeds to step 2570.

In step 2560, the method 2500 includes determining that the fetal spine presentation is anterior. The method 2500 is now complete.

In step 2570, the method 2500 includes determining that the fetal spine presentation is posterior. The method 2500 is now complete.

Fig. 26A is a plot 2600 of the midpoints of the anatomy detection boxes across a sweep, according to aspects of the present disclosure. In the example shown in Fig. 26A, the fetus has a longitudinal lie, and therefore a horizontal sweep (e.g., the C2 sweep) is selected. Visible are the placenta midpoints 2610, centroid of the placenta midpoints 2615, the amniotic fluid midpoints 2620, the spine midpoints 2630, centroid of the spine midpoints 2635, heart midpoints 2640, centroid of the heart midpoints 2645, abdomen midpoints 2650, and a reference line 2660 (which may for example represent the average of the centroids of the amniotic fluid midpoints). The centroid of each detection cluster is a handy surrogate for the center of the anatomy itself, such that the heart centroid 2645 may be presumed to be close to the center of the heart, the placenta centroid 2615 may be presumed to be close to the center of the placenta, etc.

Determining placenta location with respect to amniotic fluid is described for example in U.S. Provisional Application No. 63/611,810, filed December 19, 2023, and titled "Low-Lying Placenta and/or Placenta Location in Ultrasound Imaging with Blind Sweep Protocol", incorporated by reference as though fully set forth herein.

Fig. 26B is a synthesized ultrasound image 2670 generated based on the anatomy detection cluster centroids of Fig. 26A, according to aspects of the present disclosure. As can be seen in the image, the placenta centroid 2615 is in an anterior position, and the heart centroid 2645 is not located between the spine centroid 2635 and the placenta centroid 2615. Per Fig. 25, above, this arrangement of features indicates a fetal spine anterior presentation. Depending on the implementation, the synthesized ultrasound image 2670 may be shown to the user, along with a text indication of the fetal spine presentation, as part of the output visual representation 2499 of Fig. 24.

Fig. 26C is a schematic, diagrammatic, side cross-sectional view of a fetus 305 inside a uterus 315 of a patient 300, according to aspects of the present disclosure. The dotted line 2680 represents an imaging plane for the imaginary or synthetic ultrasound image in Fig. 26B. Thus, Fig. 26C is representative of the geometry of Figs. 26A and 26B, with fetus 305 in a longitudinal, breech lie, with anterior placenta 2615 and anterior presentation of the spine 2635. Thus, an ultrasound probe 110 placed on the abdomen of the patient first sees the placenta centroid 2615 as the shallowest feature in an ultrasound image, then the spine centroid 2635, then the heart centroid 2645, with the amniotic fluid 2620 being the deepest feature in the image.

Fig. 27A is a plot 2700 of the midpoints of the anatomy detection boxes across a sweep, according to aspects of the present disclosure. In the example shown in Fig. 27A, the fetus has a transverse lie, and therefore a Vertical sweep (e.g., the M sweep) is selected. Visible are the placenta midpoints 2610, centroid of the placenta midpoints 2615, the amniotic fluid midpoints 2620, the spine midpoints 2630, centroid of the spine midpoints 2635, heart midpoints 2640, centroid of the heart midpoints 2645, abdomen midpoints 2650, and a reference line 2660.

Fig. 27B is a synthesized ultrasound image 2770 generated based on the anatomy detection cluster centroids of Fig. 27A, according to aspects of the present disclosure. As can be seen in the image, the placenta centroid 2615 is in a posterior position, and the heart centroid 2645 is located between the spine centroid 2635 and the placenta centroid 2615. Per Fig. 25, above, this arrangement of features indicates a fetal spine anterior presentation. Depending on the implementation, the synthesized ultrasound image 2670 may be shown to the user, along with a text indication of the fetal spine presentation, as part of the output visual representation 2499 of Fig. 24.

Fig. 27C is a schematic, diagrammatic, side cross-sectional view of a fetus 305 inside a uterus 315 of a patient 300, according to aspects of the present disclosure. The dotted line 2680 represents an imaging plane for the imaginary or synthetic ultrasound image in Fig. 27B. Thus, Fig. 27C is representative of the geometry of Figs. 27A and 27B, with the fetus 305 in a longitudinal, breech lie, with posterior placenta 2615 and anterior presentation of the spine 2635. Thus, an ultrasound probe 110 placed on the abdomen of the patient 300 first sees the amniotic fluid 2620 as the shallowest feature in an ultrasound image, then the spine centroid 2635, then the heart centroid 2645, with the placenta centroid 2615 being the deepest feature in the image.

Fig. 28 is a schematic, diagrammatic representation of a sweep acceptance process 2800, according to aspects of the present disclosure. In the example shown in Fig. 28, a horizontal calibration sweep 2810 and a vertical calibration sweep 2820 each cover the entire width and height of the abdomen 310, respectively, thus providing full coverage of the fetus 305. Thus, the calibration sweeps 2810, 2820 are accepted, and the user is permitted to begin the blind sweep protocol.

Fig. 29 is a schematic, diagrammatic representation of a sweep rejection process 2900, according to aspects of the present disclosure. In the example shown in Fig. 28, a horizontal calibration sweep 2910 and a vertical calibration sweep 2920 each cover only a portion of the entire width and height of the abdomen 310, respectively, thus providing only partial coverage of the fetus 305. Thus, the calibration sweeps 2910, 2920 are rejected, and the user is instructed to repeat the calibration sweeps 2910, 2920 before the blind sweep protocol can begin.

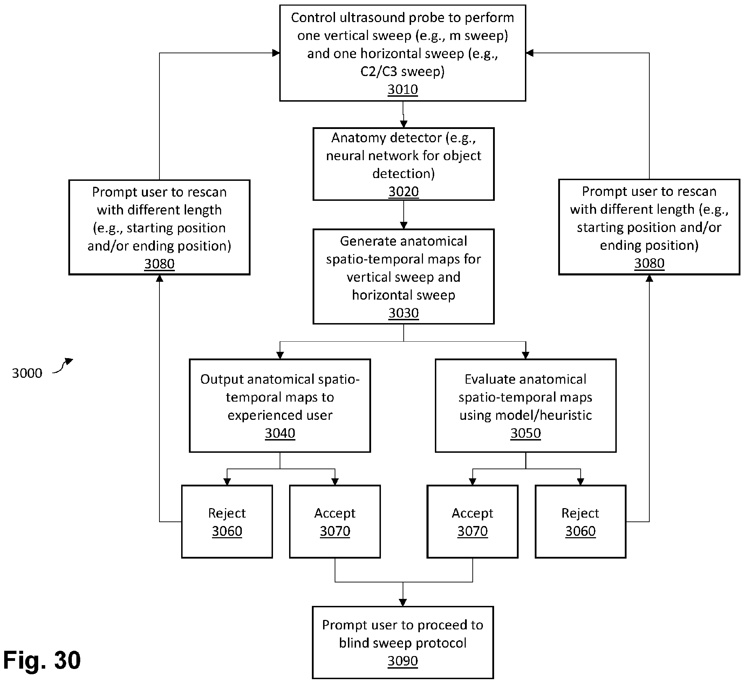

Fig. 30 is a schematic, diagrammatic representation, in hybrid flow diagram / block diagram form, of an example sweep calibration method 3000, according to aspects of the present disclosure. The purpose of the calibration method 3000 is to ensure the user understands how to perform a proper vertical sweep and a proper horizontal sweep, such that the entire fetus is imaged by at least one of the sweeps.

In step 3010, the method 3000 includes controlling the ultrasound probe to perform one vertical sweep (e.g., the M sweep) and one horizontal sweep (e.g., the C2 sweep in the case of a 3x3 protocol, or the C3 sweep in the case of a 5x5 protocol). Execution then proceeds to step 3020.

In step 3020, the method 3000 includes passing the sweeps to the anatomy detector to generate bounding boxes around detected fetal and/or maternal anatomy. Execution then proceeds to step 3030.

In step 3030, the method 3000 includes generating anatomical spatio-temporal maps for the vertical sweep and the horizontal sweep (e.g., as described above in Figs. 12A - 14). Execution then proceeds to step 3040 or step 3050, depending on whether an experienced user is available.

In step 3040, the method 3000 includes outputting the anatomical spatio-temporal maps to the experienced user, who can then manipulate a user control to accept or reject the sweeps. If reject, execution proceeds to step 3060. If accept, execution proceeds to step 3070.

In step 3050, the method 3000 includes evaluating the spatio-temporal maps using heuristics to determine whether full coverage of the fetus has been achieved. If yes, execution proceeds to step 3070. If no, execution proceeds to step 3060. In a non-limiting example, a vertical sweep may be rejected if the cervix is not detected. This is because cervix detections are expected at the beginning of vertical sweeps that have a correct starting location (low enough on the maternal abdomen/belly. In another non-limiting example, the sweeps may be accepted only if there are anatomy type detections from the fetal head to the fetal urinary bladder (e.g., a majority or full length of the fetus). In the calibration step, scan acceptance and rejection can be an automated or manual process. In case of manual, the system can directly show the visual map to the user. If the scan captures sufficient anatomies, it will be accepted; otherwise it will be rejected. In case of automation, a heuristic approach can be used where the system can check whether the cervix (and all other fetal anatomies like head, heart, abdomen, fetus urinary bladder) are captured. If so, accept the scan, and otherwise reject it. Automation may be preferred in the case of an inexperienced user such as a midwife.

In step 3060, the calibration sweeps are rejected. Execution then proceeds to step 3080.

In step 3070, the calibration sweeps are accepted. Execution then proceeds to step 3090.

In step 3080, the method 3000 includes prompting the user to rescan the patient's abdomen using different lengths (e.g., using different starting positions and/or different ending positions). Execution then returns to step 3010.

In step 3090, the method 3000 includes prompting the user to begin the blind sweep protocol. The method 3000 is now complete.

Fig. 31 is a schematic, diagrammatic representation of accepted or acceptable calibration sweeps, according to aspects of the present disclosure. A vertical sweep 3110 (e.g., an M sweep) and a horizontal sweep 3120 (e.g., a C2 or C3 sweep) each contain significant detections of the fetal head 1440, spine 1450, heart 1460, abdomen 1470, and urinary bladder 1480. Thus, it can be deduced that the calibration sweeps were performed correctly, and that the user can proceed to the blind sweep protocol.

Fig. 32 is a schematic, diagrammatic representation of rejected or unacceptable calibration sweeps, according to aspects of the present disclosure. A vertical sweep 3210 contains significant detections of the fetal head 1440 and spine 1450, but not of the heart, abdomen, or urinary bladder. Another vertical sweep 3220 (e.g., an L sweep) contains significant detections of the abdomen 1470, urinary bladder 1480, and spine 1450, but not of the heart or head. A horizontal sweep 3230 (e.g., a C2 or C3 sweep) contains significant detections of the head 1440, heart 1460, and abdomen 1470, but contains no detections of the fetal urinary bladder, and contains multiple, physically separated detections of the fetal spine 1450. Thus, it can be deduced that the calibration sweeps were performed incorrectly, and that the user must repeat the calibration sweeps before proceeding to the blind sweep protocol.

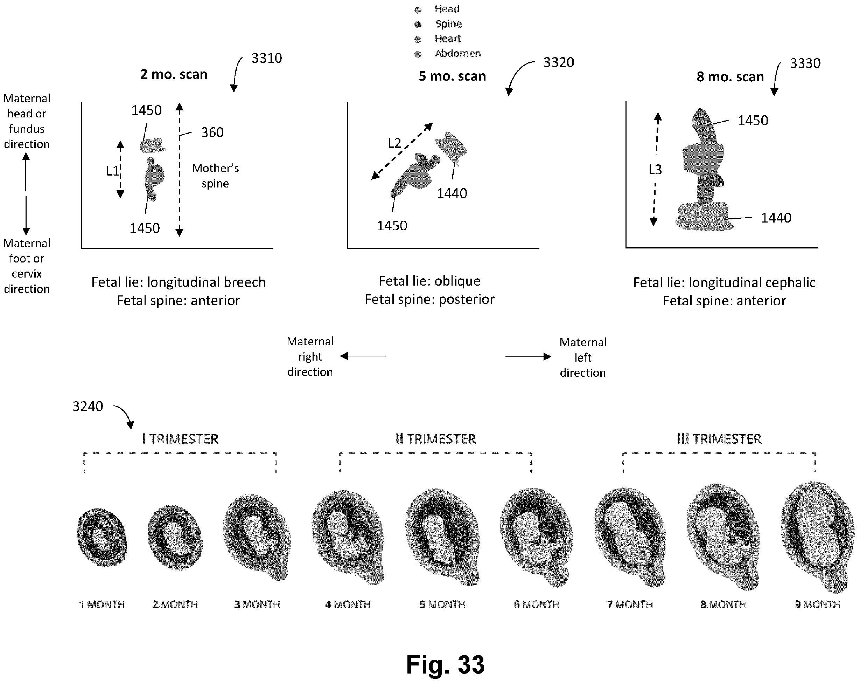

Fig. 33 is a schematic, diagrammatic representation of a fetal growth monitoring process 3300, according to aspects of the present disclosure. A spatio-temporal map 3310 of a scan performed at a gestational age of 8 weeks (2 months) shows a fetal length L1, driven by the distance between opposite ends of the head detection 1440 and spine detection 1450. Similarly, a spatio-temporal map 3320 of a scan performed at a gestational age of 20 weeks (5 months) shows a fetal length L2, which is larger than L1, and a spatio-temporal map 3330 of a scan performed at a gestational age of 32 weeks (8 months) shows a fetal length L3, which is larger than L1 or L2. These lengths can for example be measured directly by the system and displayed to the user, or can be estimated by the user based on an output to a display of the spatio-temporal maps 3310, 3320, 3330, etc., wherein the detected anatomies are growing larger as the gestational age advances. In either case, the length can be compared against a fetal size chart 3340 to determine whether the fetal length is within expected parameters for the given trimester and gestational age.

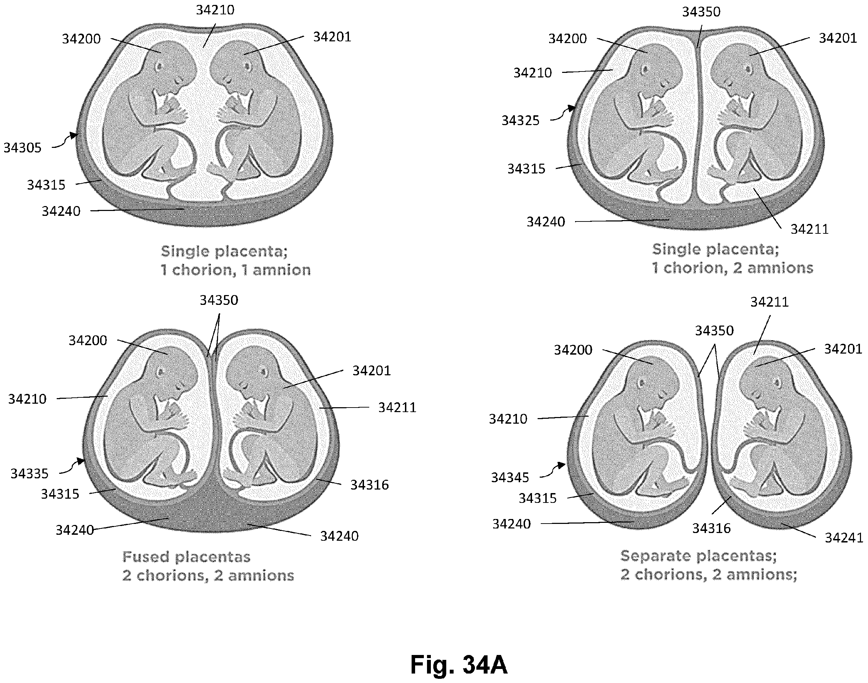

Fig. 34A is a set of diagrammatic views of two fetuses 34200, 34201 surrounded by a at least one amniotic sac or amnion 34210 and at least one chorionic sac or chorion 34315 within a uterus of a patient, representing different types of twins, according to aspects of the present disclosure. At least one placenta 34240 is also visible. Automated detection of multiple pregnancy or multiple gestation (e.g., twins) is an object of the present disclosure.

In a first example 34305, the multiple pregnancy/gestation is monochorionic (e.g., having only one chorion 34315) and monoamniotic (e.g., having only one amniotic sac 34210 that contains both fetuses 34200, 34201, and has a single placenta 34240.

In a second example 34325, the multiple pregnancy/gestation is monochorionic (e.g., having one chorion 34315) but diamniotic (e.g., having two amniotic sacs 34210, 34211, separated by a fetal membrane 34350, with each amniotic sac holding one fetus 34200, 34201), and a single placenta 34240. Detection of the fetal membrane is one way to detect a multiple gestation or multiple pregnancy.

In a third example 34335, the multiple pregnancy/gestation is dichorionic (e.g., having two chorions 34315, 34316, separated by respective membranes 34350) and diamniotic (e.g., having two amniotic sacs 34210, 34211, with each sac holding one twin 34200, 34201), and two placentas 34240 that are fused together.

In a fourth example 34345, the multiple pregnancy/gestation is dichorionic (e.g., having two separate chorions 34315, 34316, separated by respective membranes 34350) and diamniotic (e.g., having two separate amniotic sacs 34200, 34201, each holding one twin 34200, 34201), and two separate placentas 34240, 34241. This type of twin pregnancy is associated with a lower rate of complications than monochorionic or monoamniotic pregnancies.

70% of twin pregnancies are dizygotic (e.g., resulting from two fertilized eggs), and dizygotic pregnancies are believed to be always dichorionic and diamniotic. 30% of pregnancies are monozygotic (e.g., resulting from a single fertilized egg), and 20% of these pregnancies will also be dichorionic and diamniotic. Thus, detection of the fetal membrane 350 separating the two amniotic sacs can be effective in detecting up to 76% of twin pregnancies. The remaining 24% of detections may rely on other features of the pregnancy such as multiple occurrences of a unique anatomical feature (e.g., two heads, two hearts, two pelvises, etc.) and/or the spatial/geometrical relationship between fetal parts, as described below.

The uterus can be considered maternal anatomy. Depending on the context, the placenta, amniotic fluid, etc. may be considered fetal anatomy, maternal anatomy, or an interface between the two.

Examples of multiple gestation detection can be found for example in U.S. Provisional Patent Application No. 63/655,754, filed June 4, 2024, titled "Multiple Pregnancy/Gestation Detection Based On Graphing And Skeletonization Of Fetal Anatomy Detections From Ultrasound Imaging Blind Sweep Protocol", and U.S. Provisional Patent Application No. 63/620,385, filed January 12, 2024, titled "Multiple Pregnancy/Gestation Detection Using Ultrasound Imaging With Blind Sweep Protocol", each of which is incorporated by reference as though fully set forth herein.

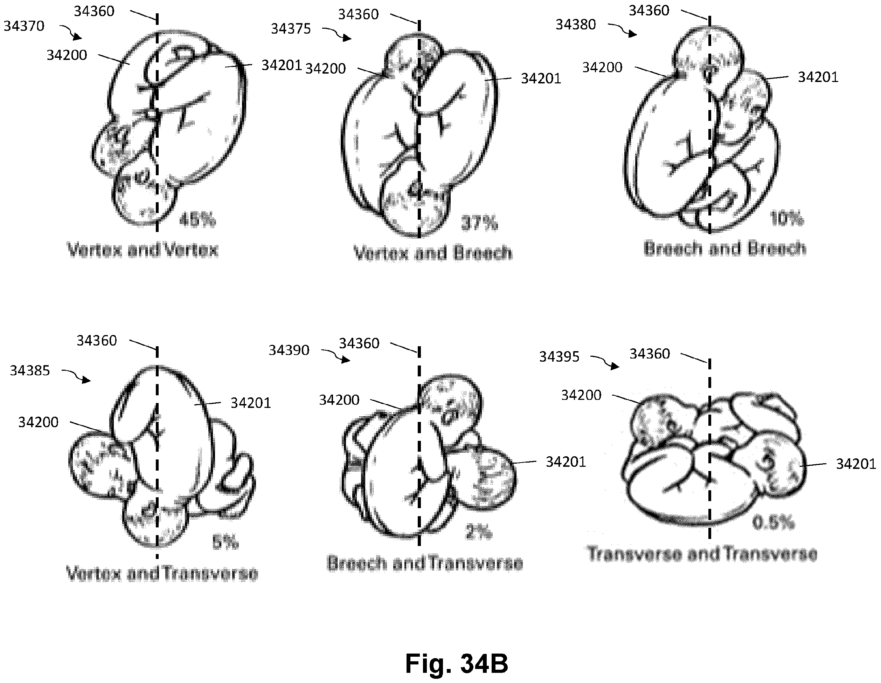

Fig. 34B is a set of schematic, diagrammatic, cross-sectional views of two fetuses 34200, 34201 arranged in different positions within a uterus of a patient as seen by an ultrasound imaging plane 34360, according to aspects of the present disclosure. The imaging plane 34360 is example of an imaging plane (e.g., for one frame during one sweep), and the same imaging plane shown in all fetal positions for twins, and indicates that different fetal parts will be visible in the same imaging plane depending on which fetal position twins are in.

In a first example 34370, both twins 34200, 34201 are in a vertex (longitudinal, head-down) position. Approximately 45% of twin pregnancies fall into this category. In example 34370, the imaging plane 34360 may not include any duplicate anatomy.

In a second example 34375, one twin 34200 is in a breech (head-up) position, and the other is in a vertex (head-down) position. Approximately 37% of twin pregnancies fall into this category. Breech births are associated with a higher rate of complications. In example 34375, the imaging plane 34360 includes the heads of both fetuses 34200, 34201.

In a third example 34380, both twins 34200, 34201 are in a breech (head-up) position. Approximately 10% of twin pregnancies fall into this category. In example 34380, the imaging plane 34360 may not contain any duplicate anatomy.

In a fourth example 34385, one twin 34200 is in a transverse (sideways) position, and the other twin 34201 is in a vertex position. Approximately 5% of twin pregnancies fall into this category. Vertex births are associated with a higher rate of complications. In example 34385, the imaging plane 34360 may include the hearts of both fetuses 34200, 34201.

In a fifth example 34390, one twin 34200 is in the breech (head-up) position, and the other twin 34201 is in the transverse (sideways) position. Approximately 2% of twin pregnancies fall into this category. In example 34390, the imaging plane 34360 may include the hearts of both fetuses 34200, 34201.

In a sixth example 34395, both twins 34200, 34201 are in the vertex position. Approximately 0.5% of twin pregnancies fall into this category. In example 34395, the imaging plane 34360 may include the hearts of both fetuses 34200, 34201.

The positions shown in Fig. 34B may also be referred to as fetal orientation, fetal presentation, or fetal lie.

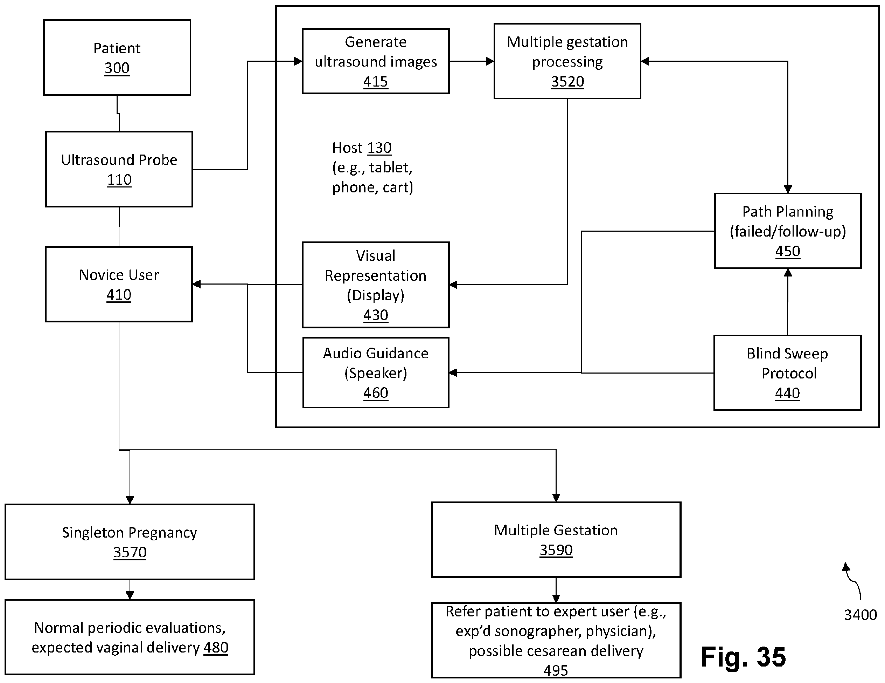

Fig. 35 is a schematic, diagrammatic representation, in hybrid block diagram/flow diagram form, of an example multiple gestation analysis system 3400, according to aspects of the present disclosure. An ultrasound probe 110 operated by a novice user 410 performs a blind sweep protocol 440 on the body of a patient 300 and sends ultrasound imaging data to a host 130, such as a tablet, smartphone, ultrasound cart, etc. In step 415, the host 130 generates ultrasound images using the ultrasound image data obtained by the ultrasound probe 110. The host 130 can control the ultrasound probe 110 to obtain the ultrasound image data (e.g., the host 130 establishes communication with the ultrasound probe 110, the host 130 sends control signals to start and/or stop acquisition of ultrasound image data, the host 130 sends power signals to power the ultrasound probe 110, etc.).

In step 3520, the host 130 uses the ultrasound images generated in step 415 and/or the ultrasound image data used to generate the ultrasound images to determine multiple gestation, as described in more detail below.

In step 450, the host 130 determines whether the planned path of the blind sweep protocol 440 has been followed adequately. If no, execution proceeds to steps 430 and 460, wherein the host 130 provides feedback (e.g., audio feedback through a speaker and/or visual feedback from a display) to repeat one or multiple sweeps of the blind sweep protocol 440. In some aspects, the determination in step 450 can be based on the results of analysis in step 420 of the fetal lie and/or in step 435 of the fetal spine's location. For example, if there is insufficient ultrasound image data to perform the determinations in step 3520, or if the ultrasound image data that has been obtained is not suitable to perform the determinations in step 3520, then step 450 can determine that the one or multiple sweeps of the blind sweep protocol 440 has not been completed correctly. The one or multiple sweeps that need to be performed again can be communicated to the user through visual or audio feedback in steps 430 and 460.

In some aspects, the planned path determined in step 450 of the blind sweep protocol 440 can be used by the host 130 to determine multiple gestation in step 3520. For example, the planned path determined in step 450 can provide absolute or relative spatial information (e.g., vertical location, horizontal location) about the ultrasound image data obtained by the ultrasound probe and/or the ultrasound images generated by the host. For example, referring again to Fig. 4, the planned path determined in step 450 can indicate that the M sweep is horizontally located (left-right direction in Fig. 4) between the R sweep and the L sweep. The host 130 can use this spatial information from step 450 to process the ultrasound image data and/or ultrasound images to determine the multiple gestation in step 3520.

Based on the visual representation 430, the novice user 410 makes a triage decision to either rule out or rule multiple gestation. In step 3570, the novice user 410 rules out multiple gestation (e.g., determines that the pregnancy is a singleton pregnancy), and in step 480, the novice user 410 performs or recommends normal periodic evaluations for the patient 300. In step 3590, the novice user 410 rules in multiple gestation, and in step 495, the novice user 410 refers the patient to an expert user such as an experienced sonographer, radiologist, obstetrician, or other physician for follow-up imaging and diagnosis.

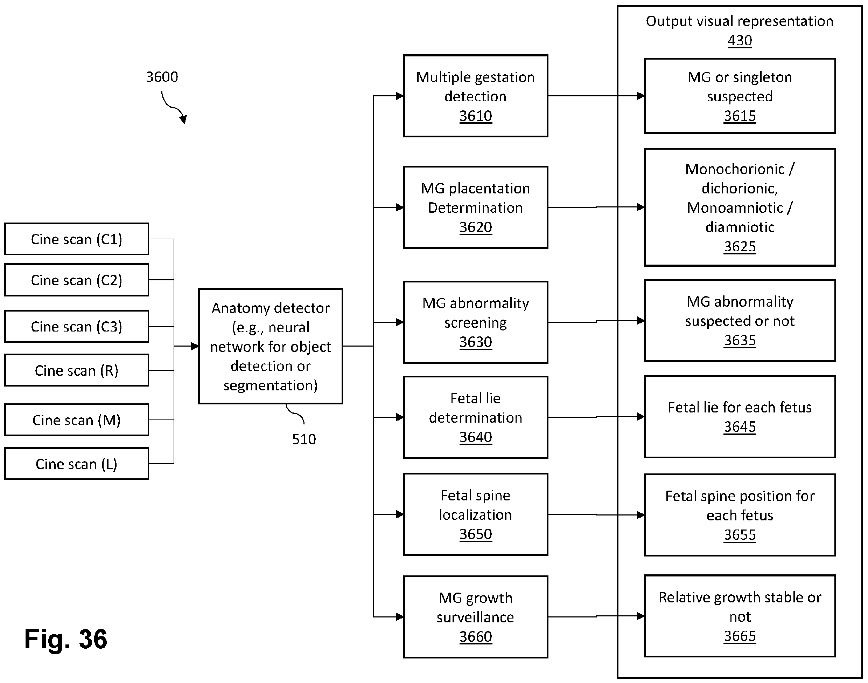

Fig. 36 is a schematic, diagrammatic representation, in hybrid block diagram/flow diagram form, of an example fetal orientation determination method 3600 to triage and/or monitor multiple gestation, according to aspects of the present disclosure. Images from a sweep of the blind sweep protocol can be organized into an image sequence known as a cine scan or cineloop (e.g., multiple image frames making up a video segment). The host thus receives or assembles cine scans C1, C2, C3, R, M, and L, corresponding to the blind abdominal sweeps C1, C2, C3, R, M, and L shown in Fig. 36.

In step 510, these cine scans are fed into an object detector (e.g., a trained neural network) to detect the presence of and, if present, the location of the anatomical region of interest (e.g., fetal head, fetal spine, etc.) in each ultrasound image frame of the cine scan. Depending on the implementation, execution then proceeds to any or all of steps 3610, 3620, 3630, 3640, 3650, or 3660.

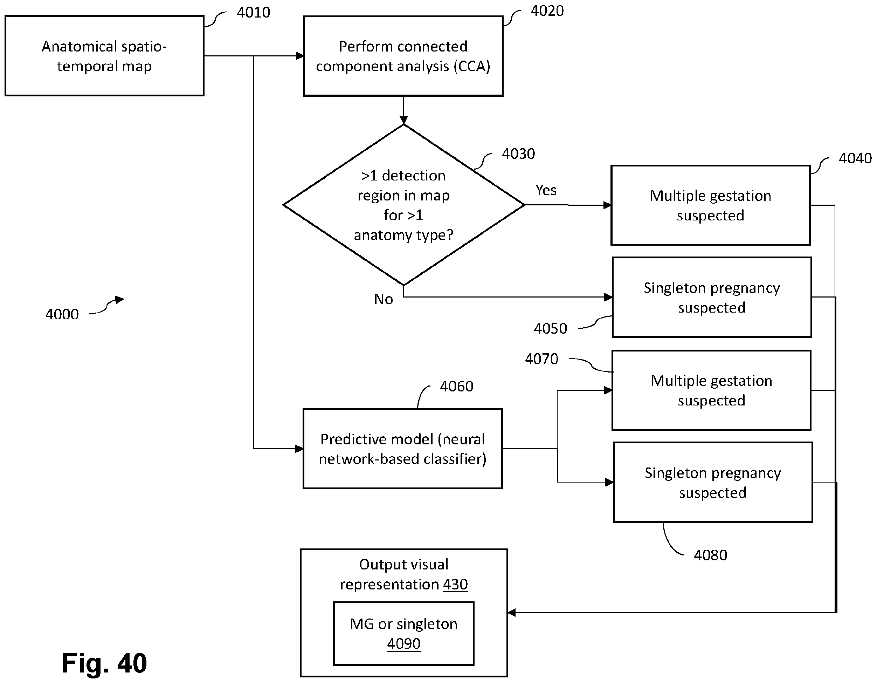

In step 3610, the method 3600 includes performing multiple gestation detection, to determine whether the pregnancy is multiple gestation or a singleton pregnancy, as described in more detail in Figs. 40-41. below. Execution then proceeds to step 3615.

In step 3615, the method 3600 includes outputting a visual representation of whether multiple gestation or a singleton pregnancy is suspected.

In step 3620, the method 3600 includes determining placentation for MG pregnancies, as described in Figs. 34A, above, and 44, below). Execution then proceeds to step 3625.

In step 3625, the method 3600 includes outputting a visual representation indicating whether the MG pregnancy is monochorionic or dichorionic, and whether it is monoamniotic or diamniotic.

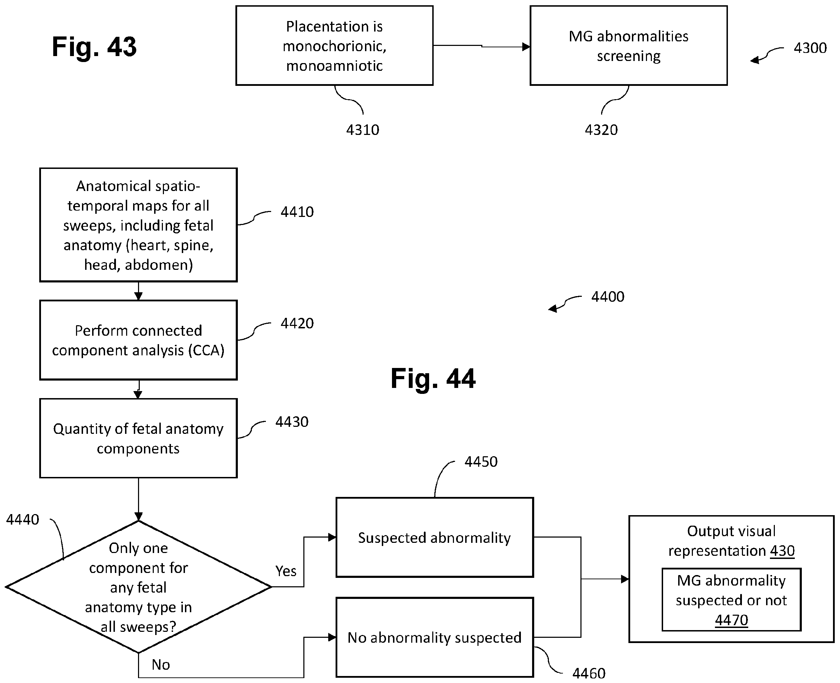

In step 3630, the method 3600 includes multiple gestation abnormality screening, as described below in Figs. 43-45. Execution then proceeds to step 3635.

In step 3635, the method 3600 includes outputting a visual representation indicating whether a multiple gestation abnormality is suspected or not.

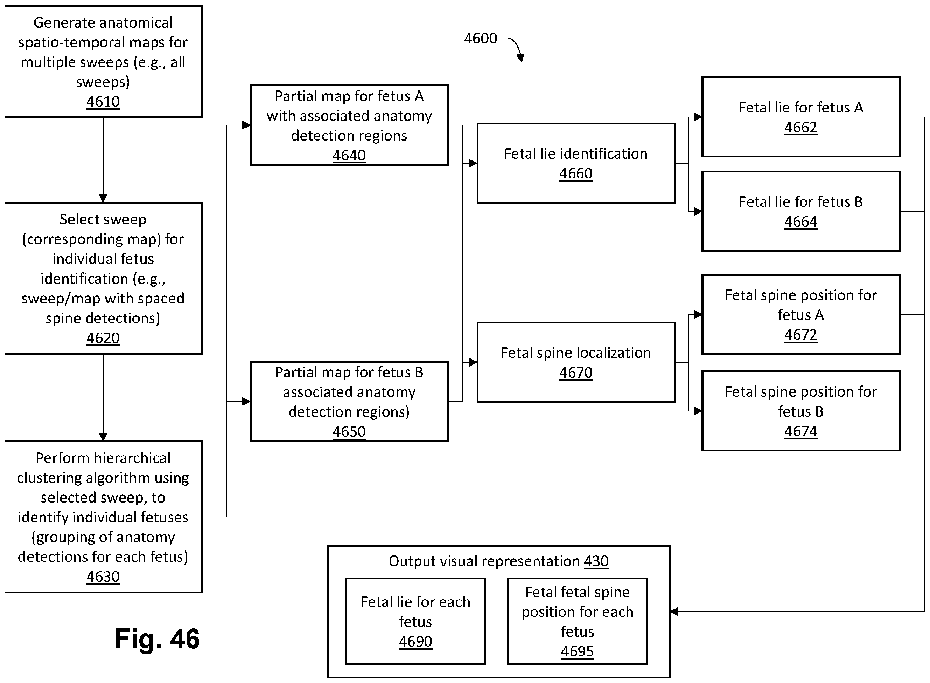

In step 3640, the method 3600 includes fetal lie determination for all fetuses in the multiple gestation, as described in Figs. 12A to 23, above and Figs. 46-48, below. Execution then proceeds to step 3645.

In step 3645, the method 3600 includes outputting a visual representation indicating the fetal lie for each fetus.

In step 3650, the method 3600 includes fetal spine localization for all fetuses in the multiple gestation, as described above in Figs. 24 to 37C, and below in Figs. 46-48. Execution then proceeds to step 3655.

In step 3655, the method 3600 includes outputting a visual representation indicative of the fetal spine position for each fetus.

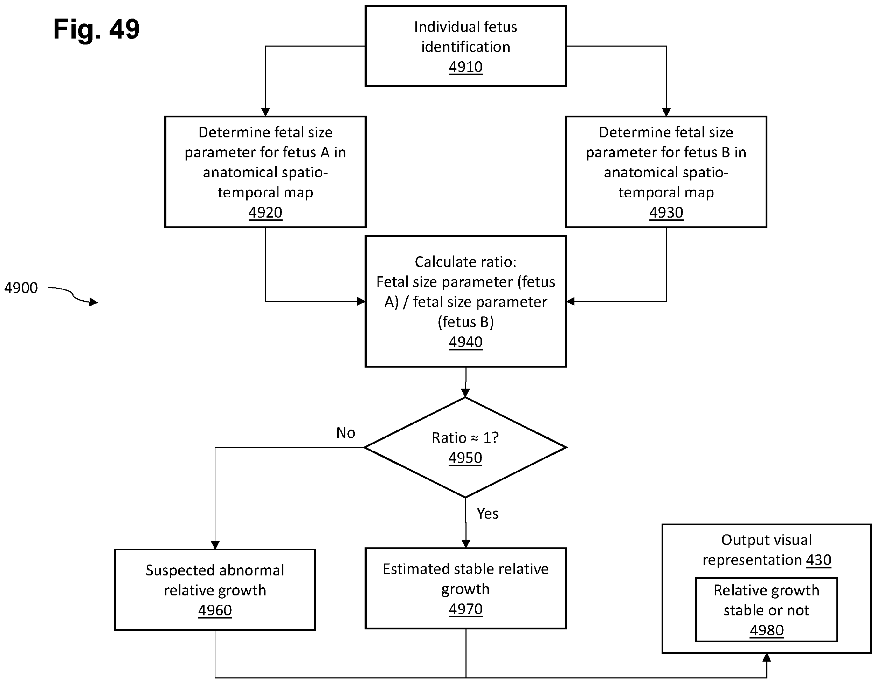

In step 3630, the method 3600 includes multiple gestation growth surveillance, as described below in Figs. 49-50. Execution then proceeds to step 3665.

In step 3655, the method 3600 includes outputting a visual representation of whether the relative growth of the fetuses is stable or not.

Depending on the implementation, the visual representation 430 can include text labels or images/graphics (including both not limited to the images/graphics described herein), and/or combinations or thereof. The anatomy detector 510 can identify fetal anatomy (fetal head, fetal spine, fetal heart, fetal abdomen, fetal urinary bladder) and/or maternal anatomy (placenta, amniotic fluid, maternal spine, etc.). Inputs to the anatomy detector 510 are ultrasound image frames from horizontal sweep(s) and/or vertical sweep(s). For example, each sweep can be a cine scan (e.g., an ultrasound video made up of multiple ultrasound image frames). The output of the anatomy detector 510 can depend on the type of predictive model/network being used. For an objection detection neural network, the output may include one or more bounding boxes identifying anatomy. For a segmentation neural network, the output may include the contour or perimeter of the anatomy.

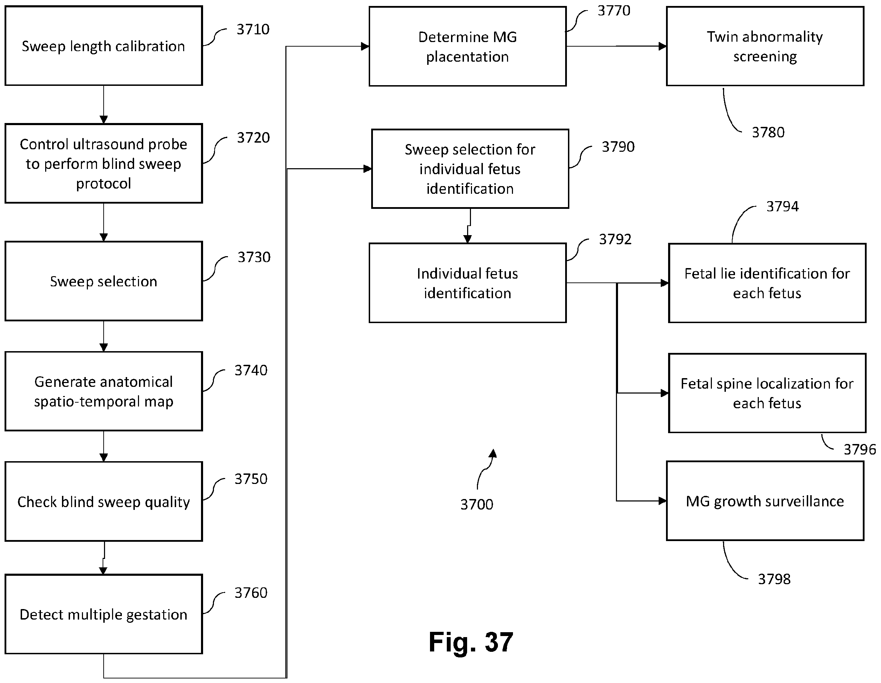

Fig. 37 is a schematic, diagrammatic representation, in hybrid block diagram / flow diagram form, of an example multiple gestation analysis method 3700, according to aspects of the present disclosure.

In step 3710, the method 3700 includes performing sweep length calibration, as described for example in Figs. 28-32, above. Execution then proceeds to step 3720.

In step 3720, the method 3700 includes controlling the ultrasound probe to perform the blind sweep protocol, as described above in Fig. 4. Execution then proceeds to step 3730.

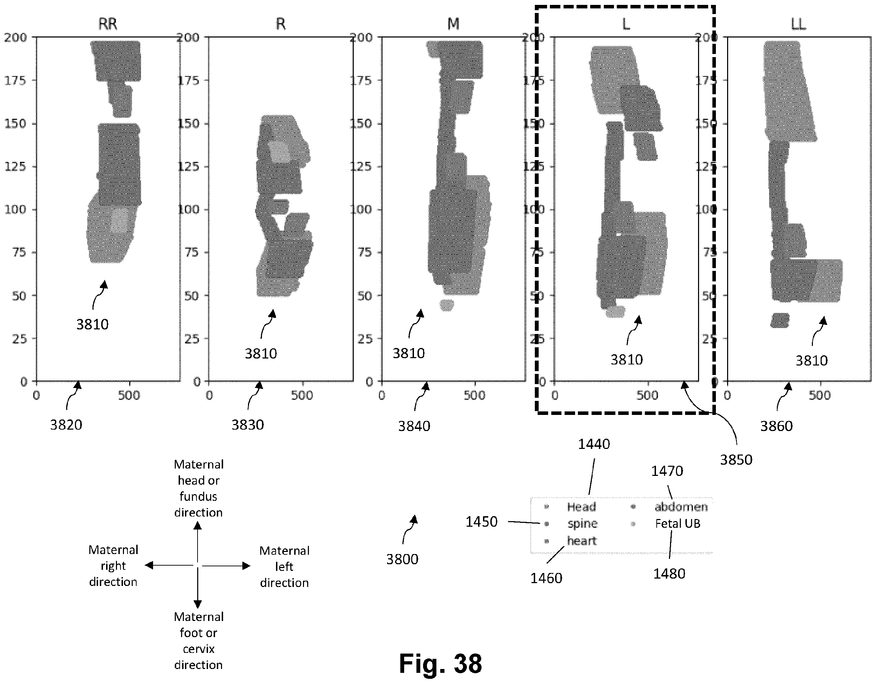

In step 3730, the method 3700 includes performing sweep selection, as described for example in Figs. 18-21, above, and Fig. 38, below. Execution then proceeds to step 3740.

In step 3740, the method 3700 includes generating an anatomical spatio-temporal map, as described for example in Figs. 11-15B, above. Execution then proceeds to step 3750.

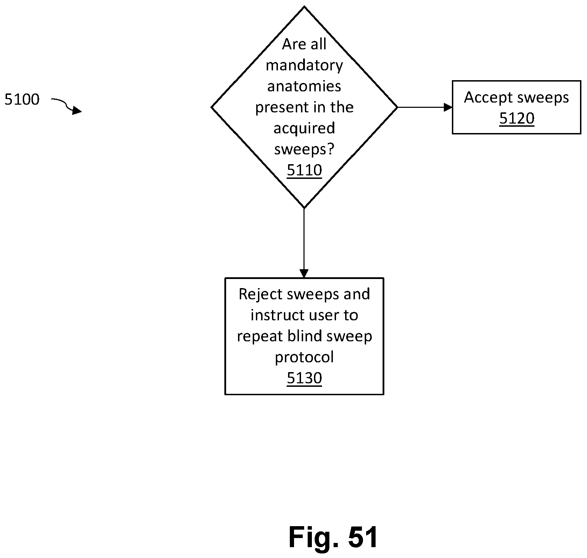

In step 3750, the method 3700 includes checking the blind sweep quality, as described for example in Figs. 51-52, below. Execution then proceeds to step 3760.

Blind sweep calibration ensures the scan positions (start and end) are correct so that the scan doesn't miss any anatomy due to positioning errors. This ensures quality of scans with respect to the blind sweep protocol. Conversely, scan quality check ensures the quality with respect to the images captured. Even if the scan passes the protocol-based quality check, there may still be a chance that image/scan captured is full of shadow or otherwise non-usable. In this scenario, there is a possibility of failure of the object detector model. Once object detector model fails to detect the required anatomy (e.g. head, heart etc.), the system can detect this issue from the anatomical map. As a result, the system can reject the scan and skip it to pass forward in the flow. This quality check ensures the algorithms to work on clean scans, thus leading to confident results.

In step 3760, the method 3700 includes detecting multiple gestation, as described for example in Figs. 40-41, below. Execution then proceeds to steps 3770 and 3790.

I n step 3770, the method 3700 includes determining placentation for the multiple gestation pregnancy, as described for example in Figs. 34A, above, and 42, below. Execution then proceeds to step 3780.

In step 3780, the method 3700 includes performing twin abnormality screening, as described for example in Figs. 43-45, below.

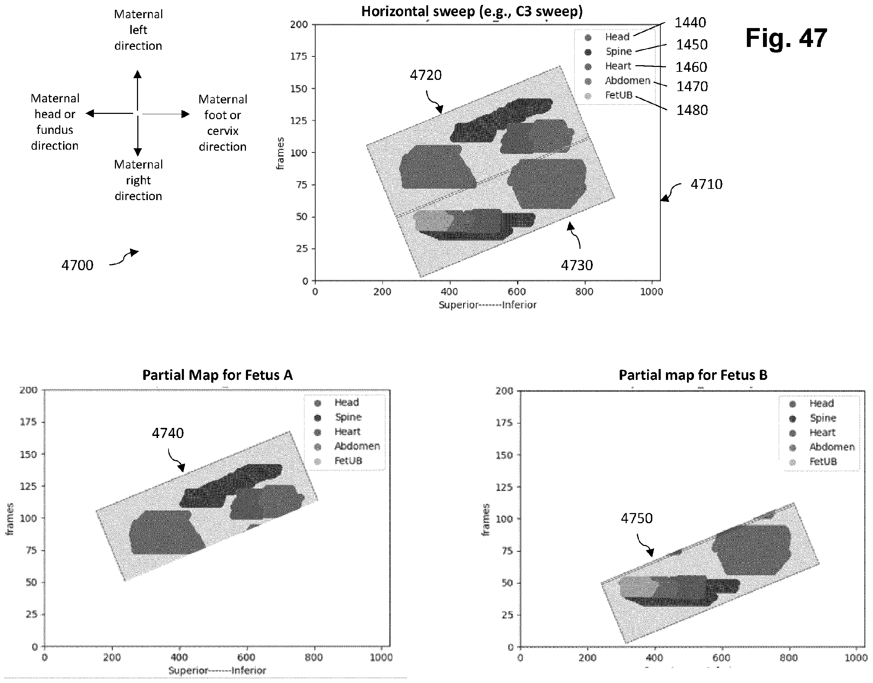

In step 3790, the method 3700 includes performing sweep selection for individual fetus identification, as described for example in Figs. 46 and 47, below. Execution then proceeds to step 3792.

In step 3792, the method 3700 includes performing individual fetus identification, as described for example in Figs. 46-48, below. Execution then proceeds to step 3794, 3796, and 3798.

In step 3794, the method 3700 includes identifying the fetal lie for each fetus, as described for example in Figs. 16-23 and 46.

In step 3796, the method 3700 includes performing fetal spine localization for each fetus, as described for example in Figs. 24-27C and 46.

In step 3798, the method 3700 includes performing multiple gestation growth surveillance, as described for example in Figs. 49-50, below.

The method 3700 is now complete.

Fig. 38 is a schematic, diagrammatic representation of a multiple gestation sweep selection process 3800 for vertical sweeps, according to aspects of the present disclosure. Visible are anatomy detections 3810 within 2D anatomical spatio-temporal maps for each of an RR sweep 3820, an R sweep 3030, an M sweep 3840, an L sweep 3850, and an LL sweep 3860. The sweep selection can employ a count-based technique (quantity of detections and/or quantity of anatomy types that are detected) and/or where the anatomy detections begin/end (as described above in Figs. 10, 18, 20, and 22). In the example shown in Fig. 38, the L sweep 3850 is selected, because it shows two separate detections for the head 1440, spine 1450, heart 1460, and abdomen 1470, because it has the largest total number of detections of all of the sweeps, and because its detections occur well clear of the beginning and end of the sweep (e.g., at least 5 frames away from the beginning or end of the sweep). Thus, the L sweep 3850 is more useful than the other sweeps for performing tasks such as fetal lie determination for both fetuses.

Another possibility of the scan which could have been selected is R. If the head and heart are visible then the system can expect two abdomens and urinary bladders as well to be present in the scans, thus showing maximum anatomies compared to other scans. Along with the presence of anatomies, the system can additionally keep track of the total detection count as well, which can be reused in later stages of the process.

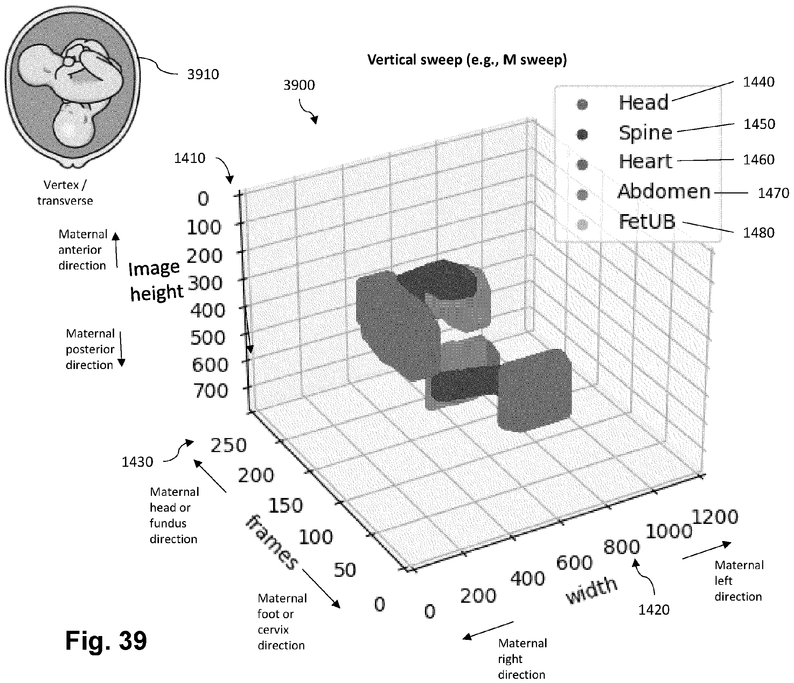

Fig. 39 is a 3D anatomical spatio-temporal map 3900 of the fetal anatomy from a vertical sweep (e.g., an M sweep), according to aspects of the present disclosure. The spatio-temporal map includes three coordinate axes: the image height axis 1410 (spanning the maternal anterior-posterior axis), image width axis 1420 (spanning the material left-right axis), and frame number 1430 (spanning the maternal cranial-caudal axis or head-foot axis). Anatomy detections 1499 are filled in by converting each anatomy detection to a mask (like segmentation mask generation, where foreground indicates 1 (white) and background 0 (black)) for each anatomy type, for each frame number 1430 of the map. In the example shown in Fig. 14, frame number 100 contains a rectangle representing a detection mask or bounding box representing a first fetal head 1440, whereas 200 contains a rectangle representing a detection mask or bounding box representing a second fetal head 1440. Thus, by summing the masks for each anatomy type and frame number, the 3D anatomical spatio-temporal map 1400 generates a 3D representation of the locations of fetal anatomy from multiple (e.g., two) fetuses within the patient's body, which can then be used to calculate the fetal lie and/or fetal presentation for each fetus. For example, in the example shown in Fig. 39, one fetus is in a vertex or longitudinal-cranial lie (see Fig. 3A), while the other fetus is in a transverse lie.

An illustration 3910 showing the vertex and transverse orientations is shown to aid the reader's understanding; a priori, the processor and the user doesn't know that this is a MG, or the particular fetal lie of each fetus. The processing steps described herein allow for determination that MG is suspected (e.g., based on the anatomical spatio-temporal map of the sweep) and/or the particular lie of each fetus (as described below in Fig. 46).

In some aspects, a 2D projection of the 3D spatio-temporal map 1400 can be displayed to the user. In case user wants to interact with the visualization map , then 3D spatio-temporal map can be displayed, where the system may include a user input to rotate the map 1400 to different viewing angles, and/or a user input to turn on/off anatomy detection regions (e.g., to turn off the placenta region and amniotic fluid region if the user only wants to see fetal anatomies).

Fig. 40 is a schematic, diagrammatic representation, in hybrid block diagram / flow diagram form, of an example multiple gestation detection method 4000, according to aspects of the present disclosure.

In step 4020, the method 4000 includes performing connected component analysis (CCA) on the anatomical spatio-temporal map 4010 from a selected sweep. Connected component analysis (CCA), also known as connected component labeling (CCL), is an algorithmic technique that identifies and labels connected regions in an image, by using graph theory to construct a graph from the image data. Vertices of the graph represent information and edges represent connections between vertices. The algorithm then labels the vertices based on the connectivity and values of their neighbor. The output of the CCA process may for example be the identification of fetal head detection region(s), fetal abdomen detection region(s), fetal spine detection region(s), fetal urinary bladder detection region(s), etc. within the anatomical spatio-temporal map 4010. Execution then proceeds to step 4030.

In step 4030, the method 4000 includes determining whether there is more than one detection region or connected component in the spatio-temporal map 4010 for more than one type of anatomy. In an example, there may be two head detection regions and two abdomen detection regions. If yes, execution proceeds to step 4040. If no, execution proceeds to step 4050.

In step 4040, the method 4000 includes determining that multiple gestation is suspected. Execution now proceeds to step 430.

In step 4050, the method 4000 includes determining that a singleton pregnancy is suspected. Execution now proceeds to step 4030.

Alternatively or in addition to step 4020, in step 4060, the method includes using a predictive model (e.g., a trained, neural network-based classifier) to receive the anatomical spatio-temporal map 4010 and output a determination of whether multiple gestation is suspected (step 4070), or singleton pregnancy is suspected (step 4080). Examples of a classifier include Convolutional Neural Networks (CNNs), Transformer, Support Vector Machines (SVMs), each operating on different data types and classification tasks depending on their architecture. Execution then proceeds to step 430.

In step 430, the method 4000 includes outputting a visual representation of the suspected singleton or MG pregnancy 4090. The method 4000 is now complete.

In some aspects, an ensemble approach may be used, that employs both CCA and a predictive model to determine multiple gestation vs. singleton pregnancy.

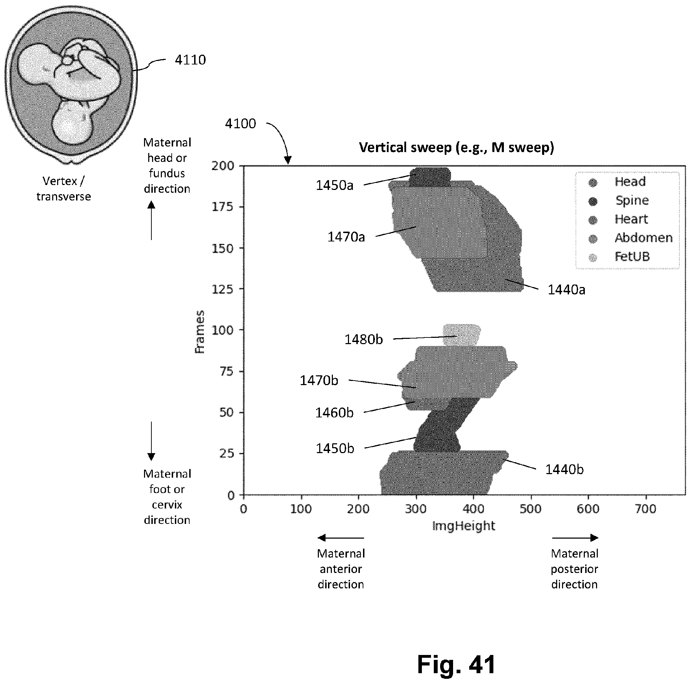

Fig. 41 is a 2D anatomical spatio-temporal map 4100 for a vertical sweep (e.g., an M sweep), according to aspects of the present disclosure. In the example shown in Fig. 41, the 2D anatomical spatio-temporal map 4100 is a projection or cross-section of the 3D anatomical spatio-temporal map 3900 of Fig. 39, in the frame number / image height plane. Visible are detection regions for two fetal heads 1440a and 1440b, two fetal spines 1450a and 1450b, two fetal hearts 1460a and 1460b, two fetal abdomens 1470a and 1470b, and one fetal urinary bladder 1480b. In this example, as shown in explanatory drawing 4110, one fetus is in a vertex (longitudinal, head-down) lie and one is in a transverse lie.

Drawing 4110 is added to aid the reader's understanding; a priori, the system and the user doesn't know that the pregnancy is a multiple gestation or the particular fetal lie of each fetus. The processing steps described herein allow for determination that MG is suspected (e.g., based on the anatomical spatio-temporal map of the selected sweep) and/or the particular lie of each fetus (e.g., as described below in Fig. 46).

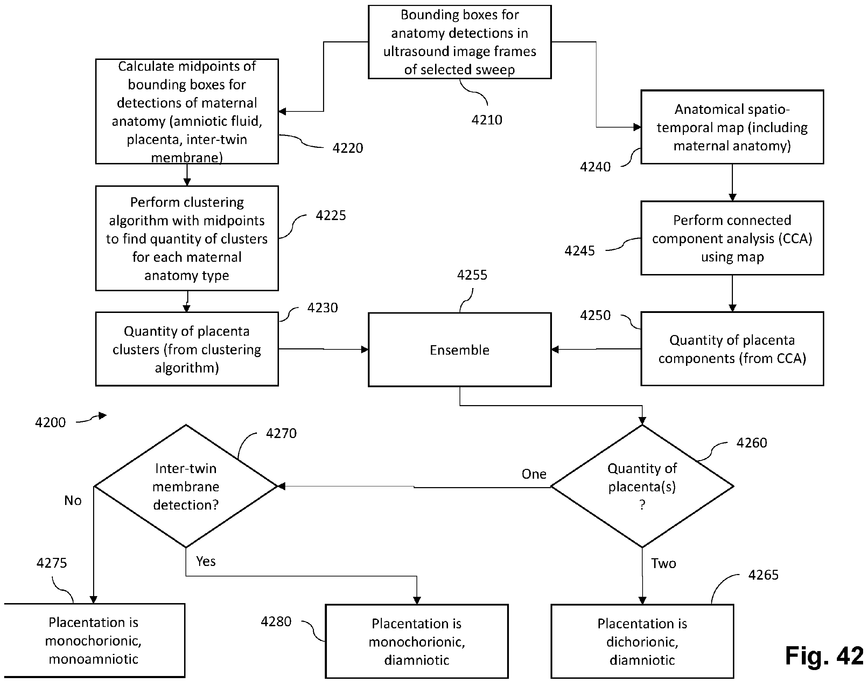

Fig. 42 is a schematic, diagrammatic representation, in hybrid block diagram / flow diagram form, of an example fetal placentation determination method 4200 for multiple gestation pregnancies, according to aspects of the present disclosure. For types of placentation, refer to Fig. 34A.

In step 4220, the method 4200 includes calculating the midpoints of the bounding boxes 4210 from maternal anatomy detections in the selected sweep. The maternal anatomy detections may for example include amniotic fluid, placenta, and inter-twin membrane detections. Execution then proceeds to step 4225.

In step 4225, the method 4200 includes performing a clustering algorithm with the midpoints from step 4220, to find a quantity of clusters for each maternal anatomy type, including a quantity of placenta clusters 4230 determined by the clustering algorithm. Examples of clustering algorithms include K-Nearest Neighbors (KNN, a supervised learning algorithm) and density-based spatial clustering of applications with noise (DBSCAN, a clustering algorithm used in machine learning to partition data into clusters based on their distance to other points). In some aspects, a segmentation algorithm may be used instead of or in addition to the clustering algorithm. Execution then proceeds to step 4255.

In step 4240, the method 4200 includes generating an anatomical spatio-temporal map that includes maternal anatomy such as amniotic fluid, the placenta, and an inter-twin membrane. Execution then proceeds to step 4245.

In step 4245, the method 4200 includes performing connected component analysis (CCA) using the anatomical spatio-temporal map of step 4240, to determine quantities of each maternal anatomy type, including a quantity 2450 of placenta components 2520. It is noted that in some cases, CCA may already have been performed (e.g., as shown in Fig. 40), in which case step 4245 includes retrieving the already-performed CCA. Execution then proceeds to step 4250.

In step 4250, the method 4200 includes generating an ensemble combination (e.g., an average, a maximum, a minimum, etc.) of the quantities 4230 and 4250 to determine a number of placentas. The ensemble approach can include weighting (e.g., weighting the result of the clustering algorithm greater than or less than the result of CCA, and vice versa). For example, if the CCA is weighted more than clustering algorithm, and CCA output is 2 placenta and clustering algorithm output is 1 placentas, then the output of the ensemble can be 2 placentas (favoring the CCA output because CCA is weighted more). Execution then proceeds to step 4260.

In step 4260, the method 4200 includes assessing the quantity of placentas. If the quantity is one, execution proceeds to step 4270. If the quantity is two, execution then proceeds to step 4265.

In step 4265, the method 4200 includes determining that the placentation is dichorionic, diamniotic. The method 4200 is now complete.

In step 4270, the method 4200 includes determining whether an inter-twin membrane was detected (e.g., if the quantity of inter-twin membranes detected is greater than zero). If yes, execution proceeds to step 4280. If no, execution proceeds to step 4275.

In step 4275, the method 4200 includes determining that the placentation is monochorionic, monoamniotic. The method 4200 is now complete.

In step 4280, the method 4200 includes determining that the placentation is monochorionic, diamniotic. The method 4200 is now complete.

Fig. 43 is a schematic, diagrammatic representation, in flow diagram form, of an example multiple gestation abnormalities determination method 4300, according to aspects of the present disclosure. In step 4310, the placentation is monochorionic, monoamniotic (e.g., from step 4275 of Fig. 42). Execution then proceeds to step 4320. In step 4320, the method 4300 includes performing a multiple gestation abnormalities screening, as described for example in Fig. 44, below. The method 4300 is now complete.

Fig. 44 is a schematic, diagrammatic representation, in hybrid block diagram / flow diagram form, of an example multiple gestation abnormalities determination method 4400, according to aspects of the present disclosure.

In step 4410, the method 4400 includes generating anatomical spatio-temporal maps for all sweeps, including fetal anatomy detections (e.g., head, spine, heart, abdomen, and urinary bladder). Execution then proceeds to step 4420.

In step 4420, the method 4200 includes performing connected component analysis (CCA) on all of the maps from step 4410, to determine quantities 4430 for each of the fetal anatomy components. Execution then proceeds to step 4440.

In step 4440, the method 4400 includes determining whether there is only one component for any fetal anatomy type in all of the sweeps. For example, if only one head is detected in all of the sweeps for a multiple gestation pregnancy whereas other anatomies appear twice, this could be indicative of fused heads. Similarly, detection of only a single abdomen could be indicative of fused abdomens. If yes, execution proceeds to step 4450. If no, execution proceeds to step 4460.

In step 4450, the method 4400 includes determining that an abnormality is suspected. Execution then proceeds to step 430.

In step 4460, the method 4400 includes determining that no abnormality is suspected. Execution then proceeds to step 430.

In step 430, the method 4400 includes outputting a visual representation indicating whether a multiple gestation abnormality is suspected or not 4470. Providing this output lets the user (e.g., a midwife) refer the patient to a trained sonographer or clinician to perform additional imaging and diagnosis. The method 4400 is now complete.

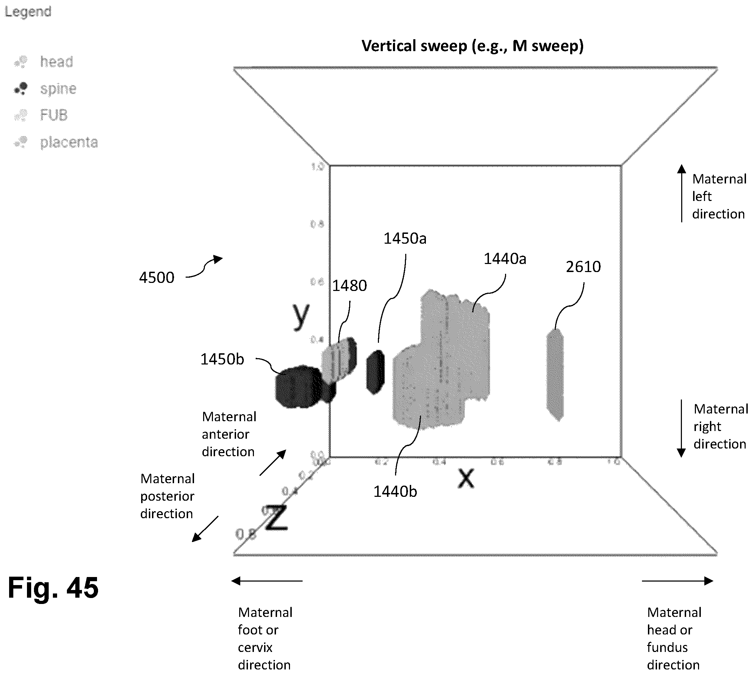

Fig. 45 is a 3D spatio-temporal map 4500 of a vertical sweep (e.g., an M sweep), according to aspects of the present disclosure. Visible are two spine detection clusters 1450a and 1450b, one urinary bladder detection cluster 1480, one placenta detection cluster 2610, and two head detection clusters 1440a and 1440b. Because the head detection clusters 1440a and 1440b do not appear to be fully separated from one another, this may be indicative of fused fetal heads and therefore a gestational anomaly, as described above in Fig. 44. In this case, the user (e.g., a midwife) can refer the patient to an expert (e.g., a sonographer or clinician) for imaging, diagnosis, and appropriate treatment/planning to minimize risk.

Fig. 46 is a schematic, diagrammatic representation, in hybrid block diagram / flow diagram form, of an example individual fetus identification method 4600, according to aspects of the present disclosure.

In step 4610, the method 4600 includes generating anatomical spatio-temporal maps for multiple sweeps (e.g., for all sweeps). Execution then proceeds to step 4620.

In step 4620, the method 4600 includes selecting a sweep (and its corresponding spatio-temporal map) for individual fetus identification. This may for example be a sweep that contains spaced detections of two different spines, or the sweep may be selected by count-based methods as described above.

In some aspects, for individual fetus identification, the two spines have to be spaced far enough apart to individually identify the fetuses. For example, the distance (e.g., a pixel distance in the anatomical map) between the two spine detection regions may have to satisfy a threshold distance (greater than or equal to the threshold distance), although other measurements could be used instead or in addition to provide adequate separation between the two detected fetuses in the selected sweep.

Execution then proceeds to step 4630.

In step 4630, the method 4600 includes performing a hierarchical clustering algorithm using the selected sweep, to identify individual fetuses (e.g., the grouping of anatomy detections for each fetus). This can then be used to generate a partial map 4640 of fetus A with associated anatomy detection regions, and a partial map 4650 of fetus B with associated anatomy detection regions. Execution then proceeds to steps 4660 and 4670.

In step 4660, the method 4600 includes fetal lie identification as described above in Fig. 16 but using the partial maps 4640, 4650 for each fetus, which yields a fetal lie 4662 for fetus A and a fetal lie 4664 for fetus B. Execution then proceeds to step 430.

In step 4670, the method 4600 includes fetal spine localization 4670, as described above in Fig. 24, but using the partial maps 4640, 4650 for each fetus, which yields a fetal spine position 4672 for fetus A and a fetal spine position 4674 for fetus B. Execution then proceeds to step 430.

In step 430, the method 4600 includes outputting a visual representation including the fetal lie for each fetus 4690 and the fetal spine position for each fetus 4695. The method 4600 is now complete.

Fig. 47 is a schematic, diagrammatic representation of an example individual fetus identification process 4700, according to aspects of the present disclosure. A selected sweep 4710 for individual fetus identification includes two head detection regions 1440, two spine detection regions 1450, two heart detection regions 1460, two abdomen detection regions 1470, and one urinary bladder detection region 1480. By the methods described above in Fig. 46, the anatomy detections can be segmented into fetus A detections 4720 and fetus B detections 4730, which can then be separated into a partial map 4740 for fetus A and a partial map 4750 for fetus B. These partial maps can then be used for calculation of fetal parameters related to each individual fetus, including fetal lie, spine position, gestational age, etc.

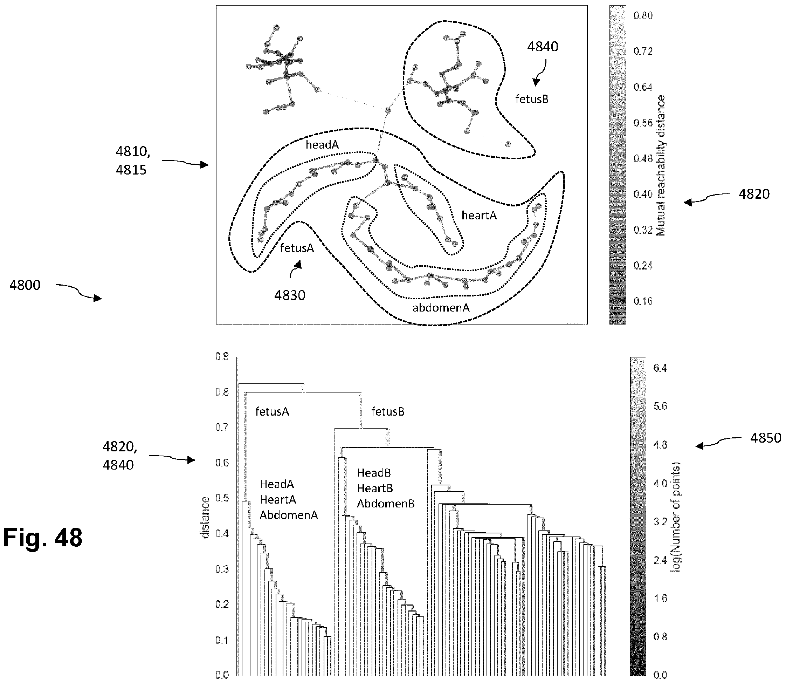

Fig. 48 is a diagrammatic representation of a density-based spatial clustering of applications with noise (DBSCAN) process 4800 using synthetic data points, according to aspects of the present disclosure. A plot 4810 of bounding box centroids 4815 by mutual reachability distance 4820 can be clustered into fetus A clusters 4830 and fetus B clusters 4840, which may for example include head A heart A, abdomen A, head B, heart B, abdomen B, and other related anatomical detections. These centroids can also be shown in a histogram plot 4840 relating mutual reachability distance 4820 to the logarithm of the number of points 4850 for each anatomy type. The labels shown in the graphs 4810, 4840 are also synthesized, and represent, as an example, how an anatomical spatio-temporal map with multiple gestation could be processed with HDBSCAN to identify fetuses individually.

Fig. 49 is a schematic, diagrammatic representation, in flow diagram form, of an example multiple gestation / twin growth surveillance method 4900, according to aspects of the present disclosure.

In step 4910, individual fetuses are identified as described above in Figs. 46-48. Execution then proceeds to steps 4920 and 4930.

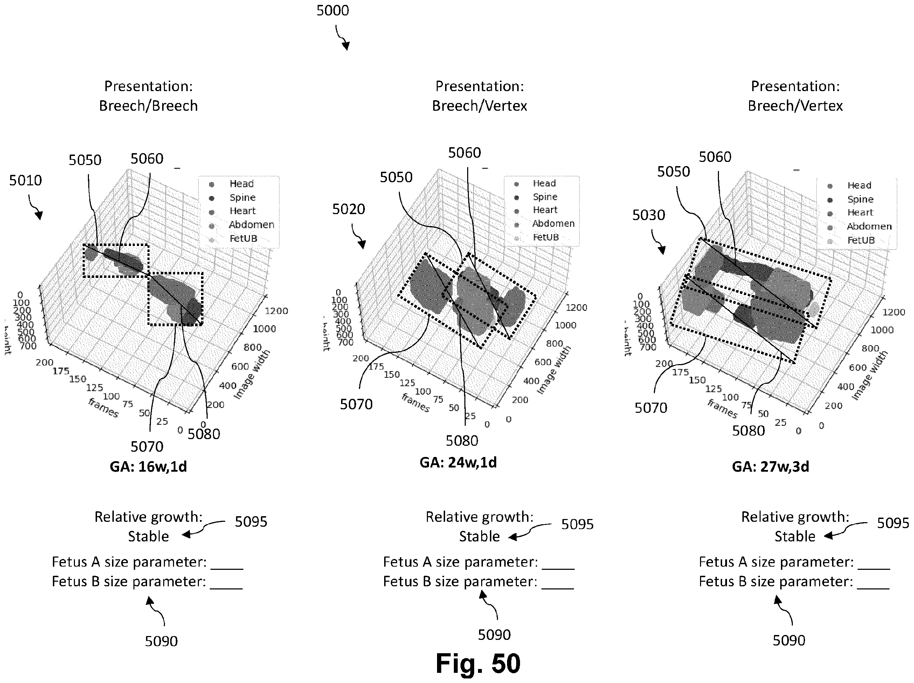

In step 4920, the method 4900 includes determining a fetal size parameter for fetus A in the anatomical spatio-temporal map of the selected sweep for individual fetus identification (see e.g., Fig. 47). Examples of fetal size parameter include (per Fig. 50, below) drawing one box around all anatomy detections for each fetus, and measuring the diagonal of each box. This is kind of a total length/size of the fetus, can be considered similar to the head-to-rump length (HRL) that is used by trained users who are trained to recognize anatomy and use digital calipers to measure fetus length. In another example (per Fig. 50, below), the system can measure geometrical properties (e.g., area for 2D map, volume for 3D map, etc.) of anatomy detection regions. For example, measuring the volume or area of the head detection region for fetus A, measuring the volume or area of abdomen detection region for fetus A, etc. Execution then proceeds to step 4940.

In step 4930, the method 4900 includes determining a fetal size parameter for fetus B in the anatomical spatio-temporal map of the selected sweep, as described above in step 4920 but for fetus B. Execution then proceeds to step 4940.

In step 4940, the method 4900 includes calculating a ratio of the fetal size parameters for fetus A and fetus B. In an example this ratio calculation is helps determine whether the two fetuses growing at the same rate. If so, then the fetal size parameters for the two fetuses will be approximately equal, resulting in a ratio approximately equal to 1. Execution then proceeds to step 4950.

In step 4950, the method 4900 includes determining whether the size parameter ratio between fetus A and fetus B is approximately equal to 1. This may mean, for example, being within a threshold of 20% or 25% of 1 (such that a growth discrepancy of 20-25% is indicative of an abnormal relative growth, e.g., one fetus is growing significantly faster than the other). If yes, the ratio is approximately equal to 1, execution then proceeds to step 4970. If no, the ratio is not approximately equal to 1, then execution proceeds to step 4960.

In step 4960, the method 4900 includes determining that there is a suspected abnormal relative growth. Thus, the user may want to consider referring the patient to a trained user (e.g., a trained sonographer or physician) for more detailed imaging. Execution then proceeds to step 430.

In step 4970, the method 4900 includes determining that there is estimated stable growth between the two fetuses. Execution then proceeds to step 430.

In step 430, the method 4900 includes outputting a visual representation including an indication 4980 of whether the relative growth between the two fetuses is stable or not.

Fig. 50 is an example screen display 5000 for multiple gestation grown surveillance, according to aspects of the present disclosure. The screen display 5000 may for example be a multiple gestation/twin growth monitoring report. The screen display 5000 can be shown to the user, so that the user (e.g., a midwife) can make decisions about delivery and/or referral to a physician. Previous scans for the patient can be stored in memory (e.g., in a patient database). To generate the MG growth monitoring report, the system can retrieve previous scans and results from those scans and combine them to generate the report.

The screen display 5000 includes three spatio-temporal maps 5010, 5020, and 5030, captured from selected sweeps (in this case, M sweeps) at three different gestational ages (in this case, 16 weeks, 24 weeks, and 27 weeks). Each map 5010, 5020, 5030 includes a bounding box 5050 drawn around fetus A, and a diagonal 5060 of the bounding box 5050 that can be used as a proxy for head-to-rump length (HRL) for fetus A, and reported as a size parameter 5090. Similarly, each map 5010, 5020, 5030 includes a bounding box 5070 for fetus B, and a diagonal 5080 that can be used as a proxy for the HRL of fetus B. Changes in the size parameters 5090 are therefore representative of changes in the length of the diagonals 5060 and 5080 over time, and are thus representative of the relative growth of fetus A and fetus B.

In other aspects, the size parameter may be a length, an area, or a volume, whether of the bounding box surrounding the fetus or of particular anatomical detections within the fetus. In either case, the size parameter is not an actual measurement of human anatomy (e.g., manual or automatic calipers on an ultrasound image), but rather are measurements or values of the anatomical spatio-temporal map (e.g., the length of the diagonal of the box around anatomies for an individual fetus, an area of a detection region, a volume of detection region, etc.).

The screen display 5000 thus provides the user with at-a-glance information about the size and growth of each fetus, as well as an indication 5095 of whether the relative growth of the fetuses is stable (e.g., whether the fetuses size parameters 5090 are within 20% or 25% of one another) or unstable (e.g., if the size parameters are different by more than 20% or 25%. Fig. 51 is a schematic, diagrammatic representation, in flow diagram form, of an example blind sweep quality check method 5100, according to aspects of the present disclosure.

In step 5110, the method includes determining whether the mandatory anatomies (e.g., head, heart, abdomen, spine, and bladder) are present in the acquired sweeps. If yes, execution proceeds to step 5120. If no, execution proceeds to step 5130.

In step 5120, the method includes accepting the blind sweeps. The method 5100 is now complete.

In step 5130, the method includes rejecting the blind sweeps and instructing the user to repeat the blind sweep protocol.

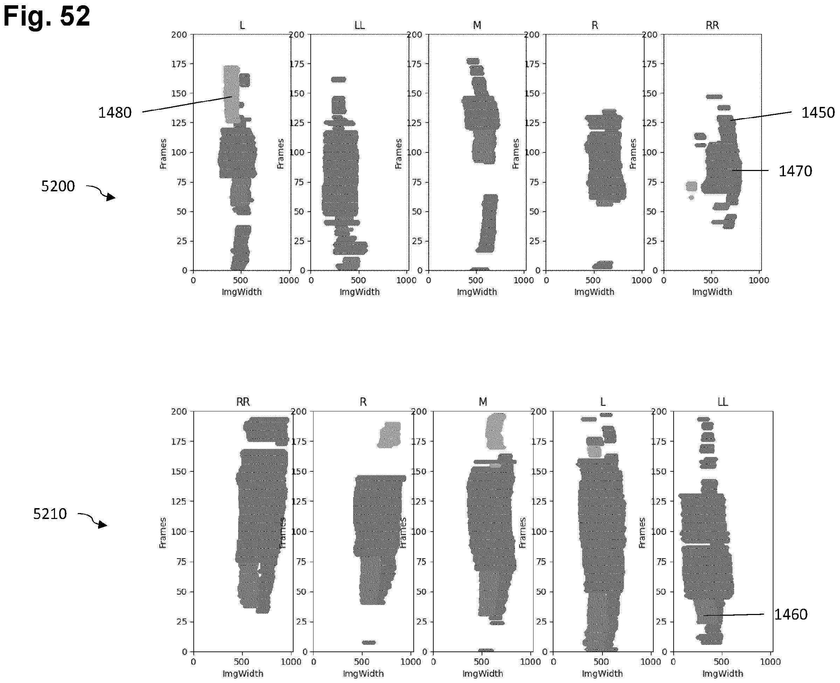

Fig. 52 shows two sets of vertical sweeps 5200, 5210 from a 5x5 blind sweep protocol, which can serve as inputs to the quality check method of Fig. 51, according to aspects of the present disclosure. Visible are the fetal spine 1450, fetal heart 1460, fetal abdomen 1470, and fetal urinary bladder 1480. Notably, none of the sweeps shows the fetal head. Since all required anatomies are not present in any of the sweeps, no sweep will be deemed acceptable, and the user will be prompted to repeat the blind sweep protocol.

This acquired data may have a scan protocol issue (e.g., the blind sweeps were performed incorrectly). However, it is also possible that the sweeps were performed correctly, but because shadows or other image artifacts, the object detector model might not be able to detect the required anatomy.

As will be readily appreciated by those having ordinary skill in the art after becoming familiar with the teachings herein, the multiple gestation analysis system advantageously permits untrained and minimally trained users to perform an ultrasound blind sweep protocol to gather reliable information about multiple gestation pregnancies. This may result in higher accuracy and higher clinician trust in the results, while potentially improving health outcomes and/or decreasing the total cost of care.

The systems, methods, and devices described herein may be applicable in point of care and handheld ultrasound use cases such as with the Philips Lumify system. The multiple gestation analysis system can be used for any handheld imaging applications, including but not limited to obstetrics, prenatal lung imaging, and prenatal echocardiography. The multiple gestation analysis system could be deployed on handheld mobile ultrasound devices, and on portable or cart-based ultrasound systems. The multiple gestation analysis system can be used in a variety of settings, including emergency departments, ambulances, accident sites, and homes. The applications could also be expanded to other settings. Thus, the present disclosure increases the value proposition of ultrasound applications in the obstetrics context, especially for use by minimally trained users.

A number of variations are possible on the examples and aspects described above. For example, the systems, methods, and devices described herein are not limited to obstetric ultrasound applications. Rather, the same technology can be applied to images of other organs or anatomical systems such as the lungs, heart, brain, digestive system, vascular system, tumors, etc. Furthermore, the technology disclosed herein is also applicable to other medical imaging modalities where 3D data are available, such as other ultrasound applications, camera-based videos, X-ray videos, and 3D volume images, such as computer aided tomography (CT) scans, magnetic resonance imaging (MRI) scans, or optical coherence tomography (OCT) scans.

Accordingly, the logical operations making up the aspects of the technology described herein are referred to variously as operations, steps, objects, layers, elements, components, algorithms, or modules. Furthermore, it should be understood that these may occur or be performed or arranged in any order, unless explicitly claimed otherwise or a specific order is inherently necessitated by the claim language.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the multiple gestation analysis system. Connection references, e.g., attached, coupled, connected, joined, or "in communication with" are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary aspects of the multiple gestation analysis system as defined in the claims. Although various aspects of the claimed subject matter have been described above with a certain degree of particularity, or with reference to one or more individual aspects, those skilled in the art could make numerous alterations to the disclosed aspects without departing from the spirit or scope of the claimed subject matter.

Still other aspects are contemplated. It is intended that all matter contained in the above description and shown in the accompanying drawings shall be interpreted as illustrative only of particular aspects and not limiting. Changes in detail or structure may be made without departing from the basic elements of the subject matter as defined in the following claims.

## Claims

1. A system, comprising:
a processor configured for communication with an ultrasound probe, wherein the processor is configured to:
control the ultrasound probe to obtain a plurality of ultrasound image frames during a blind sweep protocol on a patent with a pregnancy;
provide the plurality of ultrasound image frames as an input to a deep learning network trained to detect a plurality of fetal anatomies;
generate, as an output of the deep learning network, a plurality of detections of the plurality of fetal anatomies within the plurality of ultrasound image frames;
generate an anatomical map based on the plurality of fetal anatomies;
determine that the pregnancy is a multiple gestation based on the anatomical map; and
provide, to a display in communication with the processor, an output representative of the determination that the pregnancy is the multiple gestation.

2. The system of claim 1, wherein the processor is configured to: generate a plurality of binary masks based on the plurality of detections, wherein an individual binary mask is associated with an individual ultrasound image frame in a sweep of the blind sweep protocol; and
generate the map based on the plurality of binary masks, wherein the map is representative of the sweep as a whole.

3. The system of claim 1 or 2,
wherein the anatomical map comprises a plurality of detection regions representative of the plurality of fetal anatomies,
wherein, to determine that the pregnancy is the multiple gestation, the processor is configured to:
perform connected component analysis (CCA) on the first anatomical map;
determine, based on the CCA, that:
the pregnancy is the multiple gestation when the anatomical map comprises at least two detection regions for a first fetal anatomy and at least two detections regions for a second fetal anatomy;
the pregnancy is not the multiple gestation when the anatomical map does not comprise at least two detection regions for the first fetal anatomy and at least two detection regions for the second fetal anatomy.

4. The system of any of claims 1-3, wherein the output representative of the determination that the pregnancy is the multiple gestation comprises the anatomical map.

5. The system of any of claims 1-4,
wherein the deep learning network is trained to detect a plurality of maternal anatomies,
wherein the plurality of detections comprise the plurality of maternal anatomies,
wherein the processor is configured to:
determine a placentation of the multiple gestation based on at least one of the plurality of detections or the anatomical map; and
provide, to the display, an output representative of the placentation.

6. The system of claim 5, wherein the placentation of the multiple gestation comprises one of:
monochorionic and monoamniotic;
monochorionic and diamniotic; or
dichorionic and diamniotic.

7. The system of claim of claim 6, wherein, to determine whether the multiple gestation is monochorionic or dichorionic, the processor is configured to determine a quantity of placentas using at least one of:
a plurality of placenta clusters based on a clustering with midpoints of the plurality of detections; or
a plurality of placenta components based on connected component analysis (CCA) with the anatomical map; and
optionally wherein, to determine whether the multiple gestation is monoamniotic or diamniotic, the processor is configured to determine if the plurality of detections comprises a detection of an inter-twin membrane.

8. The system of claim 6 or 7,
wherein, when the placentation of the multiple gestation comprises monochorionic and monoamniotic, the processor is configured to determine whether a multiple gestation abnormality is suspected; and
provide, to the display, an output representative of whether the multiple gestation abnormality is suspected,
wherein the multiple gestation abnormality comprises two fetuses sharing one fetal anatomy.

9. The system of claim 1,
wherein the processor is configured to identify a first fetus and the second fetus distinctly from one another, based on the anatomical map; and
optionally wherein the anatomical map is generated based on the plurality of detections in a sweep of the blind sweep protocol resulting in a first spine detection region and a second spine detection region in the anatomical map that are spaced from one another by at least a threshold distance.

10. The system of claim 9,
wherein, to identify the first fetus and the second fetus, the processor is configured to perform a hierarchical clustering on the anatomical map; and
separate the anatomical mask into a plurality of detection regions representative of a first fetus and a plurality of detection regions representative of the second fetus.

11. The system of claim 9 or 10,
wherein the processor is configured to:
determine a fetal lie for the first fetus and a fetal lie for the second fetus, distinctly from one another; and
provide, to the display, an output representative of the fetal lie for first fetus and the fetal lie for the second fetus;
and/or
wherein the processor is configured to:
determine a fetal spine position for the first fetus and a fetal spine position for the second fetus, distinctly from one another; and
provide, to the display, an output representative of the fetal spine position for the first fetus and the fetal spine position for the second fetus.

12. The system of any of claims 9-11, wherein the processor is configured to:
perform a comparison between a fetal size parameter for the first fetus and a fetal size parameter for the second fetus;
determine a relative growth between the first fetus and the second fetus; and provide, to the display, an output of the representative of the relative growth; and
optionally wherein the fetal size parameter for the first fetus and the fetal size parameter for the second fetus each comprise at least one of a length, an area, or a volume of the anatomical map.

13. The system of claim 12,
wherein the processor is configured to:
generate a report comprising the anatomical map and a previous anatomical map; and
output to the report to the display,
wherein the first map and the previous first map each comprise a detection region representative of the first fetus and a detection region representative of the second fetus,
wherein sizes of the detection regions are configured to provide an indication of at least one of:
the relative growth;
a growth of the first fetus over time; and
a growth of the second fetus over time.

14. The system of any of the preceding claims,
wherein the processor is configured to:
control the ultrasound probe to perform a calibration sweep before the blind sweep protocol;
determine if a length of the calibration sweep is acceptable;
prompt a user to rescan when the length of the calibration sweep is not acceptable; and
prompt the user to proceed to the blind sweep protocol when the length of the calibration sweep is acceptable;
and/or
wherein the processor is configured to:
if all required anatomies are not present in the plurality of ultrasound image frames, prompt the user to proceed to the blind sweep protocol.

15. A method for multiple gestation pregnancy analysis, comprising:
controlling an ultrasound probe to obtain a plurality of ultrasound image frames during a blind sweep protocol on a patent with a pregnancy;
providing the plurality of ultrasound image frames as an input to a deep learning network trained to detect a plurality of fetal anatomies;
generating, as an output of the deep learning network, a plurality of detections of the plurality of fetal anatomies within the plurality of ultrasound image frames;
generating an anatomical map based on the plurality of fetal anatomies;
determining that the pregnancy is a multiple gestation based on the anatomical map; and
providing, to a display, an output representative of the determination that the pregnancy is the multiple gestation.
